# EUROPEAN PATENT APPLICATION

(11) **EP 4 817 617 A1**
(43) Date of publication of application: **07.10.2026**
(21) Application number: 24900633.9
(22) Date of filing: 04.12.2024
(51) Int. Cl.: A61K 31/7032, A61K 9/1271, A61K 47/14, A61K 47/18, A61K 47/24, A61K 47/28, A61P 35/00

(54) **LIPOSOME AGENT, NKT CELL ACTIVATOR, PHARMACEUTICAL COMPOSITION, AND USES OF THESE**

(30) Priority: 04.12.2023 JP 2023204574
(71) Applicant: Ambicion Co., Ltd., Tokyo 160-0017 (JP); The University of Tokyo, Bunkyo-ku, Tokyo 113-8654 (JP)
(72) Inventor: HONG Taehun, Tokyo 101-0047 (JP); IGARASHI Kazunori, Tokyo 112-0002 (JP); CABRAL Horacio, Tokyo 113-8654 (JP)
(74) Representative: Scharfenberger, Burkhard
(86) International application number: PCT/JP2024/042812
(87) International publication number: WO 2025/121339

(57) **Abstract**

The purpose of the present invention is to provide a composition that is superior for practical use with regard to a ligand for activating NKT cells. A liposome agent according to the present invention is characterized by containing a PEGylated liposome, and is also characterized in that: the PEGylated liposome contains an NKT cell ligand and a liposome-constituting lipid; the NKT cell ligand is an a-galactosylceramide, or an analog thereof, that is specifically recognized by an NKT cell receptor on an NKT cell; and the liposome-constituting lipid contains a choline-containing phospholipid, a PEGylated phospholipid, and a cholesterol-based lipid.

## Description

### TECHNICAL FIELD

The present invention relates to a liposome formulation, an NKT cell activator, a pharmaceutical composition, and uses thereof.

### BACKGROUND ART

The following mechanism is known regarding the immune system. Specifically, immature dendritic cells take up an antigen and, upon migrating to a lymph node, differentiate into mature dendritic cells and present the antigen. CD8-positive T cells (killer T cells) are then activated by the mature dendritic cells presenting the antigen, acquire cytotoxic activity, and attack cells having the antigen. However, in a living body that has developed cancer, cancer cells suppress the differentiation of the immature dendritic cells into mature dendritic cells, which causes the problem that the attack on cancer cells utilizing this mechanism is inhibited. For this reason, treatment methods that activate the immune system (commonly referred to as immunotherapy) for diseases such as cancer have become widespread.

In recent years, as a means of activating the immune system, activation of natural killer T cells (hereinafter referred to as NKT cells), which the patient himself or herself possesses in the body, has been attempted. In this method, for example, a ligand that is specifically recognized by NKT cells (hereinafter also referred to as an NKT cell-activating ligand) is administered as a drug, whereby antigen-presenting cells in the body are caused to present the ligand via CD1d. The NKT cells are activated by specifically recognizing and binding to the presented ligand. When the NKT cells are activated, differentiation into mature dendritic cells is induced in the immature dendritic cells whose differentiation has been suppressed by the cancer cells. Accordingly, cancer antigens are presented by the mature dendritic cells, and the cancer cells can be attacked by killer T cells that have acquired cytotoxic activity. In addition, the activated NKT cells can also activate other immune system cells, such as killer T cells and natural killer cells (hereinafter referred to as NK cells), through the release of cytokines such as interleukin-4 (IL-4) and interferon-γ (IFN-γ), thereby enhancing the immune response. Immunotherapy targeting NKT cells in this manner is also referred to, for example, as NKT cell-targeted therapy.

Various compounds have already been studied as the NKT cell-activating ligand, and, for example, α-galactosylceramide (α-GalCer) and analogs thereof have been reported to activate NKT cells (Patent Literatures 1 to 4 and Non-Patent Literature 1).

On the other hand, with regard to a drug, not only efficacy but also stability and effective delivery are important for practical application.

### CITATION LIST

### Patent Literature

Patent Literature 1: WO 94/09020
Patent Literature 2: WO 94/02168
Patent Literature 3: WO 94/24142
Patent Literature 4: WO 98/44928

### Non-Patent Literature

Non-Patent Literature 1: Science, 278, p. 1626-1629, 1997

### SUMMARY OF INVENTION

### Technical Problem

Therefore, an object of the present invention is to provide, with respect to a ligand that activates NKT cells, a composition having superior suitability for practical application.

### Solution to Problem

To achieve the aforementioned object, the liposome formulation of the present invention is characterized by comprising a PEGylated liposome, wherein the PEGylated liposome comprises an NKT cell-activating ligand and a liposome-constituting lipid, the NKT cell-activating ligand is α-galactosylceramide or an analog thereof, which is specifically recognized by an NKT cell receptor on an NKT cell, and the liposome-constituting lipid comprises a choline-containing phospholipid, a PEGylated lipid, and a cholesterol-based lipid.

The NKT cell activator of the present invention is characterized by comprising the liposome formulation of the present invention described above.

The pharmaceutical composition of the present invention is characterized by comprising the liposome formulation of the present invention described above.

The method of activating NKT cells of the present invention is characterized by comprising a step of bringing the liposome formulation of the present invention described above into contact with NKT cells.

### Advantageous Effects of Invention

According to the liposome formulation of the present invention, for example, the storage stability is excellent, and the effect of activating NKT cells can be obtained in a living body.

### BRIEF DESCRIPTION OF DRAWINGS

FIG. 1 is a graph showing the results of the long-term storage stability of B7 liposomes in Example 2.
FIG. 2A is a graph showing the IFN-γ concentration in serum and lymph node supernatant in Example 3.
FIG. 2B is a graph showing the IL-10 concentration in serum and lymph node supernatant in Example 3.
FIG. 3A is a histogram of NKT cells in a lymph node, which is a graph representing the distribution of the number of cells, with respect to NK1.1-positive and CD3-positive cells in the lymph node, in terms of the relative fluorescence intensity indicating CD3-positivity. FIG. 3B is a graph in which the amount of NK1.1-positive and CD3-positive NKT cells in the lymph node is quantified.
FIG. 4A is a histogram of NKT cells in a spleen, which is a graph representing the distribution of the number of cells, with respect to NK1.1-positive and CD3-positive cells in the spleen, in terms of the relative fluorescence intensity. FIG. 4B is a graph in which the amount of NK1.1-positive and CD3-positive NKT cells in the spleen is quantified.

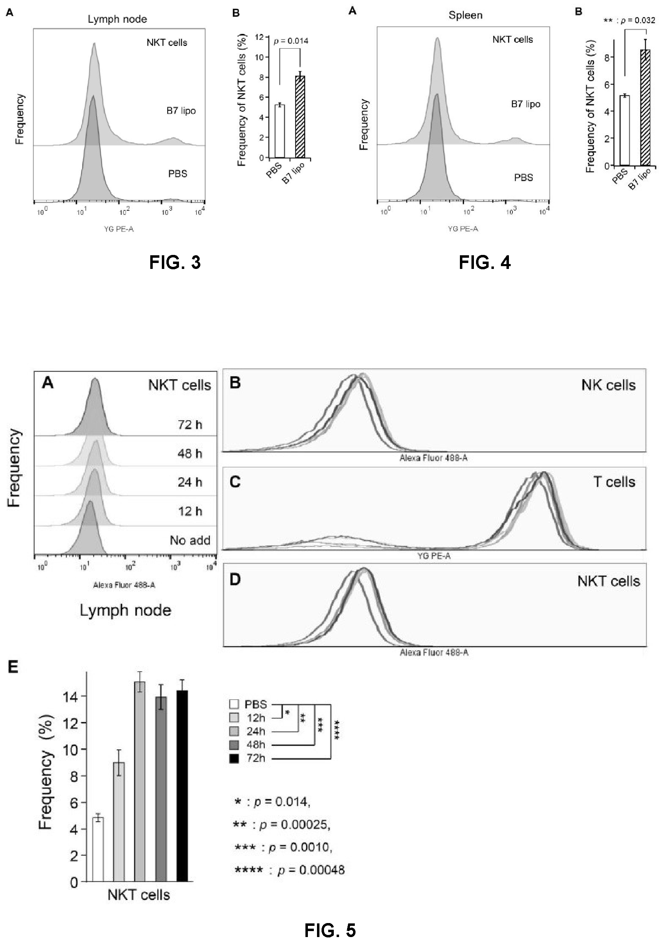

FIG. 5A is a histogram of NKT cells in a lymph node, which is a graph representing the distribution of the number of cells, with respect to NK1.1-positive and CD3-positive cells in the lymph node, in terms of the relative fluorescence intensity indicating CD3-positivity. FIG. 5B is a histogram of NK cells in the lymph node, which is a graph representing the distribution of the number of cells, with respect to NK1.1-positive NK cells in the lymph node, in terms of the relative fluorescence intensity indicating NK1.1-positivity. FIG. 5C is a histogram of T cells in the lymph node, which is a graph representing the distribution of the number of cells, with respect to CD3-positive T cells in the lymph node, in terms of the relative fluorescence intensity indicating CD3-positivity. FIG. 5D is a histogram of NKT cells in the lymph node. FIG. 5E is a graph in which the amount of NK1.1-positive and CD3-positive NKT cells in the lymph node is quantified.

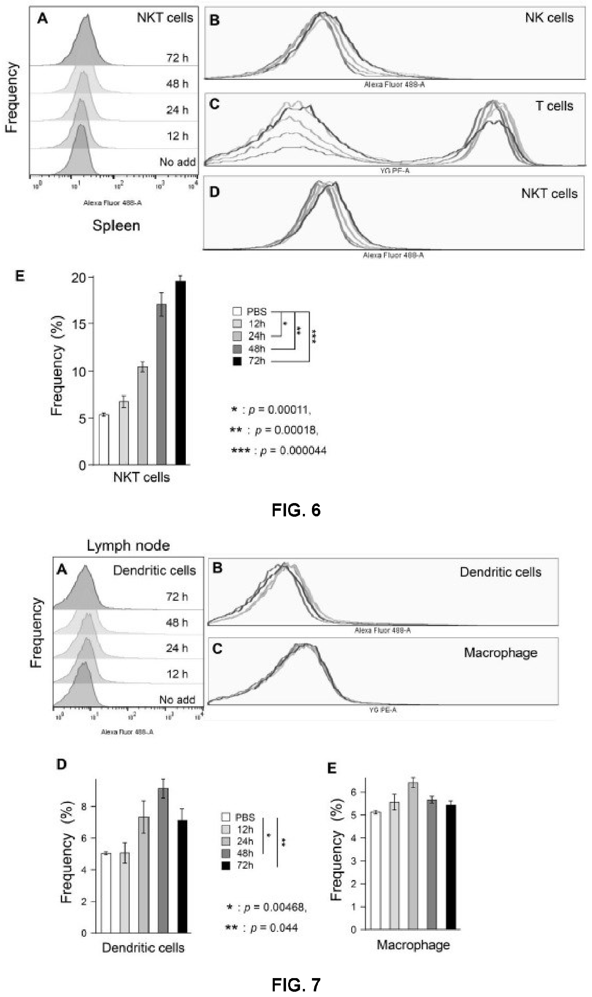

FIG. 6A is a histogram of NKT cells in a spleen, which is a graph representing the distribution of the number of cells, with respect to NK1.1-positive and CD3-positive cells in the spleen, in terms of the relative fluorescence intensity indicating CD3-positivity. FIG. 6B is a histogram of NK cells in the spleen, which is a graph representing the distribution of the number of cells, with respect to NK1.1-positive NK cells in the spleen, in terms of the relative fluorescence intensity indicating NK1.1-positivity. FIG. 6C is a histogram of T cells in the spleen, which is a graph representing the distribution of the number of cells, with respect to CD3-positive T cells in the spleen, in terms of the relative fluorescence intensity indicating CD3-positivity. FIG. 6D is a histogram of NKT cells in the spleen. FIG. 6E is a graph in which the amount of NK1.1-positive and CD3-positive NKT cells in the spleen is quantified.
FIG. 7A is a histogram of dendritic cells in a lymph node, which is a graph showing the distribution of the number of cells, with respect to CD11c-positive cells in the lymph node, in terms of the relative fluorescence intensity indicating CD11c-positivity. FIG. 7B is a histogram of dendritic cells in the lymph node. FIG. 7C is a histogram of macrophages in the lymph node, which is a graph showing the distribution of the number of cells, with respect to CD68-positive cells in the lymph node, in terms of the relative fluorescence intensity indicating CD68-positivity. FIG. 7D is a graph in which the amount of CD11c-positive dendritic cells in the lymph node is quantified. FIG. 7E is a graph in which the amount of CD68-positive macrophages in the lymph node is quantified.

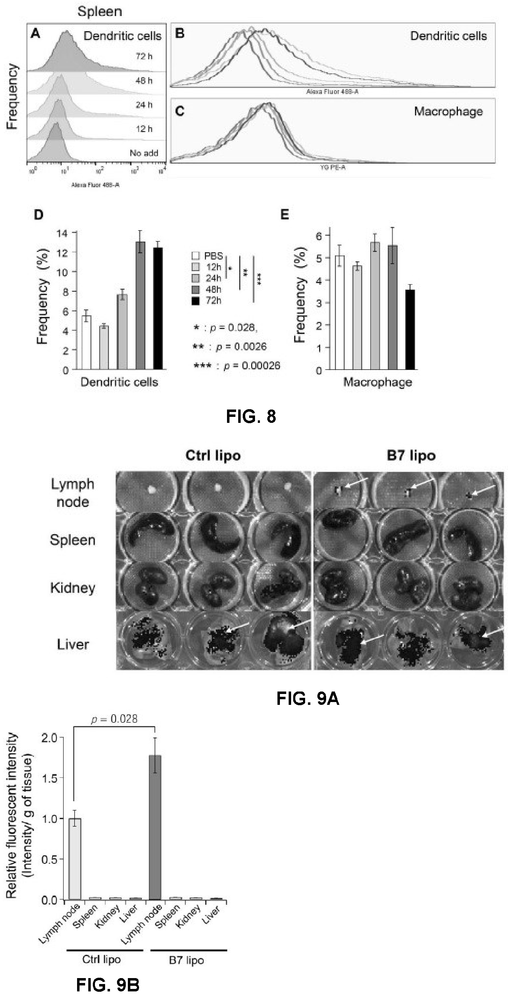

FIG. 8A is a histogram of dendritic cells in a spleen, which is a graph showing the distribution of the number of cells, with respect to CD11c-positive cells in the spleen, in terms of the relative fluorescence intensity indicating CD11c-positivity. FIG. 8B is a histogram of dendritic cells in the spleen. FIG. 8C is a histogram of macrophages in the spleen, which is a graph showing the distribution of the number of cells, with respect to CD68-positive cells in the spleen, in terms of the relative fluorescence intensity indicating CD68-positivity. FIG. 8D is a graph in which the amount of CD11c-positive dendritic cells in the spleen is quantified. FIG. 8E is a graph in which the amount of CD68-positive macrophages in the spleen is quantified.
FIG. 9A is an image showing fluorescence in each organ of a mouse 24 hours after administration.
FIG. 9B is a graph showing the relative fluorescence intensity in each organ of the mouse 24 hours after administration.
FIG. 10 is a graph showing the relationship between the tumor volume (V) of a mouse and the number of days from the start of administration.
FIG. 11 is a graph showing the relationship between the survival rate of a mouse and the number of days from the start of administration.
FIG. 12 is a graph showing the relationship between the body weight (g) of a mouse and the number of days from the start of administration.
FIG. 13 is a graph showing the relationship between the tumor volume (V) of a mouse and the number of days from the start of administration.
FIG. 14 is a graph showing the relationship between the survival rate of a mouse and the number of days from the start of administration.
FIG. 15 is a graph showing the relationship between the body weight (g) of a mouse and the number of days from the start of administration.
FIG. 16A is a graph showing the relationship between the tumor volume (V) of a mouse and the number of days from the start of administration.
FIG. 16B is a graph showing the relationship between the body weight (g) of a mouse and the number of days from the start of administration.
FIG. 17 is a graph showing the relationship between the tumor volume (V) of a mouse and the number of days from the start of administration.
FIG. 18A is an image showing fluorescence in a lymph node.
FIG. 18B is a graph showing the fluorescence intensity in all excised lymph nodes.
FIG. 19 is an image showing fluorescence in a cancer and a spleen at a site where cancer cells were inoculated.
FIG. 20 is an image showing fluorescence in a liver to which cancer has metastasized.
FIG. 21A is a graph showing the distribution of cells in terms of FITC fluorescence intensity and Cy5 fluorescence intensity. FIG. 21B is a graph showing the distribution of cells in terms of FITC fluorescence intensity. FIG. 21C is a graph in which the proportion of CD11c-positive cells is quantified. FIG. 21D is a graph showing the distribution of CD11c-positive cells in terms of Cy5 fluorescence intensity. FIG. 21E is a result obtained by quantifying the proportion of CD11c-positive and Cy5-positive cells.
FIG. 22A is a graph showing the distribution of cells in terms of FITC fluorescence intensity and Cy5 fluorescence intensity. FIG. 22B is a graph showing the distribution of cells in terms of FITC fluorescence intensity. FIG. 22C is a graph in which the proportion of CD68-positive cells is quantified. FIG. 22D is a graph showing the distribution of CD68-positive cells in terms of Cy5 fluorescence intensity. FIG. 22E is a result obtained by quantifying the proportion of CD68-positive and Cy5-positive cells.
FIG. 23A is a graph showing the distribution of cells in terms of NK1.1 fluorescence intensity and Cy5 fluorescence intensity. FIG. 23B is a graph showing the distribution of cells in terms of FITC fluorescence intensity. FIG. 23C is a graph in which the proportion of NK1.1-positive cells is quantified. FIG. 23D is a graph showing the distribution of NK1.1-positive cells in terms of Cy5 fluorescence intensity. FIG. 23E is a result obtained by quantifying the proportion of NK1.1-positive and Cy5-positive cells.
FIG. 24A is a graph showing the distribution of cells in terms of FITC fluorescence intensity and Cy5 fluorescence intensity. FIG. 24B is a graph showing the distribution of cells in terms of FITC fluorescence intensity. FIG. 24C is a graph in which the proportion of CD3-positive cells is quantified. FIG. 24D is a graph showing the distribution of CD3-positive cells in terms of Cy5 fluorescence intensity. FIG. 24E is a result obtained by quantifying the proportion of CD3-positive and Cy5-positive cells.
FIG. 25A is a graph showing the distribution of double-positive cells (NKT cells) that are CD3-positive and NK1.1-positive in terms of FITC fluorescence intensity. FIG. 25B is a result obtained by quantifying the proportion of double-positive cells (NKT cells) that are CD3-positive and NK1.1-positive. FIG. 25C is a graph showing the distribution of double-positive cells (NKT cells) that are CD3-positive and NK1.1-positive in terms of Cy5 fluorescence intensity. FIG. 25D is a result obtained by quantifying the proportion of CD3-positive/NK1.1-positive/Cy5-positive cells.
FIG. 26 is an immunohistochemical staining micrograph of a section slide of cancer in a mouse.
FIG. 27A is a diagram in which fluorescence intensity is superimposed on photographs of organs at 12 hours after administration in a mouse administered with an administration sample of B7 liposomes.
FIG. 27B is a graph in which the fluorescence intensity in a lymph node is quantified.
FIG. 28 is an electron micrograph of an administration sample.
FIG. 29A is a diagram showing the amount of accumulation of B7 liposomes in a lymph node of a mouse in terms of fluorescence intensity.
FIG. 29B is a graph in which the fluorescence intensity indicating the amount of accumulation of B7 liposomes in a lymph node of a mouse is quantified.
FIG. 30 is a graph showing the uptake of B7 liposomes into cells in terms of Cy5 fluorescence intensity derived from the B7 liposomes.
FIG. 31 is a graph showing the uptake of B7 liposomes into dendritic cells in terms of Cy5 fluorescence intensity derived from the B7 liposomes.
FIG. 32 is a graph showing the uptake of B7 liposomes into dendritic cells in terms of Cy5 fluorescence intensity derived from the B7 liposomes.
FIG. 33 is a fluorescence microscopy image of a section of a lymph node of a mouse.
FIG. 34A is a graph showing the relationship between the tumor volume (V) of a mouse and the number of days from the start of administration. FIG. 34B is a graph showing the relationship between the survival rate of a mouse and the number of days from the start of administration. FIG. 34C is a graph showing the relationship between the body weight of a mouse and the number of days from the start of administration.
FIG. 35A is a graph showing the relationship between the tumor volume (V) of a mouse and the number of days from the date of subcutaneous inoculation of cancer. FIG. 35B is a graph showing the relationship between the survival rate of a mouse and the number of days from the date of subcutaneous inoculation of cancer. FIG. 35C is a graph showing the relationship between the body weight of a mouse and the number of days from the date of subcutaneous inoculation of cancer.

### DETAILED DESCRIPTION OF EMBODIMENTS

[1] A liposome formulation, comprising:
   a PEGylated liposome,
   wherein the PEGylated liposome comprises an NKT cell-activating ligand and a liposome-constituting lipid,
   wherein the NKT cell-activating ligand is α-galactosylceramide or an analog thereof, which is specifically recognized by an NKT cell receptor on an NKT cell, and
   wherein the liposome-constituting lipid comprises a choline-containing phospholipid, a PEGylated lipid, and a cholesterol-based lipid.
[2] The liposome formulation according to [1], wherein the choline-containing phospholipid is a phosphatidylcholine.
[3] The liposome formulation according to [2], wherein two fatty acids in the phosphatidylcholine are each independently a saturated fatty acid or an unsaturated fatty acid.
[4] The liposome formulation according to [2] or [3], wherein two fatty acids in the phosphatidylcholine are each independently a fatty acid having 8 to 27 carbon atoms.
[5] The liposome formulation according to any one of [2] to [4], wherein the phosphatidylcholine is at least one selected from the group consisting of dipalmitoylphosphatidylcholine, dioleoylphosphatidylcholine, palmitoyloleoylphosphatidylcholine, and stearoyloleoylphosphatidylcholine.
[6] The liposome formulation according to any one of [1] to [5], wherein a fatty acid in the PEGylated lipid is a saturated fatty acid or an unsaturated fatty acid.
[7] The liposome formulation according to any one of [1] to [6], wherein a fatty acid in the PEGylated lipid is a fatty acid having 8 to 70 carbon atoms.
[8] The liposome formulation according to any one of [1] to [7], wherein the PEGylated lipid is at least one selected from the group consisting of DSPE-PEG, DMG-PEG, DPPE-PEG, DSG-PEG, DOPE-PEG, ceramide-PEG, and stearic acid-PEG.
[9] The liposome formulation according to any one of [1] to [8], wherein an average molecular weight of PEG in the PEGylated lipid is from 160 to 44,000 Da.
[10] The liposome formulation according to any one of [1] to [9], wherein, in the PEGylated lipid, a terminus of PEG is a hydroxyl group.
[11] The liposome formulation according to any one of [1] to [10], wherein the cholesterol-based lipid is cholesterol.
[12] The liposome formulation according to any one of [1] to [11], wherein a size of the PEGylated liposome is in a range of an average particle diameter of 10 to 500 nm.
[13] The liposome formulation according to any one of [1] to [12], wherein, in the PEGylated liposome, a mol% ratio of the choline-containing phospholipid (X), the PEGylated lipid (Y), and the cholesterol-based lipid (Z) is X:Y:Z = 1 : (0.03 to 0.176) : (0.34 to 1.35).
[14] The liposome formulation according to any one of [1] to [13], wherein the α-galactosylceramide or the analog thereof is a compound represented by formula (I), a salt thereof, or a solvate thereof: 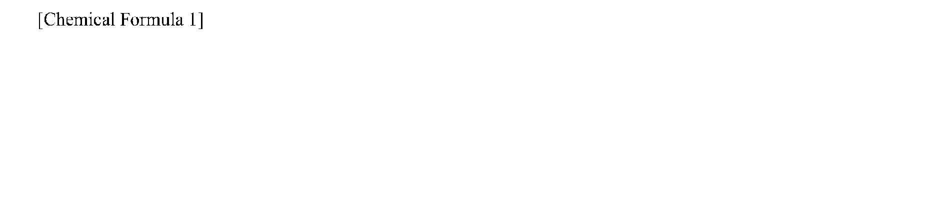 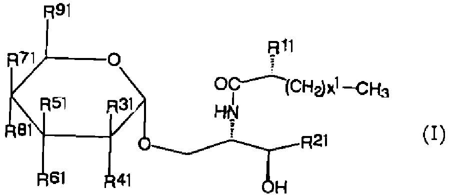
[15] The liposome formulation according to any one of [1] to [13], wherein the analog of α-galactosylceramide is a compound represented by formula (II), a salt thereof, or a solvate thereof: 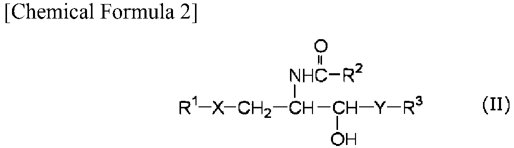
[16] The liposome formulation according to any one of [1] to [13], wherein the analog of α-galactosylceramide is a compound represented by formula (III), a salt thereof, or a solvate thereof: 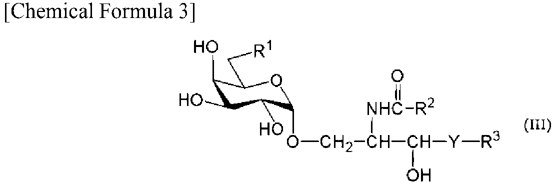
[17] The liposome formulation according to any one of [1] to [13], wherein the analog of α-galactosylceramide is a compound represented by formula (IV), a salt thereof, or a solvate thereof: 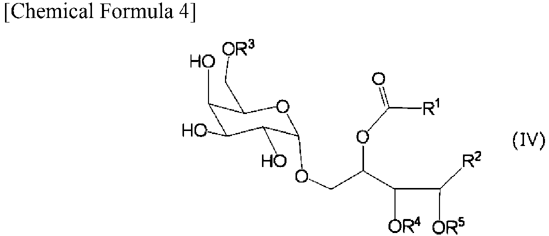
[18] The liposome formulation according to any one of [1] to [13], wherein the analog of α-galactosylceramide is a compound represented by formula (V), a salt thereof, or a solvate thereof: 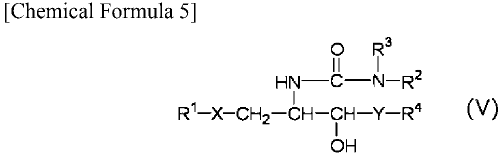
[19] The liposome formulation according to any one of [1] to [13], wherein the analog of α-galactosylceramide is a compound represented by formula (VI), a salt thereof, or a solvate thereof: 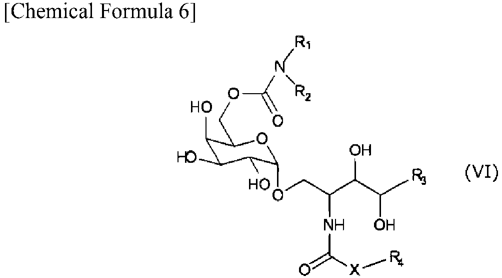
[20] The liposome formulation according to [19], wherein the analog of α-galactosylceramide is a compound represented by the following formula (VI-1), a salt thereof, or a solvate thereof: 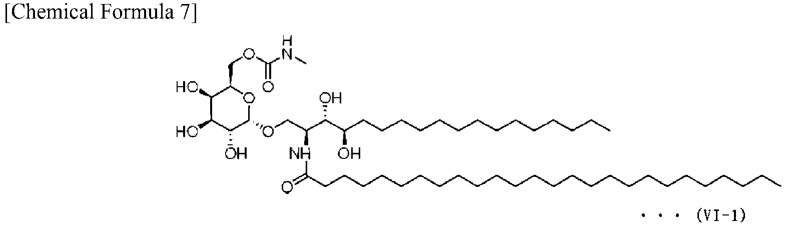
[21] The liposome formulation according to any one of [1] to [20], wherein the PEGylated liposome satisfies conditions 1, 2, and 3:
   (condition 1) a size of the PEGylated liposome is in a range of an average particle diameter of 60 to 140 nm;
   (condition 2) in the PEGylated lipid, a terminus of PEG is a hydroxyl group; and
   (condition 3) a surface charge of the PEGylated liposome is a negative charge.
[22] The liposome formulation according to [21], wherein, in condition 3, the surface charge of the PEGylated liposome is from -80 to 5 mV.
[23] An NKT cell activator, comprising the liposome formulation according to any one of [1] to [22].
[24] A pharmaceutical composition, comprising the liposome formulation according to any one of [1] to [22].
[25] The pharmaceutical composition according to [24], which is for anticancer use.
[26] The pharmaceutical composition according to [25], wherein a target cancer is breast cancer, skin cancer, hematologic cancer, colorectal cancer, prostate cancer, or ovarian cancer.
[27] The pharmaceutical composition according to any one of [24] to [26], which is an injection.
[28] The pharmaceutical composition according to any one of [24] to [27], which is for anticancer use, wherein a target cancer is triple-negative breast cancer, and the PEGylated liposome contained in the liposome formulation satisfies conditions 1, 2, and 3:
   (condition 1) a size of the PEGylated liposome is in a range of an average particle diameter of 60 to 140 nm;
   (condition 2) in the PEGylated lipid, a terminus of PEG is a hydroxyl group; and
   (condition 3) a surface charge of the PEGylated liposome is a negative charge.
[29] The pharmaceutical composition according to [28], wherein, in condition 3, the surface charge of the PEGylated liposome is from -80 to 5 mV.
[30] A method of activating an NKT cell, comprising a step of administering, to a subject, the liposome formulation according to any one of [1] to [22] or the pharmaceutical composition according to any one of [24] to [29].
[31] The method of activating an NKT cell according to [30], wherein the subject is a human or a non-human animal.
[32] A method of treating cancer, comprising a step of administering, to a subject, the liposome formulation according to any one of [1] to [22] or the pharmaceutical composition according to any one of [24] to [29].
[33] The method of treating cancer according to [32], wherein the subject is a subject having triple-negative breast cancer, and in the step of administering, the pharmaceutical composition according to [28] or [29] is administered.
[34] The method of treating cancer according to [32] or [33], wherein the subject is a human or a non-human animal.
[35] The liposome formulation according to any one of [1] to [22], for use in activating an NKT cell.
[36] The liposome formulation according to any one of [1] to [22], for use in the production of a pharmaceutical composition.

The terms used herein, unless otherwise specified, can have the meanings commonly used in the technical field.

A liposome is a lipid vesicle that has formed a lipid bilayer.

### 1. Liposome formulation

The liposome formulation of the present invention, as described above, comprises a PEGylated liposome, wherein the PEGylated liposome comprises an NKT cell-activating ligand and a liposome-constituting lipid, the NKT cell-activating ligand is α-galactosylceramide or an analog thereof, which is specifically recognized by an NKT cell receptor on an NKT cell, and the liposome-constituting lipid comprises a choline-containing phospholipid, a PEGylated lipid, and a cholesterol-based lipid. The liposome formulation of the present invention will be described below by way of examples; however, the present invention is not limited to these examples. In addition, the description of other embodiments can be applied to the liposome formulation of the present invention.

### (1) NKT cell-activating ligand

The NKT cell-activating ligand is generally a compound that is specifically recognized by the T cell receptor unique to NKT cells (NKT cell receptor) on an NKT cell, and is capable of specifically activating NKT cells. The NKT cell-activating ligand is presented, for example, on a CD1d molecule. The "NKT cell-activating ligand" in the present invention may be any compound that is specifically recognized by the aforementioned NKT cell receptor and is capable of specifically activating NKT cells. In the present invention, the NKT cell-activating ligand is not particularly limited, and examples thereof include glycolipids that have already been known to have a specific activating function on NKT cells, that is, α-galactosylceramide (α-GalCer) and derivatives thereof (analogs thereof). Hereinafter, α-GalCer and derivatives thereof will be exemplified as the NKT cell-activating ligand.

### (1-1) Compound represented by formula (I), a salt thereof, or a solvate thereof.

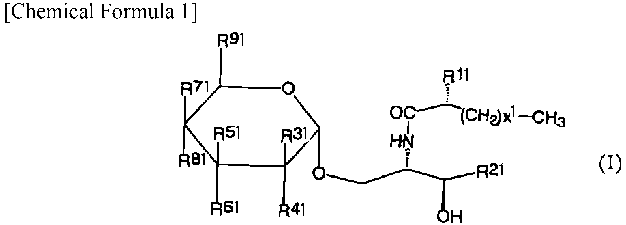

In formula (I),
R¹¹ is H or OH;
X¹ is an integer of any of 7 to 27; and
R²¹ is a substituent selected from the group consisting of the following (a) to (e) (Y¹ is an integer of any of 5 to 17):
   (a) -CH₂(CH₂)Y¹CH₃
   (b) -CH(OH)(CH₂)Y¹CH₃
   (c) -CH(OH)(CH₂)Y¹CH(CH₃)₂
   (d) -CH=CH(CH₂)Y¹CH₃
   (e) -CH(OH)(CH₂)Y¹CH(CH₃)CH₂CH₃

In formula (I), R³¹ to R⁹¹ are substituents defined by the following i) or ii):
i) When R³¹, R⁶¹, and R⁸¹ are H,
   R⁴¹ is H, OH, NH₂, NHCOCH₃, or a substituent selected from the group consisting of the following groups (A) to (D);
   R⁵¹ is OH or a substituent selected from the group consisting of the following groups (E) and (F);
   R⁷¹ is OH or a substituent selected from the group consisting of the following groups (A) to (D); and
   R⁹¹ is H, CH₃, CH₂OH, or a substituent selected from the group consisting of the following groups (A') to (D').
ii) When R³¹, R⁶¹, and R⁷¹ are H,
   R⁴¹ is H, OH, NH₂, NHCOCH₃, or a substituent selected from the group consisting of the following groups (A) to (D);
   R⁵¹ is OH or a substituent selected from the group consisting of the following groups (E) and (F);
   R⁸¹ is OH or a substituent selected from the group consisting of the following groups (A) to (D); and
   R⁹¹ is H, CH₃, CH₂OH, or a substituent selected from the group consisting of the following groups (A') to (D').

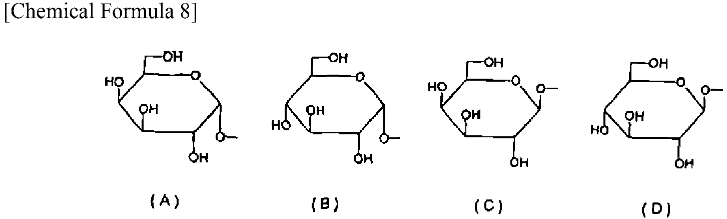

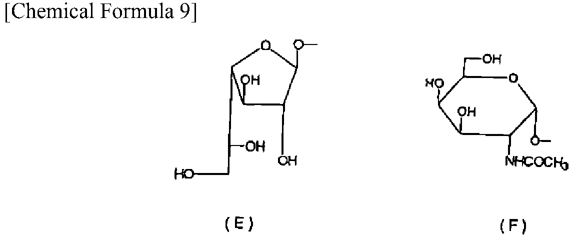

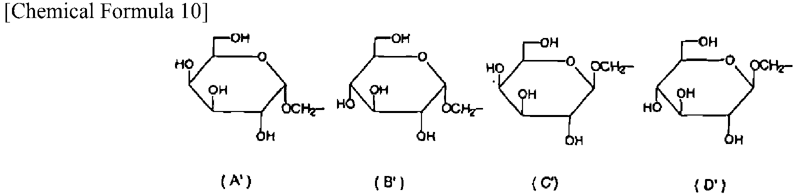

As the compound represented by formula (I), for example, α-GalCer (α-galactosylceramide) represented by the following formula (a) is preferable. The chemical name of α-GalCer is (2S,3S,4R)-1-O-(α-D-galactopyranosyl)-2-hexacosanoylamino-1,3,4-octadecanetriol.

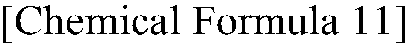

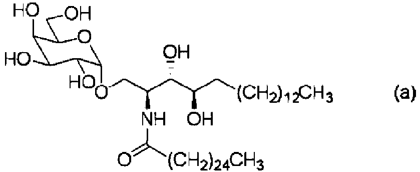

As the compound represented by formula (I), a salt thereof, or a solvate thereof, those described in, for example, patent publications (such as WO 94/09020, WO 94/02168, WO 94/24142, and WO 98/44928) and articles (such as Science, 278, p. 1626-1629, 1997) can be used, and they can also be produced by the methods described therein. It has already been confirmed in these literatures that the compound represented by formula (I) has the function of the NKT cell-activating ligand.

### (1-2) Compound represented by formula (II), a salt thereof, or a solvate thereof.

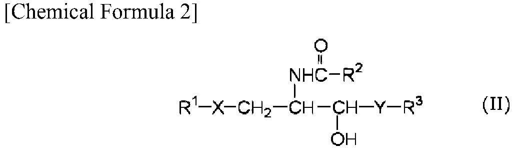

In formula (II), R¹ represents an α-carba-sugar residue; R² and R³ each independently represent a substituted or unsubstituted hydrocarbon group having 1 to 28 carbon atoms; X represents an oxygen atom, a sulfur atom, -CH₂-, or NH-; and Y represents -CH₂-, -CH(OH)-, or CH=CH-.

Among the compounds represented by formula (II), the following compounds are particularly preferable:
[1] (2S,3S,4R)-1-(5a-carba-α-D-galactopyranosyloxy)-2-(hexacosanoylamino)-3,4-octadecanediol
[2] (2S,3S,4R)-1-(5a-carba-α-D-galactopyranosylthio)-2-(hexacosanoylamino)-3,4-octadecanediol
[3] (2S,3S,4R)-1-(5a-carba-α-D-glucopyranosyloxy)-2-(hexacosanoylamino)-3,4-octadecanediol
[4] (2S,3S,4R)-1-(5a-carba-α-D-glucopyranosylthio)-2-(hexacosanoylamino)-3,4-octadecanediol
[5] (2S,3S,4R)-1-(5a-carba-α-D-fucopyranosyloxy)-2-(hexacosanoylamino)-3,4-octadecanediol

As the compound represented by formula (II), a salt thereof, or a solvate thereof, those described in, for example, patent publications (such as WO 2008/102888 and U.S. Patent No. 8,299,223) can be used, and they can also be produced by the methods described therein. It has already been confirmed in these literatures that the compound represented by formula (II) and the like have the function of the NKT cell-activating ligand.

### (1-3) Compound represented by formula (III), a salt thereof, or a solvate thereof.

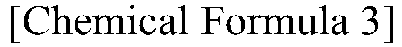

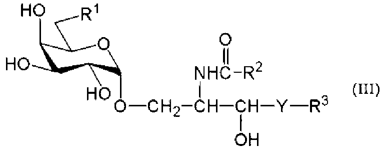

In formula (III), R¹ represents a hydrogen atom, an alkyl group having 1 to 7 carbon atoms, an alkoxy group having 1 to 6 carbon atoms, or a halogen atom; R² and R³ each independently represent a substituted or unsubstituted hydrocarbon group having 1 to 28 carbon atoms; and Y represents -CH₂-, - CH(OH)-, or CH=CH-. However, when R¹ is a hydrogen atom, R² represents a substituted or unsubstituted hydrocarbon group having 24 to 28 carbon atoms.

Among the compounds represented by formula (III), the following compounds A12, A13, A14, and A15 are particularly preferable.
A12 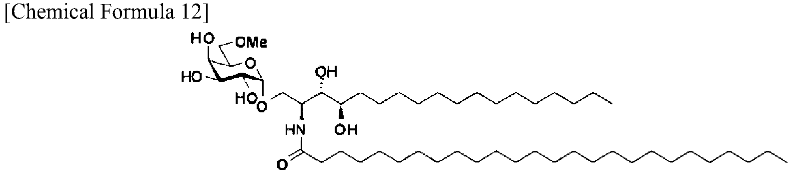
A13 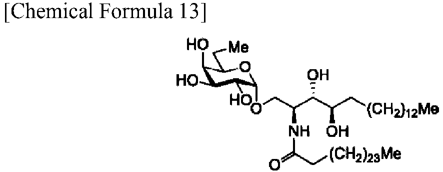
A14 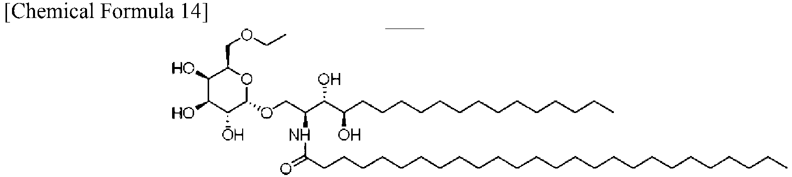
A15

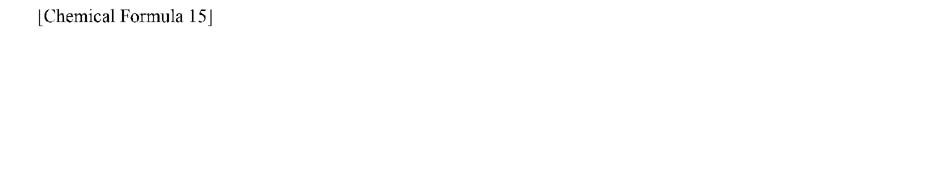

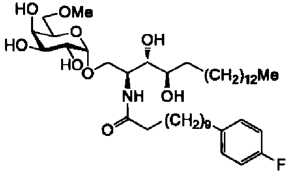

As the compound represented by formula (III), a salt thereof, or a solvate thereof, those described in, for example, patent publications (such as WO 2009/119692 and U.S. Patent No. 8,551,959) can be used, and they can also be produced by the methods described therein. It has already been confirmed in these literatures that the compound represented by formula (III) and the like have the function of the NKT cell-activating ligand.

### (1-4) Compound represented by formula (IV), a salt thereof, or a solvate thereof.

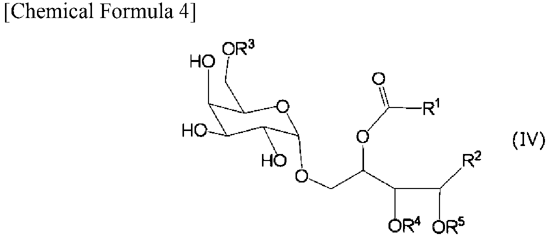

In formula (IV), R¹ represents a hydrocarbon group having 1 to 30 carbon atoms; R² represents a hydrocarbon group having 1 to 20 carbon atoms; and R³ represents a hydrogen atom or a hydrocarbon group having 1 to 5 carbon atoms. In formula (IV), R⁴ and R⁵, identical or different, may represent a hydrogen atom or a hydrocarbon group having 1 to 5 carbon atoms, or, alternatively, R⁴ and R⁵ together may form a divalent hydrocarbon group having 1 to 5 carbon atoms, which together with the adjacent ethylenedioxy may form a ring structure.

As the compound represented by formula (IV), a salt thereof, or a solvate thereof, those described in, for example, patent publications (such as WO 2010/030012 and U.S. Patent No. 8,580,751) can be used, and they can also be produced by the methods described therein. It has already been confirmed in these literatures that the compound represented by formula (IV) and the like have the function of the NKT cell-activating ligand.

### (1-5) Compound represented by formula (V), a salt thereof, or a solvate thereof.

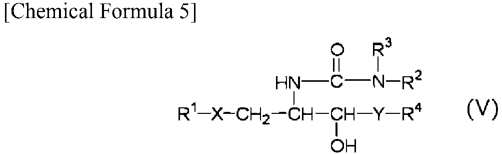

In formula (V), R' represents an aldopyranose residue, in which the 6-position hydroxyl group may be alkylated; R² represents a hydrocarbon group having 1 to 26 carbon atoms, which may have a substituent; R³ represents a hydrocarbon group having 1 to 26 carbon atoms, which may be a hydrogen atom or may have a substituent; R⁴ represents a hydrocarbon group having 1 to 21 carbon atoms, which may have a substituent; X represents an oxygen atom or CH₂-; and Y represents -CH₂-, -CH(OH)-, or CH=CH-.

Among the compounds represented by formula (V), the following compounds are particularly preferable:
[1] (2S,3S,4R)-1-(α-D-galactopyranosyloxy)-2-(tetracosanylureido)-3,4-octadecanediol
[2] (2S,3S,4R)-1-(α-D-galactopyranosyloxy)-2-(hexadecanylureido)-3,4-octadecanediol
[3] (2S,3S,4R)-1-(6-O-methyl-α-D-galactopyranosyloxy)-2-(tetracosanylureido)-3,4-octadecanediol

As the compound represented by formula (V), a salt thereof, or a solvate thereof, those described in, for example, patent publications (such as WO 2011/55284 and U.S. Patent No. 8,853,173) can be used, and they can also be produced by the methods described therein. It has already been confirmed in these literatures that the compound represented by formula (V) and the like have the function of the NKT cell-activating ligand.

### (1-6) Compound represented by formula (VI), a salt thereof, or a solvate thereof.

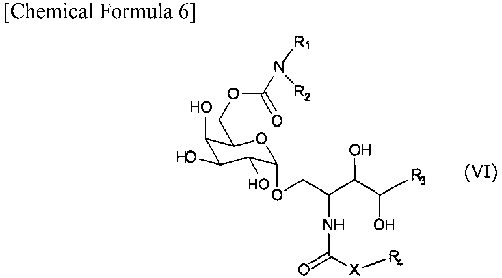

In formula (VI),
X represents an alkylene group or -NH-;
R¹ and R², identical or different, represent a hydrogen atom, an alkyl group, a hydroxyl group, an alkoxy group, or an aryl group, and may have a substituent;
R¹ and R² may form a 5- to 6-membered ring together with the adjacent nitrogen atom;
R³ represents a hydrocarbon group having 1 to 20 carbon atoms; and
R⁴ represents a hydrocarbon group having 1 to 30 carbon atoms.

Among the compounds represented by formula (VI), the following compounds are particularly preferable.
B7 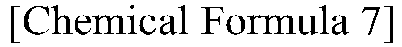 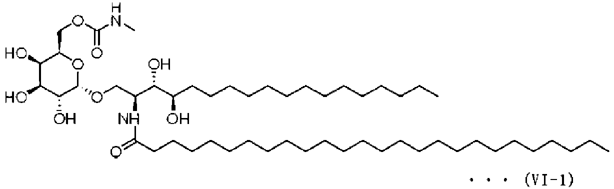
B16

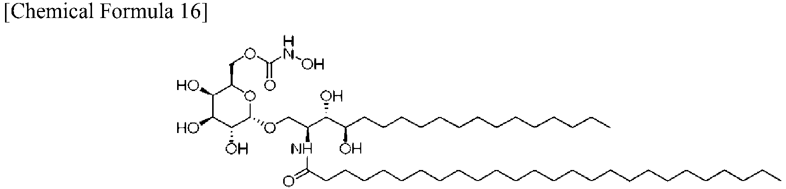

As the compound represented by formula (VI), a salt thereof, or a solvate thereof, those described in, for example, patent publications (such as WO 2013/162016 A1 and U.S. Patent Application Publication No. 2015/152128 A1) can be used, and they can also be produced by the methods described therein. It has already been confirmed in these literatures that the compound represented by formula (VI) and the like have the function of the NKT cell-activating ligand.

Among the aforementioned NKT cell-activating ligands, from the viewpoint of, for example, more strongly inducing IFN-γ production by NKT cells, the compound represented by formula (III) or formula (VI) is preferable, the compounds A14, B16, and B7 are more preferable, and B7 is particularly preferable.

### (2) Liposome-constituting lipid

The liposome-constituting lipid comprises a choline-containing phospholipid, a PEGylated lipid, and a cholesterol-based lipid. The liposome-constituting lipid may, for example, comprise only the choline-containing phospholipid, the PEGylated lipid, and the cholesterol-based lipid, or may further comprise other components. The other components may be, for example, components and amounts that do not inhibit the formation of a liposome by the liposome-constituting lipid.

### (2-1) Choline-containing phospholipid

The choline-containing phospholipid has a structure in which two fatty acids are ester-bonded to a glycerol backbone as a hydrophobic portion, and choline is phosphoester-bonded to the glycerol backbone as a hydrophilic portion. Examples of the choline-containing phospholipid include phosphatidylcholine. The choline-containing phospholipid may be, for example, PEGylated or non-PEGylated (non-PEGylated), and is preferably a non-PEGylated choline-containing phospholipid.

The fatty acid has, for example, a structure having a carboxyl group at a terminus of a carbon chain, and may be a saturated fatty acid in which the carbon chain is saturated, or an unsaturated fatty acid in which the carbon chain has a double bond. In the choline-containing phospholipid, two fatty acids may each independently be a saturated fatty acid or an unsaturated fatty acid. Examples of the combination of two fatty acids include a combination of saturated fatty acids, a combination of unsaturated fatty acids, and a combination of a saturated fatty acid and an unsaturated fatty acid. The two fatty acids may each independently be, for example, a branched fatty acid or an unbranched fatty acid. The two fatty acids may, for example, have the same number of carbon atoms or different numbers of carbon atoms, or may have carbon chains (main chains) of the same length or carbon chains (main chains) of different lengths.

The number of carbon atoms of the fatty acid is, for example, 8 to 70, 24 to 70, or 42 to 58. The number of carbon atoms of the main chain of the fatty acid is, for example, 4 to 35, 12 to 35, or 21 to 29.

Examples of the combination of fatty acids in the choline-containing phospholipid include those shown in the following table, and specific examples thereof include the following phosphatidylcholines.

**[Table 1]**

| **Choline-containing phospholipid** | | |
|---|---|---|
| **Combination of fatty acids** | | **Specific examples** |
| **Fatty acid 1** | **Fatty acid 2** | **Phosphatidylcholine** |
| Palmitic acid C16 Saturated | Palmitic acid C16 Saturated | 1,2-Dipalmitoyl-sn-glycero-3-phosphocholine (DPPC) 16:0 - 16:0 |
| Oleic acid C18 Unsaturated | Oleic acid C18 Unsaturated | 1,2-Dioleoyl-sn-glycero-3-phosphocholine (DOPC) 18:1 - 18:1 |
| Palmitic acid C16 Saturated | Oleic acid C18 Unsaturated | 1-Palmitoyl-2-oleoyl-sn-glycero-3-phosphocholine (POPC) 16:0 - 18:1 |
| Oleic acid C18 Unsaturated | Stearic acid C18 Saturated | 1-Stearoyl-2-oleoyl-sn-glycero-3-phosphocholine (PC) 18:0 - 18:1 |

### (2-2) PEGylated lipid

The PEGylated lipid is a phospholipid modified with PEG. The PEGylated lipid has a structure in which two fatty acids are ester-bonded to a glycerol backbone as a hydrophobic portion, and PEG is ester-bonded to the glycerol backbone as a hydrophilic portion.

The fatty acid may be, for example, a saturated fatty acid or an unsaturated fatty acid. The fatty acid may be, for example, a branched fatty acid or an unbranched fatty acid.

The number of carbon atoms of the fatty acid is, for example, 8 to 70, 24 to 70, or 42 to 58. The number of carbon atoms of the main chain of the fatty acid is, for example, 4 to 35, 12 to 35, or 21 to 29.

In the PEGylated lipid, the average molecular weight of PEG (polyethylene glycol) has, for example, a lower limit of 160 Da, 162 Da, 1,800 Da, or 4,500 Da; an upper limit of 44,000 Da, 5,500 Da, or 2,200 Da; and a range of 160 to 44,000 Da, 162 to 44,000 Da, 1,800 to 5,500 Da, or 1,800 to 2,200 Da. The average molecular weight is, for example, a number-average molecular weight, and can be measured by gel permeation chromatography (GPC), NMR, or the like.

Specific examples of the PEGylated lipid are shown in the following table. In the following table, the lipids in the PEGylated lipid and their fatty acids are also exemplified. The lipid may be, for example, a phospholipid. Examples of the phospholipid include phosphatidylcholine and phospholipids having phosphoethanolamine. The number of carbon atoms of the fatty acids exemplified in the following table may be varied, for example, in the range of C8 to C22.

**[Table 2]**

| **Fatty acid** | **Lipid** | **PEGylated lipid** |
|---|---|---|
| Stearic acid C18 Saturated / Stearic acid C18 Saturated | DSPE | DSPE-PEG |
| | 1,2-distearoyl-sn-glycero-3-phosphoethanolamine | 1,2-distearoyl-sn-glycero-3-phosphoethanolamine-polyethylene glycol |
| Myristic acid C14 Saturated / Myristic acid C14 Saturated | DMG | DMG-PEG |
| | 1,2-Dimyristoyl-rac-glycerol | 1,2-dimyristoyl-rac-glycero-3-methoxypolyethylene glycol |
| Palmitic acid C16 Saturated / Palmitic acid C16 Saturated | DPPE | DPPE-PEG |
| | 1,2-Dipalmitoyl-sn-glycero-3-phosphoethanolamine | N-(Methylpolyoxyethylene oxycarbonyl)-1,2-dipalmitoyl-sn-glycero-3-phosphoethanolamine |
| Stearic acid C18 Saturated / Stearic acid C18 Saturated | DSG | DSG-PEG |
| | 1,2-distearoyl-rac-glycerol | 1,2-distearoyl-rac-glycero-3-methylpolyoxyethylene |
| Oleic acid C18 Unsaturated / Oleic acid C18 Unsaturated | DOPE | DOPE-PEG |
| | 1,2-Dioleoyl-sn-glycero-3-phosphoethanolamine | 1,2-Dioleoyl-sn-glycero-3-phosphoethanolamine-polyethylene glycol methoxy |
| Caprylic acid C8 Saturated | | Ceramide-PEG |
| | Ceramide | N-octanoyl-sphingosine-1-{succinyl[methoxy(polyethylene glycol)2000] } |
| | N-palmitoyl-D-erythro-sphingosine | |
| Stearic acid C18 Saturated | Stearic acid | Stearic acid-PEG |
| | Ethyl octadecanoate stearic acid ethyl ester | Ethyl octadecanoate stearic acid ethyl ester-polyethylene glycol methoxy |

### (2-3) Cholesterol-based lipid

As the cholesterol-based lipid, reference can be made, for example, to the articles by Konrad B. et al. (Science, 1965, vol. 150, p. 3692) and Nsairat H. et al. (J. Liposome Res., 2023), and specific examples thereof that can be used include cholesterol, β-sitosterol, sodium cholate, and sodium dodecyl sulfate.

The cholesterol-based lipid may be, for example, a PEGylated cholesterol-based lipid or a non-PEGylated cholesterol-based lipid, and the former is preferable.

### (3) PEGylated liposome

The liposome formulation of the present invention, as described above, comprises a PEGylated liposome as a liposome. The liposome contained in the liposome formulation of the present invention is, as described above, formed into a liposome by the liposome-constituting lipid, and since the liposome-constituting lipid comprises the PEGylated phospholipid, it is referred to as a PEGylated liposome.

The liposome formulation of the present invention may contain, for example, one or two or more PEGylated liposomes. In the latter case, the PEGylated liposomes may be, for example, monodisperse liposomes containing molecules of a single molecular weight, or polydisperse liposomes containing molecules of different molecular weights.

The size and dispersion of the PEGylated liposome are not particularly limited. The size and dispersion of the PEGylated liposome can be measured, for example, by analytical methods such as dynamic light scattering (DLS), laser diffraction, SEM, and TEM. The size of the PEGylated liposome contained in the liposome formulation of the present invention can be represented, for example, by particle size distribution, and the aforementioned analytical methods can be used. The particle size distribution can be represented, for example, by a frequency distribution showing a relationship between frequency (%) and particle diameter, or by a cumulative distribution showing a relationship between cumulative percentage (%) and particle diameter.

In the case of evaluating the size by dynamic light scattering, for example, a zeta potential analyzer (for example, trade name Zetasizer, manufactured by Malvern Instruments Ltd.) equipped with a diode laser (for example, λ = 532 nm) can be used. The evaluation conditions are not particularly limited, and are, for example, a detection temperature of 22 to 28°C (specific example: 25°C). The intensity-average hydrodynamic diameter and the polydispersity index can be obtained, for example, in accordance with the cumulant method.

The size of the PEGylated liposome contained in the liposome formulation of the present invention can be represented, for example, by an average value (average particle diameter), a median value (also referred to as median or D50), a mode (also referred to as mode or peak), and a distribution width. As a specific example, when the size of the PEGylated liposome is determined by dynamic light scattering, the following size is preferable. In the present invention, the method of analyzing the size of the PEGylated liposome is not limited to dynamic light scattering.

In the PEGylated liposome, the Z-average hydrodynamic diameter (Z-Average) measured, for example, by the dynamic scattering method using the aforementioned zeta potential analyzer is preferably within a predetermined range. The lower limit of the Z-average hydrodynamic diameter (Z-Average) is, for example, 10 nm or more, 30 nm or more, 50 nm or more, 80 nm or more, or 90 nm or more; the upper limit thereof is, for example, 500 nm or less, 400 nm or less, 300 nm or less, 200 nm or less, 150 nm or less, 140 nm or less, 130 nm or less, 120 nm or less, or 110 nm or less; and the range thereof is, for example, 10 to 500 nm, 30 to 500 nm, 10 to 400 nm, 30 to 400 nm, 30 to 300 nm, 50 to 200 nm, or 80 to 120 nm, and is also 100 ± 50 nm, 100 ± 40 nm, 100 ± 30 nm, 100 ± 20 nm, or 100 ± 10 nm.

The Z-average hydrodynamic diameter (Z-Average) is a hydrodynamic diameter based on the ISO standard and can be determined by the cumulants method. The Z-Average can be calculated, for example, from a correlation function obtained from measurement data of dynamic light scattering (DLS) using the following equation. 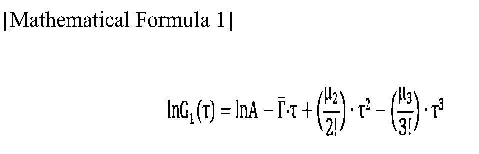 A is the intercept of the correlation function and represents the signal-to-noise ratio. Γ is the average decay rate, and the diffusion coefficient D of the particles is calculated by substituting it into the equation Γ = q²D. The average particle diameter D□, that is, the Z-average hydrodynamic diameter, is calculated by substituting the diffusion coefficient D into the Stokes-Einstein equation.

The size of the PEGylated liposome has, for example, in the case of a normal distribution, a standard deviation within ±2 STD or within ±1 STD.

The distribution of the size of the PEGylated liposome can be represented, for example, by the polydispersity index (PDI). The PDI is an index for evaluating the width of the particle size distribution. The PDI of the PEGylated liposome is, for example, 1, or 0.1 to 0.5, 0.15 to 0.35, or 0.2 to 0.3. The PDI can be obtained, for example, by a fitting analysis in accordance with the cumulant method on the distribution measured by the aforementioned dynamic light scattering method.

In the PEGylated liposome, the ratio (X:Y:Z) of the choline-containing phospholipid (X), the PEGylated lipid (Y), and the cholesterol-based lipid (Z) is not particularly limited. As a specific example, in the case of a mol% ratio, the following can be exemplified:
X:Y:Z = 1 : 0.03 to 0.176 : 0.34 to 1.35,
1 : 0.05 to 0.176 : 0.34 to 0.675,
1 : 0.06 to 0.088 : 0.5 to 0.675
Furthermore, the ratio (X+Y+Z : L) of the NKT ligand (L) to these liposome-constituting lipids (X+Y+Z) is not particularly limited. As a specific example, in the case of a mol% ratio, the following can be exemplified: (X+Y+Z) : L = 1 : (0.05 to 2), 1 : (0.2 to 1), 1 : (0.5 to 1)

In the PEGylated liposome, the amount of the NKT ligand is not particularly limited. When the amount of the PEGylated liposome is converted into total lipids, the amount of the NKT ligand per 1 mg of total lipids of the PEGylated liposome is not particularly limited; the lower limit thereof is, for example, more than 0 µg, 5 µg or more, 10 µg or more, 20 µg or more, 50 µg or more, or 100 µg or more; the upper limit thereof is, for example, 1000 µg or less, 900 µg or less, 800 µg or less, 700 µg or less, 600 µg or less, or 500 µg or less; and the range thereof is, for example, more than 0 to 1000 µg, 5 to 900 µg, 10 to 900 µg, 20 to 900 µg, 20 to 800 µg, 50 to 700 µg, 50 to 600 µg, or 100 to 500 µg.

### (4) Method of preparing the PEGylated liposome

The method of preparing the PEGylated liposome is not particularly limited; the NKT cell-activating ligand and the liposome-constituting lipid may be used as raw materials, and a general liposome formation method using lipid raw materials can be adopted. As the liposome formation method, for example, the article (O. H. Voss et al., Curr. Protoc. Immunol., 2018, vol. 120, no. 14.44, p. 1-21) and the like can be referred to. The method of preparation is exemplified below; however, the present invention is not limited to these descriptions.

A raw material solution is prepared using the NKT cell-activating ligand and the liposome-constituting lipid as raw materials. The raw material solution may be prepared, for example, by separately preparing a ligand solution containing the NKT cell-activating ligand and a lipid mixture solution containing the choline-containing phospholipid, the PEGylated phospholipid, and the cholesterol-based lipid, or by separately preparing the ligand solution, a solution containing the choline-containing phospholipid, a solution containing the PEGylated phospholipid, and a solution containing the cholesterol-based lipid. The solvent of the raw material solution is not particularly limited, and examples thereof include organic solvents. Examples of the organic solvent include alcohols such as methanol, chloroform, dichloromethane, and acetonitrile, and any one type of solvent or a mixed solvent of two or more types may be used. The raw material solution may be, for example, a diluted solution obtained by dilution with the organic solvent.

Next, the various raw material solutions are mixed. For example, the ligand solution and the lipid mixture solution may be mixed, or the ligand solution, the solution containing the choline-containing phospholipid, the solution containing the PEGylated phospholipid, and the solution containing the cholesterol-based lipid may be mixed. In this case, the mixing ratio (X:Y:Z) of the choline-containing phospholipid (X), the PEGylated lipid (Y), and the cholesterol-based lipid (Z), and the mixing ratio (X+Y+Z : L) of the NKT ligand (L) to these liposome-constituting lipids (X+Y+Z) are not particularly limited, and the aforementioned mol% ratios can be exemplified.

Then, the solvent fraction is evaporated from the raw material mixture solution in which the respective raw materials have been mixed. The evaporation of the solvent fraction can be performed, for example, by placing the raw material mixture solution in a vessel such as a flask and subjecting it to an evaporator. By the evaporation of the solvent fraction, a lipid film can be formed on the inner surface of the vessel. This lipid film is a lipid film composed of the PEGylated liposomes containing the NKT cell-activating ligand.

Then, the lipid film formed on the inner surface of the vessel is suspended in, for example, an aqueous solvent and subjected to ultrasonic treatment. By disrupting the lipid film by the ultrasonic treatment, the lipid film can be decomposed into PEGylated liposomes containing the NKT cell-activating ligand. The aqueous solvent is not particularly limited, and examples thereof include water, physiological saline, buffered physiological saline, and buffer solutions.

The decomposition product of the lipid film (a mixture of PEGylated liposomes) can be classified into liposome fractions of a desired size and dispersion, for example, by filtration using a membrane of a desired pore size.

### (5) Form of the liposome formulation

The form of the liposome formulation of the present invention is not particularly limited, and may be a liquid or a solid. In the case of the liquid, the form is, for example, a dispersion liquid dispersed in a solvent. The solvent is, for example, a solvent in which the PEGylated liposome does not dissolve, and specific examples thereof include the aforementioned aqueous solvent. In the case of the solid, the form is, for example, a state in which the aqueous solvent has been removed from the dispersion liquid, or a state in which, after removing the aqueous solvent, the dispersion liquid has been dried (dried body).

When the liposome formulation of the present invention is in the form of the liquid, the concentration of the PEGylated liposome is not particularly limited. In the liposome formulation of the present invention, the concentration of the PEGylated liposome can be represented, for example, by the amount of lipids of the PEGylated liposome. The concentration of the PEGylated liposome in the liposome formulation of the present invention, in terms of the amount of lipids of the PEGylated liposome, has, for example, a lower limit of 0.05 mg/mL, 0.1 mg/mL, or 0.5 mg/mL; an upper limit of 1 mg/mL, 5 mg/mL, or 25 mg/mL; and a range of 0.05 to 25 mg/mL, 0.1 to 5 mg/mL, or 0.5 to 1 mg/mL. The concentration may be, for example, the concentration during storage of the liposome formulation of the present invention or the concentration at the time of use.

When the liposome formulation of the present invention is stored, the conditions thereof are not particularly limited. The storage temperature is, for example, 2 to 15°C or 3 to 5°C, and the storage period is, for example, 90 days or less, 30 days or less, or 14 days or less.

The liposome formulation of the present invention may contain, for example, other additives in addition to the PEGylated liposome. The additives may be, for example, those described in connection with the NKT cell activator of the present invention described below. The descriptions of the NKT cell activator of the present invention and the pharmaceutical composition of the present invention described below can be applied to the liposome formulation of the present invention.

### (6) Properties of the liposome formulation

The liposome formulation of the present invention has, for example, excellent stability. The stability of the liposome formulation of the present invention can be represented, for example, by changes over time in the particle size distribution of the PEGylated liposome contained in the liposome formulation. The stability can be evaluated by, for example, stability with respect to the storage period (stability 1) and stability with respect to dilution after storage (stability 2).

The stability 1 with respect to the storage period can be evaluated, for example, based on the presence or absence or the amount of aggregates in the dispersion liquid after a certain period of time has elapsed. Specifically, it can be evaluated by determining whether or not aggregates have been generated in the dispersion liquid (D₁) after a certain period of time has elapsed, in comparison with the dispersion liquid (D₀) immediately after being prepared under the same conditions, and if aggregates have been generated, to what extent the aggregates have been generated.

The stability 2 with respect to dilution after storage can be evaluated, for example, based on the presence or absence or the amount of aggregates in a diluted solution obtained by diluting the dispersion liquid after a certain period of time has elapsed. Specifically, it can be evaluated by determining whether or not aggregates have been generated in the diluted solution (d₁) obtained by diluting the dispersion liquid (D₁) after a certain period of time has elapsed, in comparison with the diluted solution (d₀) immediately after the dispersion liquid (D₀) is prepared and diluted under the same conditions, and if aggregates have been generated, to what extent the aggregates have been generated.

In the liposome formulation of the present invention, the surface charge of the PEGylated liposome is not particularly limited. The examples to be described below can be applied to the surface charge, which is, for example, -80 mV to 5 mV, and a negative charge is preferable.

### (7) Use of the liposome formulation

The liposome formulation of the present invention can be used, for example, in the NKT cell activator and the method of activating NKT cells, the pharmaceutical composition and the treatment method of the present invention described below. The descriptions of the respective embodiments described below can be applied to the method of using the liposome formulation of the present invention.

### 2. NKT cell activator and method of activating NKT cells

The NKT cell activator of the present invention, as described above, is characterized by comprising the liposome formulation of the present invention. Furthermore, the method of activating NKT cells of the present invention, as described above, is characterized by using the liposome formulation of the present invention. The NKT cell activator and the activation method of the present invention are characterized by using the liposome formulation of the present invention, and other conditions are not particularly limited. The descriptions of other embodiments can be applied to the NKT cell activator and the activation method of the present invention.

### (1) NKT cell activator

The NKT cell activator of the present invention comprises the liposome formulation of the present invention. The NKT cell activator of the present invention may, for example, comprise only the liposome formulation of the present invention, or may comprise other additives. The additives are preferably, for example, pharmaceutically acceptable additives. The pharmaceutically acceptable additives include, for example, those known in the pharmaceutical field.

Examples of the additives include carriers, diluents, solvents, excipients, stabilizers, and isotonic agents. Examples of the carrier include monosaccharides such as sucrose, polysaccharides such as cellulose, and the aforementioned aqueous solvent. Examples of the diluent and the excipient include those similar to the carrier. Examples of the solvent include the aforementioned aqueous solvent. As the additives, drugs such as protein drugs (e.g., PD-L1 (Programmed cell Death ligand 1) and antibodies) and cellular drugs may also be used in combination. The additives are preferably, for example, hydrophilic or weakly hydrophobic.

In the NKT cell activator of the present invention, the PEGylated liposome may, for example, be adjusted to a concentration at the time of use, or may be at a concentration higher than that and diluted at the time of use. The concentration of the PEGylated liposome in the NKT cell activator of the present invention is not particularly limited, and, for example, the concentrations exemplified for the NKT cell activator of the present invention can be applied.

### (2) Method of activating NKT cells

The method of activating NKT cells of the present invention is a method of activating NKT cells by using the liposome formulation of the present invention. In the present invention, the liposome formulation can be read as the NKT cell activator of the present invention or the pharmaceutical composition of the present invention described below.

In the present invention, the use of the liposome formulation of the present invention may be, for example, in vivo or in vitro.

When the liposome formulation of the present invention is used in vivo, the method of activating NKT cells of the present invention comprises a step of administering the liposome formulation to a subject.

The subject is, for example, a human or a non-human animal. Examples of the non-human animal include non-human mammals such as mice, rats, rabbits, dogs, cats, cows, horses, monkeys, pigs, goats, and sheep.

The administration method is not particularly limited, and is oral administration or parenteral administration, and parenteral administration is preferable. Examples of the parenteral administration include injection and infusion. Examples of the administration site include subcutaneous, intravenous, intra-arterial, intramucosal, intra-lymph node, and local sites. The local site may be, for example, a target affected tissue, or a site capable of delivering the liposome formulation to the affected site. The form of the liposome formulation of the present invention is not particularly limited as described above, and can be appropriately determined depending on the administration form. Examples of the form include injections and infusions.

The administration conditions of the liposome formulation of the present invention are not particularly limited, and can be appropriately set depending on the animal species, age, sex, health condition, body weight, and the like of the administration target. When the administration target is an adult human male, for example, the dose of the PEGylated liposome per administration is 0.005 to 50 mg/kg body weight, 0.15 to 15 mg/kg body weight, or 1 to 15 mg/kg body weight. The number of administrations is, for example, every 1 to 28 days, 1 to 2 times per day.

When the liposome formulation of the present invention is administered subcutaneously, the PEGylated liposome can be efficiently delivered, for example, to lymph nodes such as axillary lymph nodes, the liver, and the breast. Furthermore, when the liposome formulation of the present invention is administered subcutaneously to the flank, the PEGylated liposome can be accumulated in the axillary lymph node, for example, within 12 to 48 hours (specific example: about 24 hours later).

When the liposome formulation of the present invention is used in vitro, the method of activating NKT cells of the present invention comprises, for example, a step of bringing the liposome formulation into contact with NKT cells.

### 3. Pharmaceutical composition and method of treating cancer

The pharmaceutical composition of the present invention, as described above, is characterized by comprising the liposome formulation of the present invention. Furthermore, the method of treating cancer of the present invention, as described above, is characterized by comprising a step of administering the liposome formulation of the present invention or the pharmaceutical composition of the present invention to a subject. The pharmaceutical composition and the treatment method of the present invention are characterized by using the liposome formulation of the present invention, and other conditions are not particularly limited. The descriptions of other embodiments can be applied to the pharmaceutical composition and the treatment method of the present invention. In the present invention, the liposome formulation and the pharmaceutical composition can be read as the NKT cell activator of the present invention.

### (1) Pharmaceutical composition

The pharmaceutical composition of the present invention comprises the liposome formulation of the present invention. The pharmaceutical composition of the present invention may, for example, comprise only the liposome formulation of the present invention, or may comprise other additives. As the additives, for example, the descriptions in the NKT cell activator of the present invention can be applied.

The pharmaceutical composition of the present invention can be used, for example, for a disease that can be prevented or treated by the activation of NKT cells. Examples of the disease include cancer. Examples of the target cancer include breast cancer, skin cancer, hematologic cancer, colorectal cancer, prostate cancer, and ovarian cancer. Furthermore, examples of the target cancer include highly metastatic cancers, and specific examples thereof include cancers that are likely to metastasize to other sites, such as hematologic cancer, lung cancer, breast cancer, colorectal cancer, and prostate cancer.

### (2) Method of treating cancer

The method of treating cancer of the present invention is characterized by comprising a step of administering the liposome formulation of the present invention or the pharmaceutical composition to a subject. The subject is not particularly limited, and is, as described above, a human or a non-human animal.

### 4. Use

The present invention is the aforementioned liposome formulation for use in activating NKT cells. The present invention is also the aforementioned liposome formulation for use in the production of an NKT cell activator.

The present invention is the aforementioned liposome formulation for use in the treatment of a disease such as cancer. The present invention is also the aforementioned liposome formulation for use in the production of a pharmaceutical composition such as an anticancer agent.

### [Embodiment 1]

The liposome formulation of the present invention is particularly preferably applied to NKT cell-targeted therapy for intractable cancer. In this embodiment, a liposome formulation used for NKT cell-targeted therapy for the intractable cancer is exemplified. Unless otherwise specified, the aforementioned examples can be applied.

The intractable cancer in this embodiment is a cancer type against which conventional immuno-cancer therapeutic agents such as the PD-1 antibody (anti-PD1) and the PD-L1 antibody (anti-PDL1) are not effective, and a specific example thereof is triple-negative breast cancer. Triple-negative breast cancer refers to breast cancer in which the immune reactions with all of the estrogen receptor (ER), the progesterone receptor (PgR), and HER2 (human epidermal growth factor receptor type 2), which are hormone receptors expressed on the surface of breast cancer cells, are negative. Examples of the triple-negative cancer type among breast cancers include 4T1, Hs578T, BT-20, and HCC70. The liposome formulation in this embodiment is, among intractable cancers, preferably applied to NKT cell-targeted therapy for triple-negative breast cancer.

The liposome formulation of this embodiment can be preferably used, as described above, for patients with triple-negative breast cancer. Patients with triple-negative breast cancer tend to be difficult to respond to immunotherapy, and the therapeutic effect by the aforementioned PD-1 antibody or PD-L1 antibody is difficult to obtain. Therefore, the liposome formulation of this embodiment can be particularly preferably used, for example, for patients in whom the growth of cancer cannot be suppressed even when the PD-1 antibody or the PD-L1 antibody is administered, or for patients in whom the growth of cancer is presumed to be unable to be suppressed even when the PD-1 antibody or the PD-L1 antibody is administered.

The liposome formulation of this embodiment can be applied not only to triple-negative breast cancer but also to so-called cold tumors. Cold tumors refer to, in cancer immunology, tumors to which the immune system shows almost no response. Cold tumors are generally considered difficult to treat because they are weakly attacked by the immune system, and the main characteristics thereof include few immune cells (particularly T cells) within the tumor, low immune cell infiltration, lack of antigen presentation, and an immunosuppressive environment. In addition, the 4T1 cell line is a highly metastatic tumor cell line mainly used in mouse breast cancer research, and is generally classified as a "cold tumor." This is because 4T1 tumors have weak immune responses by cells of both the innate and acquired immune systems and are characterized by poor immune cell infiltration; 4T1 tumors are considered to have an immunosuppressive microenvironment and to easily evade attack by the immune system.

The liposome formulation of this embodiment preferably satisfies the following conditions. Specifically, the liposome formulation of this embodiment preferably satisfies all of the following conditions (condition 1, condition 2, and condition 3). By satisfying these conditions, the liposome formulation can, for example, be more efficiently accumulated in the lymph node, and the liposome formulation can be more efficiently taken up by antigen-presenting cells such as dendritic cells. As a result, NKT cells can be more effectively activated, so that the growth of cancer can be suppressed even in patients for whom the aforementioned PD-1 antibody or the like is not effective.

### (Condition 1)

In the liposome formulation of this embodiment, the diameter of the PEGylated liposome is preferably as follows. That is, when expressed by the Z-average hydrodynamic diameter (Z-average), the lower limit thereof is, for example, 10 nm or more, 50 nm or more, 60 nm, 80 nm or more, or 90 nm or more; the upper limit thereof is, for example, 400 nm or less, 200 nm or less, 150 nm or less, 140 nm or less, 130 nm or less, 120 nm or less, or 110 nm or less; and the range thereof is, for example, 10 to 400 nm, 50 to 200 nm, 60 to 140 nm, 60 to 130 nm, or 80 to 120 nm. In this embodiment, the diameter is more preferably 100 ± 40 nm or 100 ± 30 nm, and particularly preferably 100 ± 20 nm or 100 ± 10 nm.

### (Condition 2)

In the liposome formulation of this embodiment, in the PEGylated lipid of the aforementioned (2-2) contained as the liposome-constituting lipid, the terminus of PEG modifying the phospholipid is preferably a hydroxyl group. PEG having a hydroxyl group at its terminus is hereinafter also referred to as PEG-OH, and a phospholipid modified with PEG-OH is hereinafter also referred to as a PEG-OH-modified lipid.

PEG can be represented by the following structural formula (1), wherein n is a positive integer. In this embodiment, in the PEG of the PEG lipid, both ends are hydrogen atoms as shown in the following structural formula (2), and the right end is preferably a hydroxyl group. When the PEG-OH-modified phospholipid modified with PEG-OH of the following formula (2) is used, in the liposome formulation of this embodiment, the liposome particles have a surface rich in hydroxyl groups derived from PEG-OH.

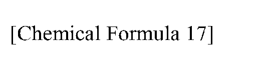

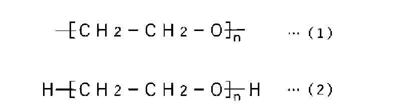

By satisfying the aforementioned condition 2, the liposome formulation of this embodiment can be, for example, more efficiently taken up by antigen-presenting cells such as dendritic cells.

### (Condition 3)

In the liposome formulation of this embodiment, the surface charge of the PEGylated liposome is preferably a negative charge, which is also referred to as the PEGylated liposome being negatively charged.

The lower limit of the zeta potential of the PEGylated liposome is, for example, -80 mV or more, - 60 mV or more, -45 mV or more, -40 mV or more, or -30 mV or more; the upper limit thereof is, for example, 5 mV or less, 0 mV or less, less than 0 mV, -5 mV or less, -10 mV or less, or -20 mV or less; and the range thereof is, for example, -80 to 5 mV, -80 to 0 mV, -60 to 0 mV, -80 to less than 0 mV, -60 to less than 0 mV, -80 to -5 mV, -45 to -5 mV, -40 to -5 mV, -40 to -10 mV, -40 to -20 mV, -30 ± 20 mV, -30 ± 10 mV, or -20 ± 10 mV. The zeta potential of the PEG liposome is, as described above, preferably a negative value, and as specific examples, -80 to -5 mV is preferable, and -30 ± 20 mV or -30 ± 10 mV is more preferable. The liposome formulation of this embodiment comprises a plurality of the PEGylated liposomes. Therefore, in the liposome formulation of this embodiment, for example, all of the PEGylated liposomes may have the same zeta potential or different zeta potentials. Furthermore, 50% or more, 80% or more, or 95% or more of all of the PEGylated liposomes may satisfy the aforementioned zeta potential. The aforementioned zeta potential may also be satisfied by the liposome formulation of the present invention as a whole.

Hereinafter, the present invention will be described in detail by way of Examples and the like; however, the present invention is not limited thereto.

### EXAMPLES

### [Reagents, etc.]

Choline-containing phospholipid:
   Phosphatidylcholine (1,2-dierucoyl-sn-glycero-3-phosphocholine, Tokyo Chemical Industry Co., Ltd.)
Cholesterol-based lipid:
   Cholesterol (Wako Pure Chemical Industries, Ltd.)
Positively charged lipid:
   Didodecyldimethylammonium bromide (DDAB, Sigma-Aldrich)
PEGylated phospholipid:
   · Phospholipid modified with PEG having a hydroxyl group at its terminus
      HO-PEG-DSPE (1,2-distearoyl-sn-glycero-3-phosphoethanolamine-polyethylene glycol, Broadpharm)
   · Phospholipid modified with PEG having a methyl group at its terminus
      METHYL-PEG-DSPE (1,2-Distearoyl-phosphatidyl Ethanolamine-Methyl-Polyethyleneglycol Conjugate, Tokyo Chemical Industry Co., Ltd.)
NKT cell-activating ligand:
   B7 (Nard Chemicals Co., Ltd.)
α-GalCer (Nard Chemicals Co., Ltd.)

Porous membrane:
Trade name: Polycarbonate membrane (Cytiva)
   1: pore size 1000 nm, recovered particle size 1000 nm
   2: pore size 400 nm, recovered particle size 400 nm
   3: pore size 200 nm, recovered particle size 200 nm
   4: pore size 100 nm, recovered particle size 100 nm
   5: pore size 50 nm, recovered particle size 50 nm
RPMI-1640 medium (RPMI, Sigma-Aldrich)
Buffer solution PBS(-) (Takara Bio Inc.)
Fetal bovine serum (FBS, Biological Industries)
Penicillin-streptomycin (Life Technologies)
Sulfo-cyanine 5 succinimidyl ester (Sulfo-Cy5-NHS, Lumiprobe)

### [Cells, etc.]

All cells in the Examples were obtained from RIKEN BioResource Research Center. Each cell was cultured using RPMI medium containing 10% FBS and 1× penicillin-streptomycin, and the culture conditions were 37°C in a 5% CO₂ atmosphere. BALB/c mice and C57BL/6j mice were purchased from Charles River Laboratories Japan, Inc. Animal experimental procedures were performed in accordance with the guidelines for the care and use of laboratory animals at the University of Tokyo.

### [Example 1]

PEGylated liposomes were prepared. The preparation was basically performed in accordance with the article (O. H. Voss et al., Curr. Protoc. Immunol., 2018), except for the reagents used and the amounts added thereof, as follows.

First, liquid reagents of the respective components were prepared. The NKT ligand (B7 or α-GalCer) was dissolved in a mixed solvent (MeOH : chloroform = 4 : 6, volume ratio) to a concentration of 1 mg/mL. α-GalCer was dissolved in a mixed solvent (MeOH : chloroform = 4 : 6, volume ratio) to a concentration of 1 mg/mL. Phosphatidylcholine was dissolved in chloroform to a concentration of 5 mg/mL. The PEGylated phospholipid (HO-PEG-DSPE or METHYL-PEG-DSPE) was dissolved in chloroform to a concentration of 5 mg/mL. The positively charged lipid (DDAB) was dissolved in chloroform to a concentration of 10 mg/mL. Cholesterol was dissolved in chloroform to a concentration of 5 mg/mL.

The liquid reagents of the respective components were mixed in a flask having a volume of 20 mL so as to have the following compositions, to prepare a total of 1 mL of a mixture. In the following mixtures, the descriptions of 50% and 0% refer to the mass% of the NKT cell-activating ligand in the mixture. As for the components other than the NKT cell-activating ligand, the molar ratio was kept constant among the mixtures, and the total lipid concentration in each mixture was set to be 1 mg/mL. To the following mixture for 50% B7 / neutral PEG terminal -OH liposome and mixture for 50% B7 / positively charged PEG terminal -OH liposome, the positively charged lipid (DDAB) was added in order to make the surface charge of the liposomes formed therefrom positive or neutral.

### [NKT cell-activating ligand: B7]

(Mixture for 50% B7 / negatively charged PEG terminal -OH liposome)
B7 solution: 500 µL
Phosphatidylcholine solution: 34 µL
Cholesterol solution: 40 µL
HO-PEG-DSPE solution: 27 µL
Chloroform: remainder
(Mixture for 10% B7 / negatively charged PEG terminal -OH liposome)
B7 solution: 100 µL
Phosphatidylcholine solution: 114 µL
Cholesterol solution: 40 µL
HO-PEG-DSPE solution: 27 µL
Chloroform: remainder
(Mixture for 2% B7 / negatively charged PEG terminal -OH liposome)
B7 solution: 20 µL
Phosphatidylcholine solution: 130 µL
Cholesterol solution: 40 µL
HO-PEG-DSPE solution: 27 µL
Chloroform: remainder
(Mixture for 50% B7 / negatively charged PEG terminal -Me liposome)
B7 solution: 500 µL
Phosphatidylcholine solution: 34 µL
Cholesterol solution: 40 µL
METHYL-PEG-DSPE: 27 µL
Chloroform: remainder
(Mixture for 50% B7 / neutral PEG terminal -OH liposome)
B7 solution: 500 µL
Phosphatidylcholine solution: 23.4 µL
Cholesterol solution: 40 µL
HO-PEG-DSPE solution: 27 µL
DDAB solution: 5.3 µL
Chloroform: remainder
(Mixture for 50% B7 / positively charged PEG terminal -OH liposome)
B7 solution: 500 µL
Phosphatidylcholine solution: 12.8 µL
Cholesterol solution: 40 µL
HO-PEG-DSPE solution: 27 µL
DDAB solution: 10.6 µL
Chloroform: remainder

### [NKT cell-activating ligand: α-GalCer]

(Mixture for 50% α-GalCer / negatively charged PEG terminal -OH liposome)
α-GalCer solution: 500 µL
Phosphatidylcholine solution: 32.7 µL
Cholesterol solution: 41 µL
HO-PEG-DSPE solution: 28 µL
Chloroform: remainder

### [NKT cell-activating ligand: not added]

(Mixture for 0% control / negatively charged PEG terminal -OH liposome)
NKT ligand solution: 0 µL
Phosphatidylcholine solution: 134 µL
Cholesterol solution: 40 µL
HO-PEG-DSPE solution: 27 µL
Chloroform: remainder
(Mixture for 0% control / negatively charged PEG terminal -Me liposome)
NKT ligand solution: 0 µL
Phosphatidylcholine solution: 134 µL
Cholesterol solution: 40 µL
METHYL-PEG-DSPE solution: 27 µL
Chloroform: remainder

Using an evaporator (Tokyo Rikakikai Co., Ltd.), the flask was rotated under a vacuum of 30 hPa at 30°C for 10 minutes to completely dry the solvent in the mixture. As a result, a thin film of lipids was formed on the inner surface of the flask. Then, 1 mL of PBS was added to the flask to resuspend the thin film. The suspension was treated with an ultrasonic bath (Aiwa Medical Industry Co., Ltd.) at 28 kHz for 5 minutes, and further treated with an ultrasonic homogenizer (Taitec Corporation) at 20 kHz for 30 seconds. As a result, the lipid thin film was disrupted, and PEGylated liposomes decomposed into small fragments were obtained.

The decomposed PEGylated liposomes were further treated using the porous membranes and an extruder (trade name AE-10, Bio-Equip) so as to have a particle size within a predetermined range. PBS was used as the solvent during the treatment. Specifically, first, the porous membrane 1 (recovered particle size 1000 nm) was set in the extruder, and the decomposed PEGylated liposomes were passed therethrough three or more times. Next, the porous membrane 2 (recovered particle size 400 nm) was set in the extruder, and the PEGylated liposome fraction that had passed through the porous membrane 1 was passed therethrough three or more times. As a result, PEGylated liposomes having a particle size of about 400 nm were collected as PEGylated liposome fraction A. Subsequently, the porous membrane 3 (recovered particle size 200 nm) was set in the extruder, and the PEGylated liposome fraction that had passed through the porous membrane 2 was passed therethrough three or more times. As a result, PEGylated liposomes having a particle size of about 200 nm were collected as PEGylated liposome fraction B. Subsequently, two sheets of the porous membrane 4 (recovered particle size 100 nm) were stacked and set in the extruder, and the PEGylated liposome fraction that had passed through the porous membrane 3 was passed therethrough three or more times. As a result, PEGylated liposomes having a particle size of about 100 nm were collected as PEGylated liposome fraction C. Furthermore, the porous membrane 5 (recovered particle size 50 nm) was set in the extruder, and the PEGylated liposomes that had passed through the porous membrane 4 were passed therethrough 15 or more times. As a result, PEGylated liposomes having a particle size of about 50 nm were collected as PEGylated liposome fraction D.

Each of the collected PEGylated liposome fractions (hereinafter also referred to as liposome samples) was suspended in PBS, and the particle size of the PEGylated liposomes (Z-average hydrodynamic diameter (Z-average)), the polydispersity index (PDI), and the zeta potential were confirmed.

Specifically, using a zeta potential analyzer (trade name Zetasizer, Malvern Instruments Ltd.) equipped with a diode laser (λ = 532 nm), the particle size of the PEGylated liposomes (Z-average hydrodynamic diameter (Z-average)), the PDI, and the zeta potential were evaluated by dynamic light scattering (DLS) at 25°C at a detection angle of 173°. The particle size and the PDI were obtained in accordance with the cumulant method.

For the liposome samples (the PEGylated liposome fractions), the results of the particle size of the liposomes (Z-average hydrodynamic diameter (Z-average)), the PDI, and the zeta potential are shown in the following table.
(1-1) 50% B7 / negatively charged PEG terminal -OH liposome, fraction C (particle size: about 100 nm)
(1-2) 50% α-GalCer / negatively charged PEG terminal -OH liposome, fraction C (particle size: about 100 nm)
(1-3) 50% B7 / negatively charged PEG terminal -OH liposome, fraction D (particle size: about 50 nm)
(1-4) 50% B7 / negatively charged PEG terminal -OH liposome, fraction B (particle size: about 200 nm)
(1-5) 50% B7 / negatively charged PEG terminal -OH liposome, fraction A (particle size: about 400 nm)
(1-6) 10% B7 / negatively charged PEG terminal -OH liposome, fraction C (particle size: about 100 nm)
(1-7) 2% B7 / negatively charged PEG terminal -OH liposome, fraction C (particle size: about 100 nm)
(1-8) 50% B7 / negatively charged PEG terminal -Me liposome, fraction C (particle size: about 100 nm)
(1-9) 50% B7 / neutral PEG terminal -OH liposome, fraction C (particle size: about 100 nm)
(1-10) 50% B7 / positively charged PEG terminal -OH liposome, fraction C (particle size: about 100 nm)
(1-11) 0% control / negatively charged PEG terminal -OH liposome, fraction C (particle size: about 100 nm)
(1-12) 0% control / negatively charged PEG terminal -Me liposome, fraction C (particle size: about 100 nm)

**[Table 3]**

| **PEGylated liposome fraction Sample No.** | **NKT ligand** | | **PEG terminus** | **Particle size Z-average (nm)** | **PDI** | **Zeta potential (mV)** |
|---|---|---|---|---|---|---|
| | **%** | **Type** | | | | |
| 1-1 | 50% | B7 | -OH | 108.6 | 0.203 | -36.2 |
| 1-2 | 50% | α-GalCer | -OH | 117.7 | 0.147 | -22.4 |
| 1-3 | 50% | B7 | -OH | 33.36 | 0.255 | -34.8 |
| 1-4 | 50% | B7 | -OH | 208.6 | 0.378 | -37.2 |
| 1-5 | 50% | B7 | -OH | 405.5 | 0.484 | -39.5 |
| 1-6 | 10% | B7 | -OH | 101.0 | 0.221 | -30.9 |
| 1-7 | 2% | B7 | -OH | 110.5 | 0.198 | -35.6 |
| 1-8 | 50% | B7 | -Me | 102.1 | 0.219 | -36.0 |
| 1-9 | 50% | B7 | -OH | 115.4 | 0.199 | -0.36 |
| 1-10 | 50% | B7 | -OH | 111.6 | 0.246 | +34.5 |
| 1-11 | 0% | - | -OH | 102.4 | 0.165 | -30.2 |
| 1-12 | 0% | - | -Me | 103.6 | 0.151 | -39.4 |

Furthermore, except that dye-containing PBS was used instead of PBS, various liposome fractions were prepared as liposome samples in the same manner as using PBS. The dye-containing PBS was prepared as follows. That is, a dye reagent (Sulfo-cyanine 5 succinimidyl ester, trade name: Sulfo-Cy5^{®} -NHS, Lumiprobe) was mixed with PBS to a concentration of 0.1 mg/mL and left to stand for 24 hours. By leaving the dye reagent in PBS, -NHS was removed from the dye reagent by hydrolysis, and a dye-containing PBS containing Sulfo-Cy5^{®} at a concentration of 0.1 mg/mL was prepared. It has been confirmed that each of the PEGylated liposomes prepared using the dye-containing PBS has the same particle size and PDI as the aforementioned PEGylated liposomes prepared using the dye-free PBS.

Hereinafter, unless otherwise specified, PBS refers to the dye-free PBS. In addition, the PEGylated liposomes prepared using the dye-free PBS are referred to as liposomes, and the PEGylated liposomes prepared using the dye-containing PBS are referred to as liposomes (dye-containing PBS).

### [Example 2]

The stability of the PEGylated liposomes prepared in Example 1 was confirmed.

### (1) Long-term storage stability

The B7 liposome (sample No. 1-1), the α-GC liposome (sample No. 1-2), and the NKT ligand(-) liposome (sample No. 1-11) prepared in Example 1 were each adjusted to a PEGylated liposome concentration of 1 mg/mL (in terms of the amount of lipids; the same applies hereinafter) and used as liposome samples. PBS was used for the adjustment of the PEGylated liposome concentration. The NKT ligand concentration in each of the liposome samples was measured using pyrene-1-boronic acid (Sigma-Aldrich) by a conventional glycolipid detection method using a fluorescently labeled boronic acid (https://trace.tennessee.edu/utk_graddiss/5634/). The B7 concentration of the liposome sample (PEGylated liposome concentration: 1 mg/mL) prepared from the aforementioned 50% B7 mixture was 0.471 mg/mL (about 0.5 mg/mL), and the GalCer concentration of the liposome sample (PEGylated liposome concentration: 1 mg/mL) prepared from the aforementioned 50% α-GalCer mixture was 0.472 mg/mL (about 0.5 mg/mL).

The liposome samples were stored at 4°C. For the liposome samples before the start of storage (Day 0) and for the liposome samples at 10 days after the start of storage (Day 10) and at 90 days after the start of storage (Day 90), the particle size (number-average hydrodynamic diameter) and the polydispersity index (PDI) were respectively obtained based on the particle size distribution chart obtained by the measurement of dynamic light scattering (DLS). The change rate (%) of the particle size after storage was calculated based on the particle size before the start of storage (Day 0) as a reference. The storage stability was evaluated from the change rate of the particle size and the PDI.

The results of the long-term storage stability of the B7 liposome and the control liposome are shown in the following tables. Table 4A shows the change rate of the particle size, and Table 4B shows the polydispersity index (PDI).

**[Table 4A]**

| **Sample** | | **Particle size change rate (%)** | | | |
|---|---|---|---|---|---|
| | **No.** | **0 Days** | **10 Days** | **45 Days** | **90 Days** |
| NKT ligand(-) liposome | 1-11 | 0 | 0.997 | 2.89 | 15.9 |
| B7 liposome | 1-1 | 0 | -1.01 | -6.87 | -2.22 |
| α-GC liposome | 1-2 | 0 | 3.70 | -13.1 | -8.50 |

**[Table 4B]**

| **Sample** | | **PDI** | | | |
|---|---|---|---|---|---|
| | **No.** | **0 Days** | **10 Days** | **45 Days** | **90 Days** |
| NKT ligand(-) liposome | 1-11 | 0.120 | 0.176 | 0.230 | 0.420 |
| B7 liposome | 1-1 | 0.170 | 0.153 | 0.245 | 0.190 |
| α-GC liposome | 1-2 | 0.142 | 0.116 | 0.364 | 0.308 |

As shown in the aforementioned tables, the change rate of the particle size of the B7 liposome from 10 Days to 90 Days remained in the range of -1.01% to -6.87% relative to Day 0 (0%), and the change rate of the particle size of α-GalCer from 10 Days to 90 Days also remained in the range of -13.1% to +3.70% relative to Day 0 (0%). On the other hand, the change rate of the particle size of the control NKT ligand(-) liposome from 10 Days to 90 Days increased over time to +0.997% to +15.9% relative to Day 0 (0%). Furthermore, as shown in the aforementioned tables, the PDI of the B7 liposome from 10 Days to 90 Days remained within a variation in the range of 0.153 to 0.245 relative to Day 0 (0.170), and the PDI of α-GalCer from 10 Days to 90 Days also remained within a variation in the range of 0.116 to 0.364 relative to Day 0 (0.142). In contrast, the PDI of the control NKT ligand(-) liposome from 10 Days to 90 Days increased over time to a range of 0.176 to 0.420 relative to Day 0 (0.120). From these results, it was found that the B7 liposome and the α-GC liposome can stabilize the liposomes by using cholesterol and the PEGylated phospholipid (PEG-DSPE), and are, for example, more stable than the control liposome of NKT ligand(-) during long-term storage of 90 days, and can prevent, for example, the formation of aggregates of 1000 nm or more.

### (2) Stability after dilution

The B7 liposome sample (Day 14) at 14 days after the start of storage in the aforementioned (1) was diluted with PBS at various dilution factors (2-fold to 1024-fold) to prepare a plurality of diluted samples. Then, for the undiluted sample that had not been diluted after storage and the diluted samples, the stability was evaluated from the particle size (Z-average hydrodynamic diameter (Z-average)) and the polydispersity index (PDI).

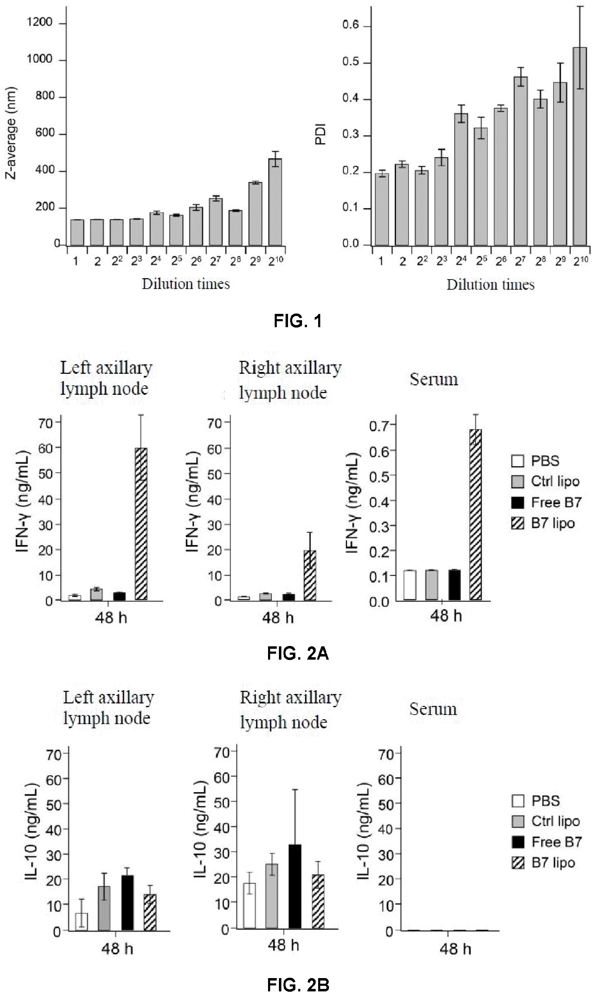

The results of the B7 liposome are shown in FIG. 1. FIG. 1 is a graph showing the results of the dilution stability, in which the left figure shows the particle size of the diluted samples of the B7 liposome, and the right figure shows the PDI of the diluted samples of the B7 liposome. As shown in the figure, in the B7 liposome, the formation of aggregates having a particle size exceeding 1000 nm was not confirmed even when, for example, the B7 liposome was diluted approximately 1000-fold (1/2¹⁰).

### [Example 3]

The function of the PEGylated liposomes prepared in Example 1 was confirmed by administering the same.

### (1) Immune activation ability

The immune activation ability of the PEGylated liposomes was confirmed.

The B7 liposome (sample No. 1-1) and the NKT ligand(-) liposome (sample No. 1-11) prepared in Example 1 were each adjusted to a PEGylated liposome concentration of 1 mg/mL (in terms of total lipids), and used as administration samples. PBS was used for the adjustment of the PEGylated liposome concentration. As control samples for administration, free B7 not formulated into a liposome and PBS were respectively used.

The administration sample was subcutaneously administered to the left flank of a BALB/c mouse (6 weeks old, female). The mice 48 hours after administration were sacrificed, and blood and lymph nodes were collected. The collected lymph nodes were the right accessory axillary lymph node, the right proper axillary lymph node, the left accessory axillary lymph node, and the left proper axillary lymph node. The collected blood was centrifuged at 2000 g for 15 minutes to recover serum. The right accessory axillary lymph node and the right proper axillary lymph node were collectively referred to as the right axillary lymph node, and the left accessory axillary lymph node and the left proper axillary lymph node were collectively referred to as the left axillary lymph node. The weight of the collected lymph nodes was measured, PBS was added so as to give 50 µg/µL, and the mixture was homogenized with a Dounce homogenizer. The homogenized mixture was centrifuged at 2000 g for 15 minutes, and the supernatant was recovered. Using the serum and the lymph node supernatant as samples, IFN-γ and IL-10 in the samples were measured using an IFN-γ ELISA kit (BioLegend, catalog number 430804) in accordance with the manufacturer's instructions.

The results are shown in FIG. 2A. FIG. 2A is a graph showing the IFN-γ concentration in the serum and the lymph node supernatant, and FIG. 2B is a graph showing the IL-10 concentration in the serum and the lymph node supernatant. In FIGS. 2A and 2B, the left is the result of the left axillary lymph node supernatant, the middle is the result of the right axillary lymph node supernatant, and the right is the result of the serum. In each graph of FIG. 2A, the vertical axis is the IFN-γ concentration of the sample (ng/mL), and in each graph of FIG. 2B, the vertical axis is the IL-10 concentration of the sample (ng/mL). In FIGS. 2A and 2B, the bars are, from left, the results of administering PBS, the NKT ligand(-) liposome (0% Ctrl lipo-OH(-) 100 nm), free B7 (Free B7), and the B7 liposome (50% B7 lipo-OH(-) 100 nm).

As shown in FIG. 2A, by administering the B7 liposome, a marked increase in IFN-γ was confirmed in the left axillary lymph node supernatant at 48 hours after administration. In contrast, when B7 was administered in a free form, it was at the same level as the control such as PBS, and no immune activation was observed. Furthermore, in the right axillary lymph node supernatant, although lower than in the left axillary lymph node, an increase in IFN-γ was confirmed. This indicates that not only the regional lymph node of the administered left flank but also the lymph nodes of the whole body can be activated, and it was found that a therapeutic effect on the whole body can be expected. Furthermore, it is considered that the B7 liposome accumulates in the lymph node at about 24 hours after administration, is activated at about 48 hours after administration, and acts on the cancer at about 72 hours after administration. Therefore, for example, a decrease in IFN-γ in the lymph node can be expected at about 96 hours after administration; however, in that case, the continuous effect can be maintained by administering the B7 liposome again at 4 to 5 days after the first administration.

Furthermore, as shown in FIG. 2A, in the serum, even when the B7 liposome was administered, the immune activation was lower than the immune activation in the lymph node. From this, it was found that the administration of the B7 liposome exhibits, for example, markedly higher immune activation in the lymph node compared with the spleen or other immune organs.

It is known that repetitive administration of α-GalCer causes an anergy effect, resulting in immune regulation (Sci. Rep., 2018, vol. 8, p. 8225). Furthermore, it is also known that such immune regulation is associated with the secretion of IL-10 (JCI, 2014, vol. 124(9), p. 3725), and the suitability of repetitive administration of an immune-activating substance can be determined by measuring the amount of IL-10. Therefore, when IL-10 was examined, as shown in FIG. 2B, as a result of subcutaneously administering the B7 liposome, no increase in IL-10 was observed at 48 hours after administration.

Thus, since no increase in the secretion of IL-10 was observed even by the subcutaneous administration of the B7 liposome, it was found that the B7 liposome can suppress the anergy effect. Furthermore, from these results, it is considered that the problem in the repetitive administration of conventional NKT-activating ligands, namely, the problem that the effect thereof decreases with repetitive administration, can also be avoided.

### (2) In vitro immune activity

After adding the PEGylated liposomes to dendritic cells, the dendritic cells were co-cultured with lymphocytes or splenocytes, and changes in the number of NKT cells were examined.

The B7 liposome (sample No. 1-1) prepared in Example 1 was adjusted to a PEGylated liposome concentration of 1 mg/mL (in terms of total lipids), and used as an administration sample. PBS was used for the adjustment of the PEGylated liposome concentration. As a control sample for administration, PBS was used.

Lymphocytes and splenocytes were prepared as follows. First, the spleen and lymph nodes were collected from a BALB/c mouse (7 weeks old, female). The collected lymph nodes were the right accessory axillary lymph node, the right proper axillary lymph node, the left accessory axillary lymph node, and the left proper axillary lymph node. Each of the collected lymph nodes was minced and placed in a cell strainer, and gently ground in RPMI containing 1% FBS to obtain single cells. The single cells were centrifuged at 400 g for 4 minutes to recover only viable lymphocytes. The spleen was similarly made into single cells using a cell strainer, and the cells were recovered by centrifugation. 10% RBC Lysis Buffer (10X, ThermoFisher) was added to PBS (containing 1% FBS) so as to give 1×, and the single cells of the spleen were resuspended in this mixture and allowed to stand for 5 minutes to remove red blood cells. Then, the fraction after the removal of the red blood cells was centrifuged at 400 g for 4 minutes to recover viable splenocytes.

DC2.4 (Sigma-Aldrich), which is a mouse dendritic cell, was cultured in RPMI. To each well of a 6-well plate, fresh RPMI and 5×10⁵ of the cultured dendritic cells were seeded, the B7 liposome was added, and the cells were cultured for 24 hours. The B7 liposome was added so as to give 0.1 mg/mL in terms of final total lipids in each well. Then, after 24 hours of culture, further 7.5×10⁵ of the lymphocytes or the splenocytes per well were added, and the cells were co-cultured for 24 hours. After 24 hours of co-culture, all the cells were recovered. The recovered cell population was blocked by a treatment at 4°C for 45 minutes using a blocking reagent. As the blocking reagent, Blocking One (Nacalai Tesque) containing CD16/CD32 Monoclonal Antibody (ThermoFisher) was used.

Next, a labeling treatment was performed on the blocked cell population, and the labeling of NK cells and the labeling of T cells were performed simultaneously. For the labeling treatment of NK cells, PBS (containing 1% FBS) containing Anti-mouse NK1.1-InVivo (Selleck) and FlexAble CoraLite^{®} Plus 488 Antibody Labeling Kit for mouse IgG (Proteintech) was used as a reagent, and for the labeling treatment of T cells, PBS (containing 1% FBS) containing PE anti-mouse CD3 Antibody (BioLegend) was used as a reagent. The respective labeling reagents were added simultaneously to the blocked cell population and allowed to stand at 4°C for 45 minutes, whereby the NK cells were labeled and the T cells were simultaneously labeled in the cell population. The labeled cell population was washed three times with PBS (containing 1% FBS) and then measured by flow cytometry (BD FACSAria^{™} III Cell Sorter, BD Biosciences). For the labeling, the staining concentration of each antibody was performed in accordance with the manufacturer's instructions (the same applies hereinafter).

Furthermore, the co-culture and the measurement were performed in the same manner, except that, in place of the B7 liposome, PBS (containing 1% FBS) was added per well in an amount of 1/10 (volume ratio) of the culture solution.

First, the results of the lymph node are shown in FIG. 3. FIG. 3A is a histogram of NKT cells in a lymph node, which is a graph representing the distribution of the number of cells, with respect to NK1.1-positive and CD3-positive cells in the lymph node, in terms of the relative fluorescence intensity indicating CD3-positivity. In FIG. 3A, the X-axis is the relative fluorescence intensity of the labeling reagent against CD3, and the Y-axis is the number of events (number of cells) showing the fluorescence. In FIG. 3A, among the two peaks, the right peak shows NK1.1-positive and CD3-positive cells, that is, NKT cells. FIG. 3B is a graph in which, based on FIG. 3A, for three administration groups, the amount of NK1.1-positive and CD3-positive NKT cells in the lymph node was quantified at n=4 (mice).

As shown in FIG. 3A, in the B7 liposome group (B7 lipo), the amount of NK1.1-positive and CD3-positive NKT cells (the right peak in FIG. 3A) increased by co-culturing the dendritic cells, to which the B7 liposome had been administered, and the lymphocytes, compared with the group to which PBS had been administered (PBS).

Next, the results of the spleen are shown in FIG. 4. FIG. 4A is a histogram of NKT cells in a spleen, which is a graph representing the distribution of the number of cells, with respect to NK1.1-positive and CD3-positive cells in the spleen, in terms of the relative fluorescence intensity. In FIG. 4A, the X-axis is the relative fluorescence intensity of the labeling reagent against CD3, and the Y-axis is the number of events (number of cells) showing the fluorescence. In FIG. 4A, among the two peaks, the right peak shows NK1.1-positive and CD3-positive cells, that is, NKT cells. FIG. 4B is a graph in which the amount of NK1.1-positive and CD3-positive NKT cells in the spleen was quantified at n=4 (mice).

As shown in FIG. 4A, also for the spleen, in the same manner as for the lymph node, in the B7 liposome (B7 lipo) group, the amount of NK1.1-positive and CD3-positive NKT cells (the right peak in FIG. 4A) increased compared with the group to which PBS had been administered (PBS).

### (3) Changes in the number of immune cells

Changes in the number of immune cells in the body after administration of the PEGylated liposomes were examined.

The B7 liposome (sample No. 1-1) prepared in Example 1 was adjusted to a PEGylated liposome concentration of 1 mg/mL (in terms of total lipids), and used as an administration sample. As a control sample for administration, PBS was used (NKT ligand not added).

The administration sample was subcutaneously administered to the left flank of a BALB/c mouse (6 weeks old, female). The mice after a predetermined time (12, 24, 48, or 72 hours) from administration were sacrificed, and the spleen and the lymph nodes were collected as organs. The collected lymph nodes were the right accessory axillary lymph node, the right proper axillary lymph node, the left accessory axillary lymph node, and the left proper axillary lymph node. The size of each collected organ was observed. Each of the collected lymph nodes was minced and placed in a cell strainer, and gently ground in RPMI containing 1% FBS to obtain single cells. The single cells were centrifuged at 400 g for 4 minutes to recover only viable lymphocytes. The spleen was similarly made into single cells using a cell strainer, and the cells were recovered by centrifugation. 10% RBC Lysis Buffer (10X, ThermoFisher) was added to PBS (containing 1% FBS) so as to give 1×, and the splenocytes were resuspended in this mixture and allowed to stand for 5 minutes to remove red blood cells. The fraction after the removal of the red blood cells was centrifuged at 400 g for 4 minutes to recover viable splenocytes.

For each of the recovered lymphocyte population and the splenocyte population, blocking and simultaneous labeling of NK cells and T cells were performed by the same method as in the aforementioned (2). The labeled cells were washed and measured by flow cytometry in the same manner as in the aforementioned (2).

Furthermore, for each of the recovered lymphocyte population and the splenocyte population, blocking was performed by the same method as in the aforementioned (2), and dendritic cells were further labeled. For the labeling of dendritic cells, PBS (containing 1% FBS) containing FITC anti-mouse CD11c Antibody (BioLegend) was used as a reagent. That is, the reagent was added to the cell population and allowed to stand at 4°C for 45 minutes, whereby the dendritic cells in the cell population were labeled. Simultaneously, macrophage labeling was further performed on each of the cell populations on which dendritic cell labeling had been performed. For the labeling of macrophages, PBS (containing 1% FBS) containing Anti-mouse CD68 (Miltenyi) and FlexAble CoraLite^{®} Plus 555 Antibody Labeling Kit for rabbit IgG (Proteintech) was used as a reagent. That is, the reagent was added to the cell population and allowed to stand at 4°C for 45 minutes, whereby the macrophages were labeled simultaneously with the dendritic cell labeling. The labeled cells were washed and measured by flow cytometry in the same manner as in the aforementioned (2).

First, changes in the ratio of the numbers of T cells, NK cells, and NKT cells in the lymph node are shown in FIG. 5. FIG. 5A is a histogram of NKT cells in a lymph node, which is a graph representing the distribution of the number of cells, with respect to NK1.1-positive and CD3-positive cells in the lymph node, in terms of the relative fluorescence intensity indicating CD3-positivity. In FIG. 5A, the X-axis is the relative fluorescence intensity of the labeling reagent against CD3, and the Y-axis is the number of events (number of cells) showing the fluorescence. In FIG. 5A, the time (h) is the treatment time after administration of the administration sample, and the histograms of the respective administration groups are shown. FIG. 5B is a histogram of NK cells in the lymph node, which is a graph representing the distribution of the number of cells, with respect to NK1.1-positive NK cells in the lymph node, in terms of the relative fluorescence intensity indicating NK1.1-positivity. In FIG. 5B, the histograms of five administration groups are shown in an overlaid manner. FIG. 5C is a histogram of T cells in the lymph node, which is a graph representing the distribution of the number of cells, with respect to CD3-positive T cells in the lymph node, in terms of the relative fluorescence intensity indicating CD3-positivity. In FIG. 5C, the histograms of five administration groups are shown in an overlaid manner. FIG. 5D is a histogram of NKT cells in the lymph node, in which the histograms of the five administration groups in FIG. 5A are shown in an overlaid manner. In the lines of FIGS. 5B, 5C, and 5D, the darker the color, the longer the treatment time after administration of the administration sample. FIG. 5E is a graph in which, based on FIG. 5A, for the five administration groups, the amount of NK1.1-positive and CD3-positive NKT cells in the lymph node was quantified at n=4 (mice). Specifically, with reference to the relative fluorescence intensity at which the ratio of NKT cells in the lymphocytes of the PBS administration group in FIG. 5A is 5%, the graph shows the ratio (%) of NKT cells of the other administration groups at the same relative fluorescence intensity.

As shown in FIGS. 5A, 5D, and 5E, in the B7 liposome group, the number of NKT cells in the lymph node was confirmed to increase over time at 12 hours after administration and 24 hours after administration, and at 48 hours after administration and 72 hours after administration, it was confirmed that the increased number of NKT cells was maintained. Furthermore, as shown in FIGS. 5B and 5C, the numbers of NK cells and T cells were similarly confirmed to increase over time after administration, reach a peak at 24 hours to 48 hours after administration, and thereafter, those cell numbers were maintained. From these results, it was found that, in vivo, by administering the B7 liposome, the immune activity gradually occurs between 12 hours and 24 hours after administration, and is maintained until 72 hours after administration.

Next, changes in the ratio of the numbers of T cells, NK cells, and NKT cells in the spleen are shown in FIG. 6. FIG. 6A is a histogram of NKT cells in a spleen, which is a graph representing the distribution of the number of cells, with respect to NK1.1-positive and CD3-positive cells in the spleen, in terms of the relative fluorescence intensity indicating CD3-positivity. In FIG. 6A, the X-axis is the relative fluorescence intensity of the labeling reagent against CD3, and the Y-axis is the number of events (number of cells) showing the fluorescence. In FIG. 6A, the time (h) is the treatment time after administration of the administration sample, and the histograms of the respective administration groups are shown. FIG. 6B is a histogram of NK cells in the spleen, which is a graph representing the distribution of the number of cells, with respect to NK1.1-positive NK cells in the spleen, in terms of the relative fluorescence intensity indicating NK1.1-positivity. In FIG. 6B, the histograms of five administration groups are shown in an overlaid manner. FIG. 6C is a histogram of T cells in the spleen, which is a graph representing the distribution of the number of cells, with respect to CD3-positive T cells in the spleen, in terms of the relative fluorescence intensity indicating CD3-positivity. In FIG. 6C, the histograms of five administration groups are shown in an overlaid manner. FIG. 6D is a histogram of NKT cells in the spleen, in which the histograms of the five administration groups in FIG. 6A are shown in an overlaid manner. In the lines of FIGS. 6B, 6C, and 6D, the darker the color, the longer the treatment time after administration of the administration sample. FIG. 6E is a graph in which, based on FIG. 6A, for the five administration groups, the amount of NK1.1-positive and CD3-positive NKT cells in the spleen was quantified at n=4 (mice). Specifically, with reference to the relative fluorescence intensity at which the ratio of NKT cells in the splenocytes of the PBS administration group in FIG. 6A is 5%, the graph shows the ratio (%) of NKT cells of the other administration groups at the same relative fluorescence intensity.

As shown in FIGS. 6A, 6D, and 6E, in the B7 liposome group, the number of NKT cells in the spleen was confirmed to increase over time until 72 hours after administration. Furthermore, as shown in FIG. 6E, although an increase in NKT cells was confirmed at 12 hours after administration in the B7 liposome administration group compared with the PBS administration group, there was no significant difference, and since the number of NKT cells continuously increased until 72 hours after administration in the B7 liposome administration group, it was suggested that the immune activity in the spleen occurs more slowly than in the lymph node and continues for a longer time than in the lymph node. This is considered to be because, for example, the drug administered subcutaneously was first absorbed into the adjacent lymph node and then migrated to the spleen.

As shown in FIG. 6B, the number of NK cells was also confirmed to increase over time after administration, similarly to NKT cells. On the other hand, as shown in FIG. 6C, regarding the number of T cells, CD3-positive cells (the right peak) decreased over time, and CD3-negative cells (the left peak) increased over time and reached a peak at 48 hours after administration. From this, it was found that along with the immune activity in the spleen, the ratio of the number of T cells decreased. This is considered to be because the immune activity caused by the initial administration of the B7 liposome was innate immune activity, and mainly an increase in NK cells and NKT cells was performed.

Next, changes in the ratio of the numbers of dendritic cells and macrophages in the lymph node are shown in FIG. 7. FIG. 7A is a histogram of dendritic cells in a lymph node, which is a graph showing the distribution of the number of cells, with respect to CD11c-positive cells in the lymph node, in terms of the relative fluorescence intensity indicating CD11c-positivity. In FIG. 7A, the X-axis is the relative fluorescence intensity of the labeling reagent against CD11c, and the Y-axis is the number of events (number of cells) showing the fluorescence. FIG. 7B is a histogram of dendritic cells in the lymph node, in which the histograms of the five administration groups in FIG. 7A are shown in an overlaid manner. FIG. 7C is a histogram of macrophages in the lymph node, which is a graph showing the distribution of the number of cells, with respect to CD68-positive cells in the lymph node, in terms of the relative fluorescence intensity indicating CD68-positivity. In FIG. 7C, the X-axis is the relative fluorescence intensity of the labeling reagent against CD68, and the Y-axis is the number of events (number of cells) showing the fluorescence. FIG. 7D is a graph in which, based on FIG. 7B, for the five administration groups, the amount of CD11c-positive dendritic cells in the lymph node was quantified at n=4 (mice). Specifically, with reference to the relative fluorescence intensity at which the ratio of dendritic cells in the lymphocytes of the PBS administration group in FIG. 7B is 5%, the graph shows the ratio (%) of dendritic cells of the other administration groups at the same relative fluorescence intensity. FIG. 7E is a graph in which, based on FIG. 7C, for the five administration groups, the amount of CD68-positive macrophages in the lymph node was quantified at n=4 (mice). Specifically, with reference to the relative fluorescence intensity at which the ratio of macrophages in the lymphocytes of the PBS administration group in FIG. 7C is 5%, the graph shows the ratio (%) of dendritic cells of the other administration groups at the same relative fluorescence intensity.

As shown in FIGS. 7A, 7B, and 7D, in the B7 liposome administration group, the ratio of dendritic cells in the lymph node gradually increased to about twice that of the PBS administration group until 48 hours after administration, and slightly decreased at 72 hours after administration. On the other hand, as shown in FIGS. 7C and 7E, in the B7 liposome administration group, the ratio of macrophages slightly increased after administration of the B7 liposome, but there was no significant difference compared with the PBS administration group.

Next, changes in the ratio of the numbers of dendritic cells and macrophages in the spleen are shown in FIG. 8. FIG. 8A is a histogram of dendritic cells in a spleen, which is a graph showing the distribution of the number of cells, with respect to CD11c-positive cells in the spleen, in terms of the relative fluorescence intensity indicating CD11c-positivity. In FIG. 8A, the X-axis is the relative fluorescence intensity of the labeling reagent against CD11c, and the Y-axis is the number of events (number of cells) showing the fluorescence. FIG. 8B is a histogram of dendritic cells in the spleen, in which the histograms of the five administration groups in FIG. 8A are shown in an overlaid manner. FIG. 8C is a histogram of macrophages in the spleen, which is a graph showing the distribution of the number of cells, with respect to CD68-positive cells in the spleen, in terms of the relative fluorescence intensity indicating CD68-positivity. In FIG. 8C, the X-axis is the relative fluorescence intensity of the labeling reagent against CD68, and the Y-axis is the number of events (number of cells) showing the fluorescence. FIG. 8D is a graph in which, based on FIG. 8B, for the five administration groups, the amount of CD11c-positive dendritic cells in the spleen was quantified at n=4 (mice). Specifically, with reference to the relative fluorescence intensity at which the ratio of dendritic cells in the splenocytes of the PBS administration group in FIG. 8B is 5%, the graph shows the ratio (%) of dendritic cells of the other administration groups at the same relative fluorescence intensity. FIG. 8E is a graph in which, based on FIG. 8C, for the five administration groups, the amount of CD68-positive macrophages in the spleen was quantified at n=4 (mice). Specifically, with reference to the relative fluorescence intensity at which the ratio of macrophages in the splenocytes of the PBS administration group in FIG. 8C is 5%, the graph shows the ratio (%) of dendritic cells of the other administration groups at the same relative fluorescence intensity.

As shown in FIGS. 8A, 8B, and 8D, in the B7 liposome administration group, similarly to the lymph node, the ratio of dendritic cells in the spleen increased to about twice that of the PBS administration group. On the other hand, as shown in FIGS. 8C and 8E, in the B7 liposome administration group, no large change as seen in the dendritic cells was observed in the ratio of macrophages. Thus, in the splenocytes, the increase in dendritic cells was observed more remarkably than the increase in the lymph node.

### [Example 4]

The biodistribution of the PEGylated liposomes prepared in Example 1 was confirmed.

### (1) Biodistribution of B7 liposome

The following liposome samples prepared using the dye-containing PBS in Example 1 were used.
(1-1) 50% B7 / negatively charged PEG terminal -OH liposome, 100 nm
(1-11) 0% control / negatively charged PEG terminal -OH liposome, 100 nm
The liposome samples were adjusted to a PEGylated liposome concentration of 1 mg/mL (in terms of total lipids), and used as administration samples. The dye-containing PBS was used for the adjustment of the PEGylated liposome concentration. The administration sample prepared from the aforementioned (1-1) was used as the B7 liposome administration sample of the Example (B7 lipo), and the administration sample prepared from the aforementioned (1-11) was used as the control liposome administration sample (Ctrl lipo).

30 µL of the administration samples were subcutaneously administered to the left flank of a BALB/c mouse (6 weeks old, female) having breast cancer (4T1, average tumor volume 150 mm³) and a BALB/c mouse (6 weeks old, female) not having breast cancer. The administration amount of each administration sample was as follows. 30 µL of the B7 liposome administration sample: 30 µL/mouse, amount of B7: 15 µg. 30 µL of the control liposome administration sample: 30 µL/mouse. The mice 24 hours after administration were sacrificed, and the left axillary lymph node, the spleen, the kidney, and the liver were collected.

Then, using an IVIS imaging system (trade name IVIS Spectrum, Sumisho Pharma International Corporation) having a fluorescence channel of 650 nm/680 nm, each of the collected tissues was visualized.

The results are shown in FIG. 9. FIG. 9A is an image (converted to black and white) showing the fluorescence in each organ of a mouse 24 hours after administration. In FIG. 9A, the regions indicated by the arrowheads are the regions where the fluorescence intensity was particularly strongly displayed. Since FIG. 9A is a black-and-white display of a color image showing the fluorescence, the color does not indicate the intensity of the fluorescence in the fluorescence region. FIG. 9B is a graph showing the relative fluorescence intensity in each organ of the mouse 24 hours after administration. In FIG. 9B, the relative fluorescence intensity is a value obtained by dividing the fluorescence intensity in each organ by the weight of each organ and normalizing the value of Ctrl lipo to 1.

As shown in FIG. 9A, in the B7 liposome administration group (B7 lipo), strong fluorescence was detected in the left axillary lymph node 24 hours after administration, and the accumulation of a large amount of B7 liposome was confirmed. Fluorescence was also detected in the liver, and the accumulation of a part of the B7 liposome was confirmed. On the other hand, as shown in FIG. 9A, strong fluorescence was not detected in the spleen, the kidney, or other organs (thymus, lung, heart; not shown). From these results, it was found that the B7 liposome enables, in particular, selective delivery to the lymph node. On the other hand, in the control liposome administration group (Ctrl lipo), fluorescence was detected in the liver, but almost no fluorescence was detected in the other organs. Furthermore, also in FIG. 9B, the relative fluorescence intensity in the lymph node was higher in the B7 liposome administration group (B7 lipo) than in the control liposome administration group (Ctrl lipo), and thus it was confirmed that the B7 liposome effectively accumulates in the lymph node.

### [Example 5]

For the PEGylated liposomes prepared in Example 1, the antitumor effect against breast cancer cells was confirmed by repetitive subcutaneous administration.

The following liposome samples prepared using PBS in Example 1 were used.
(1-1) 50% B7 / negatively charged PEG terminal -OH liposome, 100 nm
(1-11) 0% control / negatively charged PEG terminal -OH liposome, 100 nm
The liposome samples were adjusted to a PEGylated liposome concentration of 1 mg/mL (in terms of total lipids), and used as administration samples. PBS was used for the adjustment of the PEGylated liposome concentration. The administration sample prepared from the aforementioned (1-1) was used as the B7 liposome administration sample of the Example (B7 lipo), and the administration sample prepared from the aforementioned (1-11) was used as the control liposome administration sample (Ctrl lipo). In addition, a free B7 sample (Free B7) in which free B7 not formulated into a liposome was suspended in PBS, and PBS, were each used as administration samples.

### (1) Antitumor effect

As breast cancer cells, triple-negative breast cancer 4T1 cells were used. 4T1 cells (5.0×10⁵ to 1.0×10⁶ cells/mouse) were subcutaneously inoculated into the vicinity of the second breast (second teat from the top) of the left chest of a BALB/c mouse (5 weeks old, female, n=6 to 7). When the average tumor volume reached 30 to 180 mm³, the mice were randomly divided into groups. Then, according to administration condition A or administration condition B described below, the administration sample was subcutaneously injected (Day 0) into the left flank subcutaneously or under both sides of the mouse. The administration amount of each administration sample was as follows.
30 µL of the B7 liposome administration sample: 30 µL/mouse, amount of B7: 15 µg, total lipid amount: 30 µg
30 µL of the control liposome administration sample: 30 µL/mouse, total lipid amount: 30 µg
30 µL of the free B7 sample: 30 µL/mouse, amount of B7: 15 µg
PBS sample: 30 µL/mouse

Every two days from the start of administration, the growth of the tumors in the mice and the body weight of the mice were monitored. The tumors were measured with calipers, and the tumor volume V was calculated as follows, wherein L is the length of the tumor and W is the width of the tumor.$V = π / 6 \times L \times {W}^{2}$ Furthermore, the survival of the mice was monitored, and the survival rate was analyzed by the Kaplan-Meier method. Mice with a tumor volume exceeding 2000 mm³ were euthanized, and that day was recorded as the date of death of the mouse.

### (Administration condition A)

8 days after the inoculation of the 4T1 cells, the first administration (1st) of the administration sample to the left flank was performed. With this set as Day 0, further subcutaneous injections to the left flank were performed under the same conditions four times every 6 days (5 times in total).

### (A1) Antitumor effect

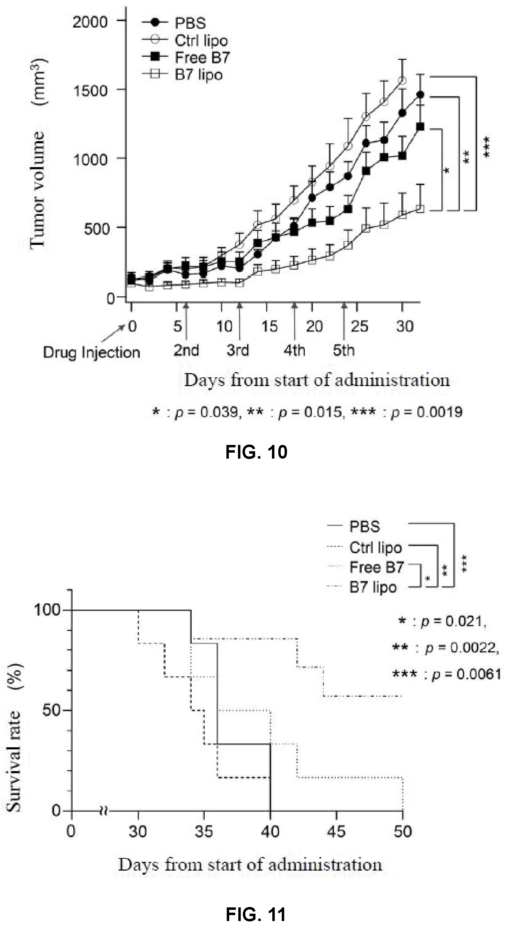

The results of the antitumor effect are shown in FIG. 10. FIG. 10 is a graph showing the relationship between the tumor volume (V) of the mice and the number of days from the start of administration. As shown in FIG. 10, the tumor volume on Day 0 was 100 mm³. Furthermore, as shown in FIG. 10, in the PBS-only administration group and the control liposome administration group (Ctrl lipo), the increase in tumor volume on Day 30 compared with Day 0 was about 12 to 20-fold. Furthermore, in the free B7 administration group (Free B7), the increase in tumor volume on Day 30 compared with Day 0 was about 10-fold, and the increase in tumor was suppressed more than in the PBS administration group and the administration group (Ctrl lipo). In contrast, in the B7 liposome administration group (B7 lipo), the increase in tumor volume on Day 30 compared with Day 0 remained at about 5-fold, and it was confirmed that the increase in tumor volume was markedly suppressed. From this result, it was found that the growth of breast cancer can be effectively suppressed by administering B7 as a PEGylated liposome.

Next, the results of the survival rate are shown in FIG. 11. FIG. 11 is a graph showing the relationship between the survival rate of the mice and the number of days from the start of administration. As shown in FIG. 11, in the B7 liposome administration group, the survival rate of the mice markedly increased, specifically, the survival period was about 4 times longer compared with the PBS administration group. From this result, it is considered that immune activity is caused by the administration of the B7 liposome, whereby the growth of cancer is suppressed, and as a result, the survival period of the mice was extended.

### (A2) Tolerability/safety

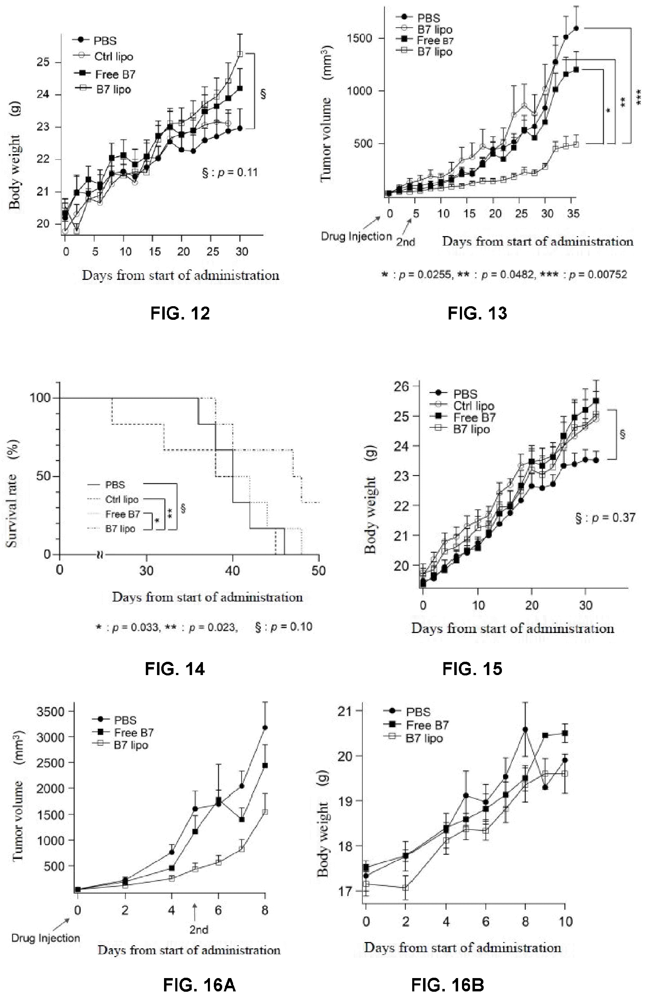

The results of the tolerability and safety are shown in FIG. 12. FIG. 12 is a graph showing the relationship between the body weight of the mice and the number of days from the start of administration. As shown in FIG. 12, compared with the PBS-only administration group, no significant decrease in the body weight of the mice was observed in the control liposome administration group (Ctrl lipo), the free B7 administration group (Free B7), or the B7 liposome administration group (B7 lipo). From this result, it was confirmed that the B7 liposome, the control liposome, and the free B7 do not cause significant side effects on the mice, similarly to PBS.

### (Administration condition B)

5 days after the inoculation of the 4T1 cells, the first administration (1st) of the administration sample to both flanks was set as Day 0, and 5 days later (Day 5), the second subcutaneous injection to both flanks was performed under the same conditions (2 times in total).

### (B1) Antitumor effect

The results of the antitumor effect are shown in FIG. 13. FIG. 13 is a graph showing the relationship between the tumor volume (V) of the mice and the number of days from the start of administration. As shown in FIG. 13, the tumor volume on Day 0 was 36 mm³. Furthermore, as shown in FIG. 13, in the PBS-only administration group and the control liposome administration group (Ctrl lipo), the increase in tumor volume on Day 32 compared with Day 0 was about 30 to 40-fold. Furthermore, in the free B7 administration group (Free B7), the increase in tumor volume on Day 32 compared with Day 0 was about 27-fold, and the increase in tumor was suppressed more than in the PBS administration group and the administration group (Ctrl lipo). In contrast, in the B7 liposome administration group (B7 lipo), the increase in tumor volume on Day 32 compared with Day 0 remained at about 7-fold, and it was confirmed that the increase in tumor volume was markedly suppressed. From this result, it was found that the growth of breast cancer can be effectively suppressed by administering B7 as a PEGylated liposome.

Next, the results of the survival rate are shown in FIG. 14. FIG. 14 is a graph showing the relationship between the survival rate of the mice and the number of days from the start of administration. As shown in FIG. 14, in the B7 liposome administration group, the survival rate of the mice could be markedly maintained compared with the other administration groups.

Since the same results were obtained for the subcutaneous administration of the aforementioned administration condition A and the subcutaneous administration of this administration condition B, it was found that the B7 liposome activates the immune system by subcutaneous administration without being affected by these differences in administration conditions, and thereby exhibits excellent antitumor effect and life-prolonging effect.

### (B2) Tolerability/safety

The results of the tolerability and safety are shown in FIG. 15. FIG. 15 is a graph showing the relationship between the body weight of the mice and the number of days from the start of administration. Furthermore, as shown in FIG. 15, compared with the PBS-only administration group, no significant decrease in the body weight of the mice was observed in the control liposome administration group (Ctrl lipo), the free B7 administration group (Free B7), or the B7 liposome administration group (B7 lipo).

### [Example 6]

For the PEGylated liposomes prepared in Example 1, the antitumor effect against skin cancer cells was confirmed by repetitive subcutaneous administration.

The same administration samples as in the aforementioned Example 5 were used as follows.
30 µL of the B7 liposome administration sample (B7 lipo): 30 µL/mouse, PEGylated liposome concentration 1 mg/mL (in terms of total lipids), amount of B7: 15 µg
30 µL of the control liposome administration sample (Ctrl lipo): 30 µL/mouse, PEGylated liposome concentration 1 mg/mL (in terms of total lipids)
30 µL of the free B7 sample: 30 µL/mouse, amount of B7: 15 µg
PBS sample: 30 µL/mouse

As skin cancer cells, B16F10 was used. B16F10 cells (1.0×10⁶ cells/mouse) were subcutaneously inoculated into the vicinity of the second teat from the top of the left chest of a C57BL/6j mouse (5 weeks old, female, n=6 to 7). Then, in the same manner as in the aforementioned Example 5, 30 µL of the administration sample was administered, and the antitumor effect and safety/tolerability were confirmed.

The results of the antitumor effect are shown in FIG. 16. FIG. 16A is a graph showing the relationship between the tumor volume (V) of the mice and the number of days from the start of administration. FIG. 16B is a graph showing the relationship between the body weight (g) of the mice and the number of days from the start of administration. As shown in FIG. 16A, in the PBS administration group (closed circles) and the free B7 administration group (closed squares), the increase in tumor volume on Day 6 compared with Day 0 was about 40 to 50-fold. In contrast, in the B7 liposome administration group (open squares), the increase in tumor volume on Day 6 compared with Day 0 remained at about 15-fold, and it was confirmed that the increase in tumor volume was markedly suppressed. From this result, it was found that the growth of skin cancer can be effectively suppressed by administering B7 as a PEGylated liposome. Furthermore, as shown in FIG. 16B, in Day 0 to Day 10, no significant decrease in body weight was confirmed in any of the administration groups. From this result, it was confirmed that the B7 liposome does not exhibit serious toxicity, similarly to free B7.

### [Example 7]

For the PEGylated liposomes prepared in Example 1, the antitumor effect against breast cancer cells was confirmed by repetitive subcutaneous administration.

The following liposome samples prepared using PBS in Example 1 were used.
(1-2) 50% α-GalCer / negatively charged PEG terminal -OH liposome, 100 nm
(1-11) 0% control / negatively charged PEG terminal -OH liposome, 100 nm
The liposome samples were adjusted to a PEGylated liposome concentration of 1 mg/mL (in terms of total lipids), and used as administration samples. PBS was used for the adjustment of the PEGylated liposome concentration. The administration sample prepared from the aforementioned (1-2) was used as the α-GalCer liposome administration sample of the Example (αGal lipo), and the administration sample prepared from the aforementioned (1-11) was used as the control liposome administration sample (Ctrl lipo). In addition, a free GalCer sample (Free αGal) in which free α-GalCer not formulated into a liposome was suspended in PBS, and PBS, were each used as administration samples.

As breast cancer cells, triple-negative breast cancer 4T1 cells were used. 4T1 cells (5.0×10⁵ to 1.0×10⁶ cells/mouse) were subcutaneously inoculated into the vicinity of the second teat from the top of the left chest of a BALB/c mouse (5 weeks old, female, n=2). When the average tumor volume reached 40 to 100 mm³, the mice were randomly divided into groups. Then, the administration sample was subcutaneously injected into the left flank of the mouse (Day 0). The administration amount of each administration sample was as follows.
30 µL of the α-GalCer liposome administration sample: 30 µL/mouse, amount of α-GalCer: 15 µg, total lipid amount: 30 µg
30 µL of the control liposome administration sample: 30 µL/mouse, total lipid amount: 30 µg
30 µL of the free α-GalCer sample: 30 µL/mouse, amount of α-GalCer: 15 µg
PBS: 30 µL/mouse

Then, with the first administration (1st) of the administration sample to the left flank set as Day 0, further subcutaneous injections to the left flank were performed under the same conditions three times every 5 days (4 times in total). Then, in the same manner as in the aforementioned Example 5, the antitumor effect and safety/tolerability were confirmed.

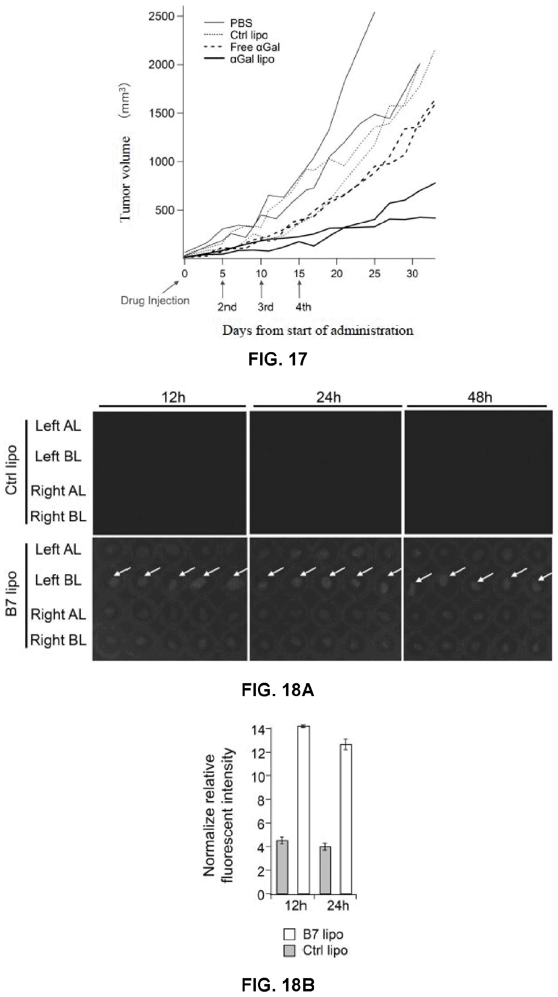

The results of the antitumor effect are shown in FIG. 17. FIG. 17 is a graph showing the relationship between the tumor volume (V) of the mice (n=2 each) of each administration group and the number of days from the start of administration. As shown in FIG. 17, in the PBS administration group (PBS) and the control liposome administration group (Ctrl lip), the tumor volume markedly increased with the lapse of days. Compared with these administration groups, in the free GalCer administration group (Free αGal), the increase in tumor volume was suppressed. On the other hand, in the α-GalCer liposome administration group (αGal lipo), more marked suppression of the increase in tumor volume was shown than in the free GalCer administration group (Free αGal), and significant suppression of the growth of cancer was confirmed. From this result, it was found that the growth of breast cancer can be effectively suppressed by administering α-GalCer as a PEGylated liposome. Although the results of n=2 for each group are shown in FIG. 17, the other mice of each group also showed similar behavior.

### [Example 8]

The PEGylated liposomes prepared in Example 1 were administered to cancer cell-transplanted mice, and the biodistribution was confirmed.

### (1) Biodistribution after administration of B7 liposome in tumor-bearing mice

The following liposome samples prepared using the dye-containing PBS in Example 1 were used.
(1-1) 50% B7 / negatively charged PEG terminal -OH liposome, 100 nm
(1-11) 0% control / negatively charged PEG terminal -OH liposome, 100 nm
The liposome samples were adjusted to a PEGylated liposome concentration of 1 mg/mL (in terms of total lipids), and used as administration samples. The dye-containing PBS was used for the adjustment of the PEGylated liposome concentration. The administration sample prepared from the aforementioned (1-1) was used as the B7 liposome administration sample of the Example (B7 lipo), and the administration sample prepared from the aforementioned (1-11) was used as the control liposome administration sample (Ctrl lipo).

As breast cancer cells, triple-negative breast cancer 4T1 cells were used. To BALB/c mice not having cancer (5 weeks old, female), 4T1 cells (5.0×10⁵ to 1.0×10⁶ cells/mouse) were subcutaneously inoculated into the vicinity of the second teat from the top of the left chest (Day 0). When the average tumor volume reached 100 mm³ (Day 8), 30 µL of the administration sample was subcutaneously injected into the left flank subcutaneously or under both sides of the mouse. The administration amount of each administration sample was as follows.
30 µL of the B7 liposome administration sample: 30 µL/mouse, amount of B7: 15 µg
30 µL of the control liposome administration sample: 30 µL/mouse

At 12 hours, 24 hours, and 48 hours after administration, the mice of each administration group were euthanized, and the lymph nodes, cancer cells, liver, and spleen were excised to confirm the distribution of the B7 liposome in vivo. Unless otherwise specified, the procedure was performed in the same manner as in the aforementioned Example 4.

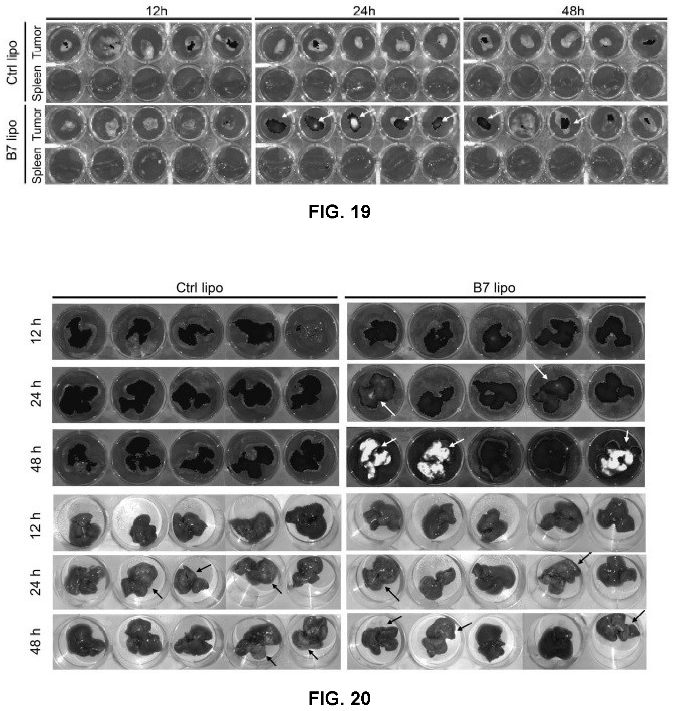

These results are shown in FIGS. 18 to 20. FIG. 18A is an image (converted to black and white) showing the fluorescence in the lymph nodes, wherein AL is the proper axillary lymph node and BL is the accessory axillary lymph node. FIG. 18B is a graph showing the fluorescence intensity in all excised lymph nodes. FIG. 19 is an image (converted to black and white) showing the fluorescence in the cancer of the site where the cancer cells were inoculated and in the spleen. FIG. 20 is an image (converted to black and white) showing the fluorescence in the liver to which the cancer has metastasized. In FIG. 20, the lower figure is a photograph of the collected organ, and the upper figure is a figure in which the fluorescence distribution is overlaid on the photograph. In the images showing fluorescence in FIGS. 18 to 20, the parts having stronger fluorescence intensity in the B7 liposome administration group (B7 lipo) compared with the control administration group (Ctrl lipo) are indicated by white arrows. Furthermore, in the photograph of the liver in the lower figure of FIG. 20, the parts where severe metastasis of cancer has occurred are indicated by black arrows.

As shown in FIGS. 18 and 19, in the control liposome administration group (Ctrl lipo), no fluorescence could be confirmed in the lymph nodes and the cancer, and no localization of the liposome was observed. In contrast, in the B7 liposome administration group (B7 lipo), as shown in FIG. 18 (lymph nodes) and FIG. 19 (cancer), fluorescence was confirmed, and the localization of the B7 liposome was observed. Furthermore, in the B7 liposome administration group, the distribution of the B7 liposome in the lymph nodes was confirmed at 12 to 24 hours after administration (FIG. 18), and the distribution of the B7 liposome in the cancer was confirmed at 24 to 48 hours after administration (FIGS. 19 and 20). Furthermore, in the B7 liposome administration group, in mice in which many cancer metastases to the liver had occurred, the accumulation of the B7 liposome in the liver also became high (the upper figure of FIG. 20, B7 lipo / 48 hours / the first, second, and fifth livers from the left). On the other hand, in the control liposome administration group (Ctrl lipo), no accumulation of the fluorescent dye was observed even in the liver in which metastasis was occurring. From these results, it is suggested that, by containing B7 in the liposome, for example, not non-specific accumulation such as the EPR (Enhanced Permeability and Retention) effect, but a stronger selective interaction than that is caused, and the substances in the liposome (i.e., B7 and the fluorescent dye) are effectively accumulated in the cancer. Then, it is considered that the administered B7 liposome is taken up into the lymph nodes, further taken up into the dendritic cells, and then the dendritic cells that have taken up the B7 liposome migrate to the cancer.

### (2) Distribution of B7 liposome inside cancer cells in tumor-bearing mice and changes in the number of immune cells inside cancer cells

**(2A)** In order to examine which cells delivered the liposome to the cancer when the B7 liposome was administered, the co-localization of the fluorescently labeled liposome with cells in the cancer was measured using flow cytometry.

The same administration samples as in the aforementioned (1) were used, and the administration conditions of each administration sample were as follows.
30 µL of the B7 liposome administration sample: 30 µL/mouse, amount of B7: 15 µg
30 µL of the control liposome administration sample: 30 µL/mouse

As breast cancer cells, triple-negative breast cancer 4T1 cells were used. To BALB/c mice not having cancer (5 weeks old, female, n=4), 4T1 cells (5.0×10⁵ to 1.0×10⁶ cells/mouse) were subcutaneously inoculated into the vicinity of the second teat from the top of the left chest (Day 0). When the average tumor volume reached 100 mm³ (Day 8), 30 µL of the administration sample was subcutaneously injected into the left flank subcutaneously or under both sides of the mouse. At 24 hours after administration, the mice of each administration group were euthanized, and the cancer was excised. The collected cancer was minced and shaken in RPMI containing 0.2% collagenase (Fujifilm Wako) for 30 minutes at 37°C and 500 RPM. Thereafter, these mixtures were placed in a cell strainer and gently ground in RPMI containing 1% FBS to obtain single cells. The single cells were centrifuged at 400 g for 4 minutes to recover only viable cells.

Blocking was performed on the recovered cell population in the same manner as in the aforementioned Example 3 (3). Next, the labeling of NK cells and the labeling of T cells were performed simultaneously on the blocked cell population. For the labeling treatment of NK cells, PBS (containing 1% FBS) containing Anti-mouse NK1.1-InVivo (Selleck) and FlexAble CoraLite^{®} Plus 488 Antibody Labeling Kit for mouse IgG (Proteintech) was used as a reagent, and for the labeling treatment of T cells, PBS (1% FBS) containing PE anti-mouse CD3 Antibody (BioLegend) was used as a reagent. The respective labeling reagents were added simultaneously to the blocked cell population and allowed to stand at 4°C for 45 minutes, whereby the NK cells were labeled and the T cells were simultaneously labeled in the cell population.

Furthermore, to the recovered cell population, PBS (containing 1% FBS) containing FITC anti-mouse CD11c Antibody (BioLegend) was added, and the mixture was allowed to stand at 4°C for 45 minutes, whereby the dendritic cells in the cell population were labeled. Furthermore, to the cell population, PBS (1% FBS) containing Anti-mouse CD68 (Miltenyi) and FlexAble CoraLite^{®} Plus 488 Antibody Labeling Kit for rabbit IgG (Proteintech) was added, and the mixture was allowed to stand at 4°C for 45 minutes, whereby the macrophages in the cell population were labeled.

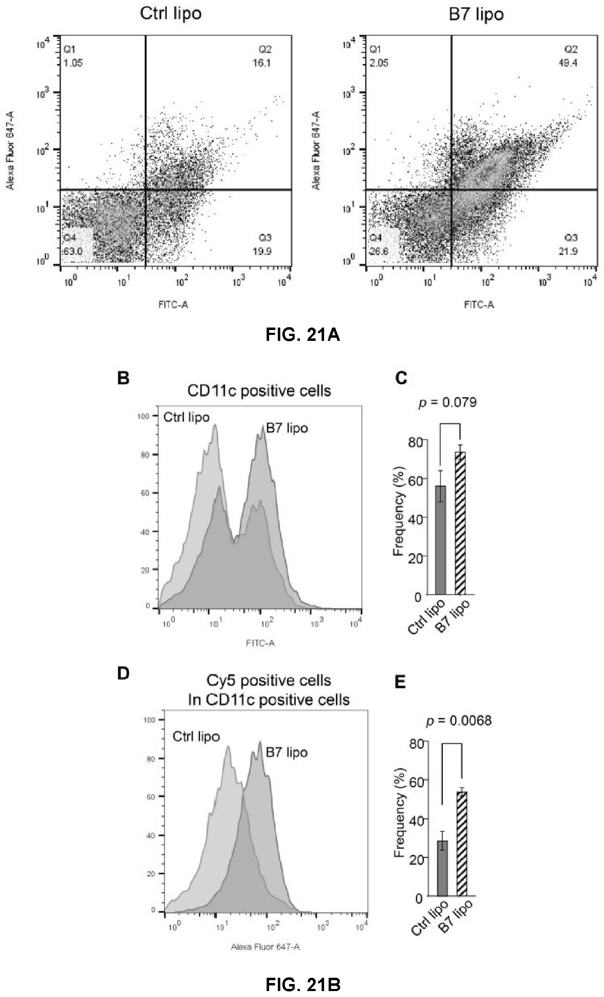

The labeled cell population was washed three times with PBS (containing 1% FBS) and then measured by flow cytometry (BD FACSAria^{™} III Cell Sorter, BD Biosciences). These results are shown in FIGS. 21-25 (n=4).

FIG. 21 shows the results of flow cytometry showing the co-localization of dendritic cells and liposomes in the cancer. The dendritic cells can be detected by flow cytometry by the fluorescence intensity based on the FITC staining against CD11c, and the liposomes can be detected by flow cytometry by the fluorescence intensity based on Cy5 of the aforementioned dye-containing PBS.

FIG. 21A is a graph showing the distribution of cells based on the fluorescence intensity of FITC and the fluorescence intensity of Cy5, wherein the horizontal axis is the fluorescence intensity of FITC and the vertical axis is the fluorescence intensity of Cy5.
FIG. 21B is a graph showing the distribution of cells based on the fluorescence intensity of FITC, wherein the horizontal axis is the fluorescence intensity of FITC and the vertical axis is the ratio of the number of cells. In each administration group, the left peak represents CD11c-negative cells, and the right peak represents CD11c-positive cells (dendritic cells).
FIG. 21C is a graph in which the ratio of the CD11c-positive cells (the right peak) in FIG. 21B was quantified.
FIG. 21D is a graph in which only the CD11c-positive cells (the right peak) in FIG. 21B are gated, and the distribution of the CD11c-positive cells is shown by the Cy5 fluorescence intensity, wherein the horizontal axis is the fluorescence intensity of Cy5 and the vertical axis is the ratio of the number of cells.
FIG. 21E is the result of quantifying the ratio of CD11c-positive and Cy5-positive cells in FIG. 21D.

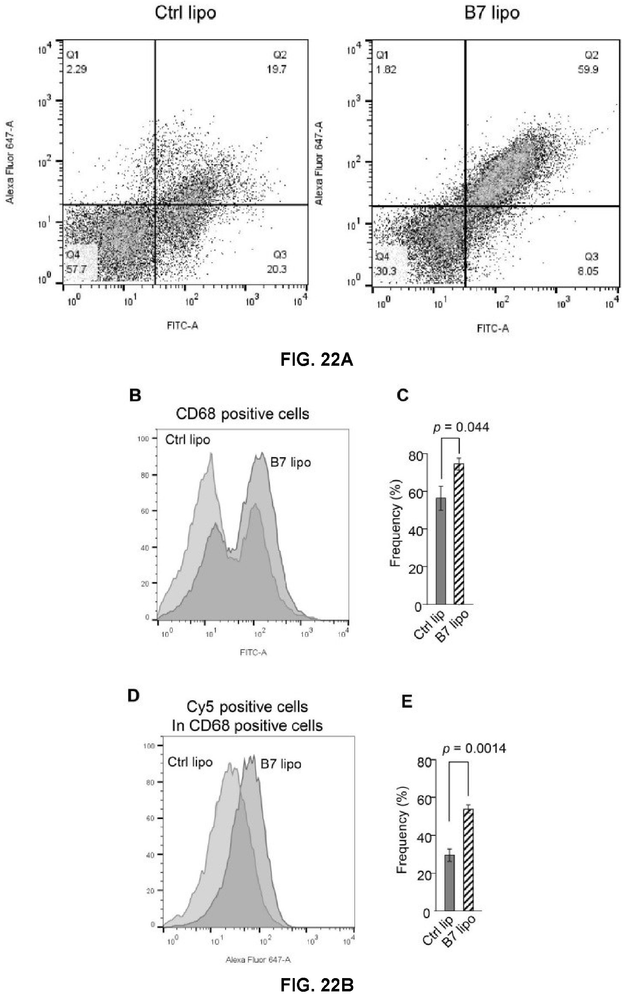

FIG. 22 shows the results of flow cytometry showing the co-localization of macrophages and liposomes in the cancer. The macrophages can be detected by flow cytometry by the fluorescence intensity based on the FITC staining against CD68, and the liposomes can be detected by flow cytometry by the fluorescence intensity based on Cy5 of the aforementioned dye-containing PBS.

FIG. 22A is a graph showing the distribution of cells based on the fluorescence intensity of FITC and the fluorescence intensity of Cy5, wherein the horizontal axis is the fluorescence intensity of FITC and the vertical axis is the fluorescence intensity of Cy5.
FIG. 22B is a graph showing the distribution of cells based on the fluorescence intensity of FITC, wherein the horizontal axis is the fluorescence intensity of FITC and the vertical axis is a graph showing the ratio of the number of cells. In each administration group, the left peak represents CD68-negative cells, and the right peak represents CD68-positive cells (macrophages).
FIG. 22C is a graph in which the ratio of the CD68-positive cells (the right peak) in FIG. 22B was quantified.
FIG. 22D is a graph in which only the CD68-positive cells in FIG. 22B are gated, and the distribution of the CD68-positive cells is shown by the Cy5 fluorescence intensity, wherein the horizontal axis is the fluorescence intensity of Cy5 and the vertical axis is the ratio of the number of cells.
FIG. 22E is the result of quantifying the ratio of CD68-positive and Cy5-positive cells in FIG. 22D.

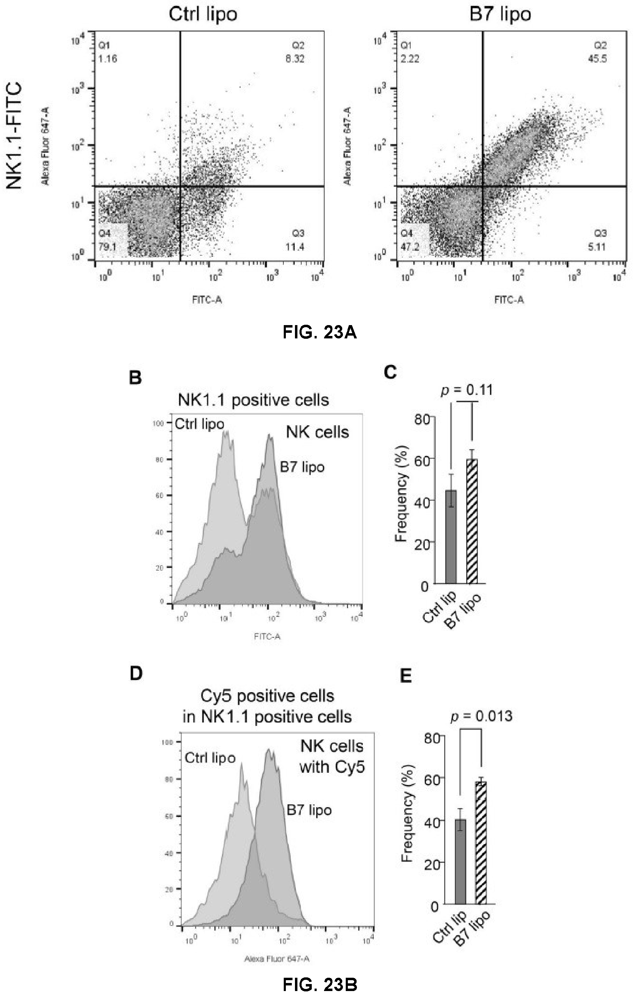

FIG. 23 shows the results of flow cytometry showing the co-localization of NK cells and liposomes in the cancer. The NK cells can be detected by flow cytometry by the fluorescence intensity based on the FITC staining against NK1.1, and the liposomes can be detected by flow cytometry by the fluorescence intensity based on Cy5 of the aforementioned dye-containing PBS.

FIG. 23A is a graph showing the distribution of cells based on the fluorescence intensity of NK1.1 and the fluorescence intensity of Cy5, wherein the horizontal axis is the fluorescence intensity of NK1.1 and the vertical axis is the fluorescence intensity of Cy5.
FIG. 23B is a graph showing the distribution of cells based on the fluorescence intensity of FITC, wherein the horizontal axis is the fluorescence intensity of NK1.1 and the vertical axis is the ratio of the number of cells. In each administration group, the left peak represents NK1.1-negative cells, and the right peak represents NK1.1-positive cells (NK cells).
FIG. 23C is a graph in which the ratio of the NK1.1-positive cells (the right peak) in FIG. 23B was quantified.
FIG. 23D is a graph in which only the NK1.1-positive cells (the right peak) in FIG. 23B are gated, and the distribution of the NK1.1-positive cells is shown by the Cy5 fluorescence intensity, wherein the horizontal axis is the fluorescence intensity of Cy5 and the vertical axis is the ratio of the number of cells.
FIG. 23E is the result of quantifying the ratio of NK1.1-positive and Cy5-positive cells in FIG. 23D.

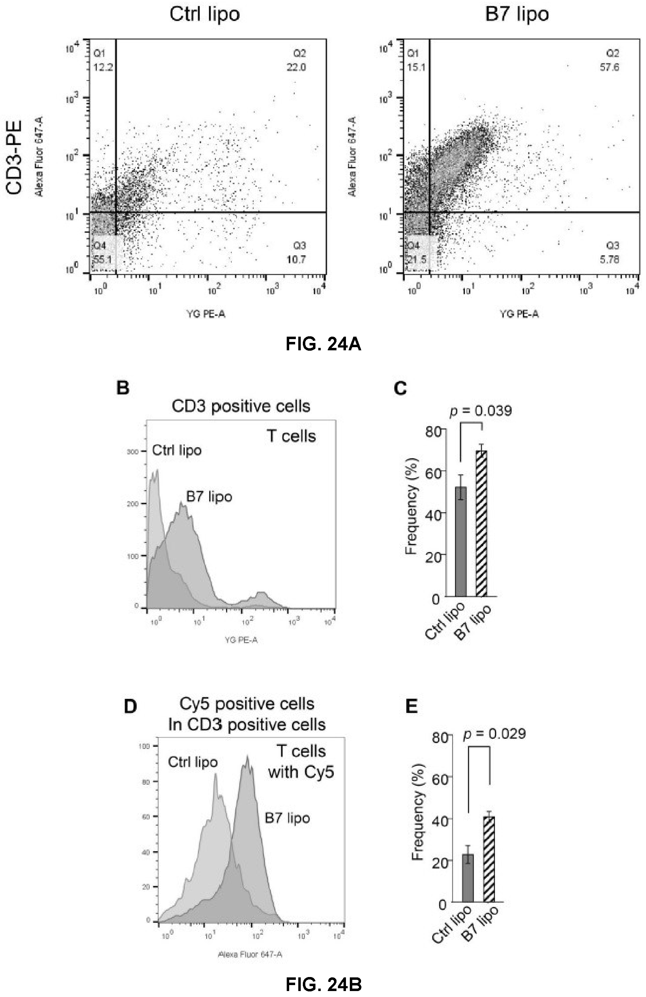

FIG. 24 shows the results of flow cytometry showing the co-localization of T cells and liposomes in the cancer. The T cells can be detected by flow cytometry by the fluorescence intensity based on the PE staining against CD3, and the liposomes can be detected by flow cytometry by the fluorescence intensity based on Cy5 of the aforementioned dye-containing PBS.

FIG. 24A is a graph showing the distribution of cells based on the fluorescence intensity of FITC and the fluorescence intensity of Cy5, wherein the horizontal axis is the fluorescence intensity of CD3 and the vertical axis is the fluorescence intensity of Cy5.
FIG. 24B is a graph showing the distribution of cells based on the fluorescence intensity of FITC, wherein the horizontal axis is the fluorescence intensity of FITC and the vertical axis is the ratio of the number of cells. In each administration group, the left peak represents CD3-negative cells, and the right peak represents CD3-positive cells (T cells).
FIG. 24C is a graph in which the ratio of the CD3-positive cells (the right peak) in FIG. 24B was quantified.
FIG. 24D is a graph in which only the CD3-positive cells (the right peak) in FIG. 24B are gated, and the distribution of the CD3-positive cells is shown by the Cy5 fluorescence intensity, wherein the horizontal axis is the fluorescence intensity of Cy5 and the vertical axis is the ratio of the number of cells.
FIG. 24E is the result of quantifying the ratio of CD3-positive and Cy5-positive cells in FIG. 24D.

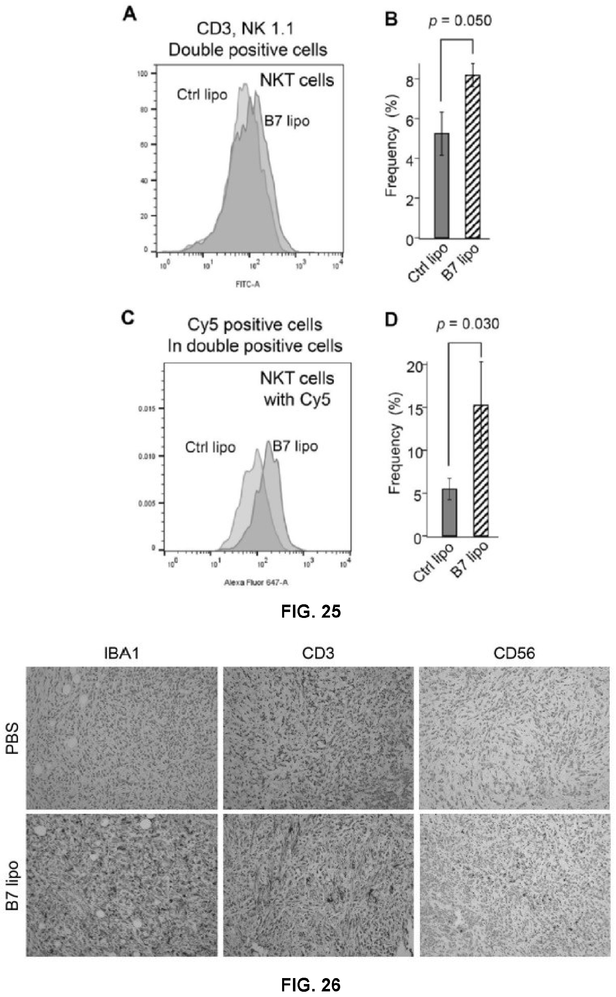

FIG. 25 shows the results of flow cytometry showing the co-localization of CD3-positive and NK1.1-positive double-positive cells (NKT cells) and liposomes in the cancer.
FIG. 25A is a graph showing the distribution of CD3-positive and NK1.1-positive double-positive cells (NKT cells) based on the FITC fluorescence intensity, wherein the horizontal axis is the fluorescence intensity of FITC and the vertical axis is the ratio of the number of cells.
FIG. 25B is the result of quantifying the ratio of CD3-positive and NK1.1-positive double-positive cells (NKT cells) in FIG. 25A.
FIG. 25C is a graph showing the distribution of the CD3-positive and NK1.1-positive double-positive cells (NKT cells) in FIG. 25B based on the Cy5 fluorescence intensity.
FIG. 25D is the result of quantifying the ratio of CD3-positive / NK1.1-positive / Cy5-positive cells in FIG. 25C.

First, as shown in FIGS. 21A, 21B, and 21C regarding dendritic cells, in the B7 liposome administration group (B7 lipo), the number of dendritic cells in the cancer increased compared with the control liposome administration group (Ctrl lipo). Furthermore, as shown in FIGS. 21D and 21E, in the B7 liposome administration group (B7 lipo), it was confirmed that, by administering B7 as a B7 liposome, the co-localization of dendritic cells (CD11c-positive) and liposomes (Cy5-positive) increased more than in the control liposome administration group (Ctrl lipo). In particular, in the B7 liposome administration group (B7 lipo), the ratio of the number of dendritic cells in the cancer increased by about 20% compared with the control liposome administration group (Ctrl lipo), whereas the number of dendritic cells containing the liposome (CD11c-positive and Cy5-positive cells) increased by about 100%. From this result, it is presumed that the dendritic cells delivered the liposomes to the cancer. Furthermore, the results regarding macrophages in FIGS. 22A, 22B, 22C, and 22E were similar to those of FIG. 21 regarding dendritic cells described above. From these facts, it is presumed that the antigen-presenting phagocytic cells such as dendritic cells and macrophages delivered the liposomes to the cancer.

Similarly, as shown in FIG. 23 (23A to 23E), FIG. 24 (24A to 24E), and FIG. 25 (25A to 25E) regarding NK cells, T cells, and NKT cells, an increase in the number of cells in the cancer and the number of cells containing the liposome (Cy5-positive cells) was also confirmed for these cells. In particular, for the NKT cells shown in FIG. 25, in the B7 liposome administration group (B7 lipo), the number of cells containing the liposome in the cancer (Cy5-positive cells) showed an increase of 200% compared with the control liposome administration group (Ctrl lipo), showing a markedly high increase rate. However, since T cells, NK cells, and NKT cells do not have phagocytic action, it is unlikely that these cells directly took up the liposomes. Therefore, it is presumed that the aforementioned phagocytic cells (dendritic cells and macrophages) took up the liposomes, absorbed the Cy5 contained therein, and in this state, interacted with T cells, NK cells, and NKT cells, and Cy5 was transferred from the phagocytic cells to T cells, NK cells, and NKT cells. In particular, based on the increase in fluorescence intensity in the lymph nodes shown in the aforementioned FIG. 9, the increase in dendritic cells and NKT cells in the lymph nodes at 12 to 24 hours after administration shown in FIGS. 5-8, and the result of no increase in macrophages, the following hypothesis can be further established. That is, when the B7 liposome is administered, the B7 liposome is taken up into the lymph nodes and is phagocytosed and absorbed by the dendritic cells. Then, in the lymph nodes, the dendritic cells perform antigen presentation of B7, which activates NKT cells. At this time, Cy5 that has been taken up into the dendritic cells is transferred to the NKT cells. Thereafter, it is considered that the activated immune system (e.g., NKT cells, dendritic cells, etc.) migrates to the cancer and induces the anticancer action.

### (2B)

The cancer excised in the aforementioned (2A) was fixed with 20% formaldehyde for 48 hours, the fixed tissue was paraffin-embedded, and 5 µm sections were prepared. The paraffin embedding was performed in accordance with the equipment and the protocol provided by Abcam. The sections were placed on a slide glass and deparaffinized (treated in the order of xylene, ethanol, and water), and antigen activation was performed by treating the section slide with a citrate buffer (pH 6.0) at 98°C for 20 minutes.

For the section slide, blocking was performed at 4°C for 30 minutes in the same manner as in the aforementioned (2A). Next, primary antibody staining was performed on the blocked sections, followed by secondary antibody staining. For the primary antibody staining, PBS (containing 1% FBS) containing CD56 (NCAM) Antibodies (ThermoFisher), PBS (containing 1% FBS) containing IBA1 Recombinant Rabbit Monoclonal Antibody (ThermoFisher), or PBS (containing 1% FBS) containing CD3 Antibodies (ThermoFisher) was used as a reagent, and the treatment condition was incubation at 25°C for 45 minutes. Furthermore, the secondary antibody staining was performed using Goat anti-Rabbit IgG (H+L) Secondary Antibody, HRP (ThermoFisher) as a reagent, and the treatment condition was incubation at room temperature for 30 minutes to 1 hour. Then, AEC ((3-Amino-9-ethylcarbazole), Peroxidase substrate) substrate solution (Abcam) was added to the section slide and allowed to stand for 5 minutes to stain the target protein red. Thereafter, distilled water was added to the section slide to stop the staining reaction, and the section slide was further subjected to hematoxylin staining to stain the nuclei blue. The stained tissue on the section slide was observed with a microscope.

These results are shown in FIG. 26. FIG. 26 shows micrographs of the section slides, specifically, photographs in which macrophages, NK cells, and T cells were visualized by staining of the marker proteins IBA1, CD56, and CD3, respectively. As shown in FIG. 26, compared with the PBS administration group, in the B7 liposome administration group (B7 lipo), the numbers of macrophages, NK cells, and T cells in the cancer increased. This suggests that the administration of the B7 liposome activated the entire immune system, which influenced the anticancer action.

### [Example 9]

For the various B7 liposomes prepared in Example 1, the influence of changes in the conditions on the biodistribution and the immune activity was confirmed.

### (1) Influence of changes in size of B7 liposome on uptake into dendritic cells in lymph node

### (1A)

The following liposome samples prepared using the dye-containing PBS in Example 1 were used.
(1-1) 50% B7 / negatively charged PEG terminal -OH liposome, 100 nm
(1-3) 50% B7 / negatively charged PEG terminal -OH liposome, 50 nm
(1-4) 50% B7 / negatively charged PEG terminal -OH liposome, 200 nm
(1-5) 50% B7 / negatively charged PEG terminal -OH liposome, 400 nm
The liposome samples were adjusted to a PEGylated liposome concentration of 1 mg/mL (in terms of total lipids), and used as administration samples. The dye-containing PBS was used for the adjustment of the PEGylated liposome concentration. The administration samples had an amount of B7 of 15 µg/30 µL.

30 µL of the administration samples was subcutaneously injected into the left flank of a BALB/c mouse (5 weeks old, female) not having cancer. At 12 hours and 24 hours after administration, the mice were euthanized, and the lymph nodes, spleen, kidney, lung, heart, thymus, and liver were excised.

The distribution of the B7 liposomes in each of the excised organs was confirmed in the same manner as in the aforementioned Example 4, unless otherwise specified.

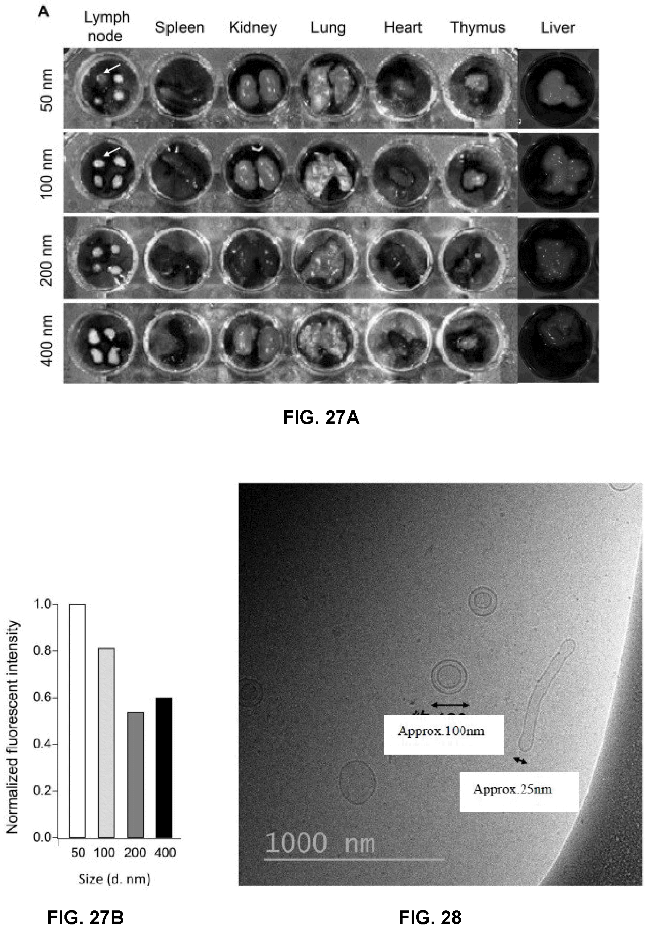

These results are shown in FIG. 27. FIG. 27A is a figure in which the fluorescence intensity is overlaid on the photographs of each organ at 12 hours after administration, for the mice to which the B7 liposome administration samples had been administered. In FIG. 27A, the parts with particularly strong fluorescence intensity are indicated by white arrows. FIG. 27B is a graph in which the fluorescence intensity in the lymph nodes was quantified.

As shown in FIG. 27, the lymph nodes showed higher fluorescence intensity than other organs other than the lymph nodes. For example, the fluorescence intensity of the 50 nm lung, which had the highest fluorescence intensity among the other organs, was equal to or less than the fluorescence intensity of the 200 nm in the lymph nodes. Furthermore, when the fluorescence intensity was divided by the organ weight and normalized to the fluorescence intensity per gram, the amount of accumulation in the lymph nodes was 10 times or more higher than in other organs.

As shown in FIG. 27B, the B7 liposome administration group, regardless of the size, effectively accumulated in the lymph nodes at 12 hours after administration, and the result was that the smaller-sized liposomes accumulated more effectively in the lymph nodes. Furthermore, while there is a report in the prior art that particles of 50 nm or more do not accumulate in lymph nodes, in this Example, it was confirmed that even liposomes of 50 nm or more (for example, 100 nm liposomes) accumulate in the lymph nodes.

(1B)
Next, the administration sample (1-1, 100 nm) was used as a cryo specimen, and observed using a transmission electron microscope (CryoTEM). The CryoTEM specimen was prepared in accordance with the protocol provided by Leica Microsystems, using LEICA EM GP (Leica Microsystems). The CryoTEM was measured using JEM2100 (JEOL Ltd.).

These results are shown in FIG. 28. FIG. 28 is an electron micrograph of the administration sample (1-1). As shown in FIG. 28, by the observation under the electron microscope, many spherical B7 liposomes were confirmed, and some B7 liposomes having a long elongated structure also existed, the length of the short side of which was about 25 nm. The structure of the latter liposomes is considered to be due to soft elongation by the interaction with the edge of the grid on which the administration sample was placed. From this result, it is considered that the B7 liposomes of this Example, for example, even if the average particle size is larger than 50 nm, were effectively taken up into the lymph nodes due to their softness and ability to deform in shape.

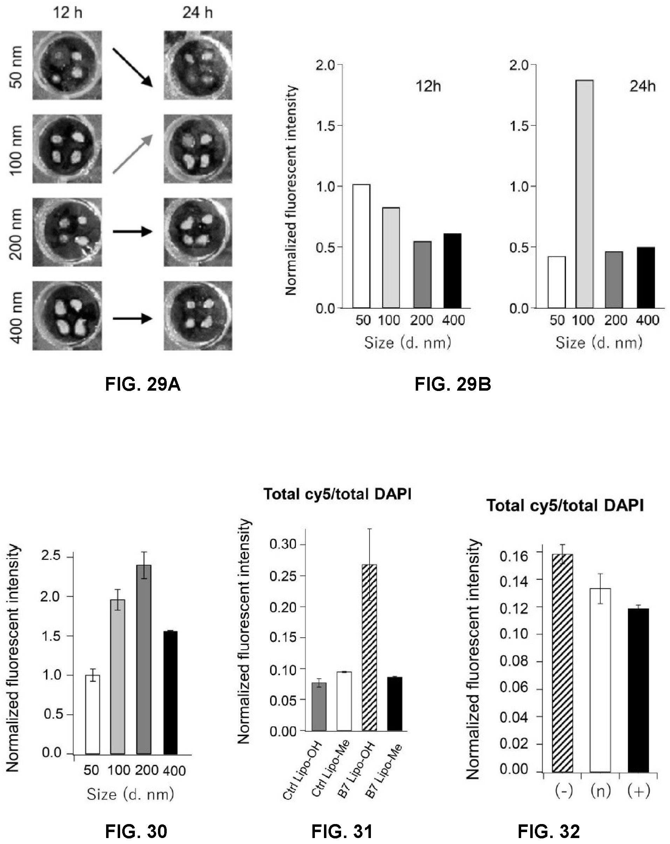

(1C)
Next, FIG. 29 shows the results of the amount of accumulation of the B7 liposomes in the lymph nodes at 24 hours after administration, for the mice to which the B7 liposome administration samples had been administered. FIG. 29A is a figure showing the amount of accumulation of the B7 liposomes in the lymph nodes of mice at 12 hours and 24 hours after administration in terms of fluorescence intensity. The increase or decrease in fluorescence intensity from 12 hours after administration to 24 hours after administration is indicated by arrows. A downward-right arrow indicates a decrease from 12 hours after administration to 24 hours after administration, and an upward-right arrow indicates an increase from 12 hours after administration to 24 hours after administration. FIG. 29B is a graph in which, based on FIG. 29A, the fluorescence intensity showing the amount of accumulation of the B7 liposomes in the lymph nodes of mice at 12 hours and 24 hours after administration was quantified. As shown in FIG. 29, at 12 hours after the administration of the B7 liposomes, the administration groups in which the particle size of the B7 liposomes was different showed similar amounts of accumulation. Then, the administration group of the (1-1) administration sample (average diameter 100 nm) showed a marked increase in the amount of accumulation from 12 hours after administration to 24 hours after administration, and showed the highest amount of accumulation among all the administration groups.

(1D)
Next, human dendritic cell line DC2.4 cells were seeded in an 8-well glass bottom chamber so as to give 20000 cells/well, and incubated overnight at 37°C in RPMI containing 10% V/V FBS and 1% V/V penicillin-streptomycin. Then, the same administration sample as in the aforementioned (1A) (B7 concentration: 15 µg/30 µL) was added to the wells so as to give a final B7 concentration of 0.05 mg/mL, and incubated for 6 hours. After incubation, the cells in the wells were washed with fresh medium (the aforementioned RPMI) to remove the excess administration sample, a staining reagent (0.1 mg/mL Hoechst 33342) was added to the wells, and the nuclei of the cells were stained by incubation for 15 minutes. Next, the cells in the wells were washed with fresh medium (the aforementioned RPMI), and then the cells in the wells were fixed with 4% PFA (PBS solution containing 4% paraformaldehyde) at room temperature for 15 minutes. The fixed cell samples were observed with a confocal laser scanning microscope (CLSM) (LSM 880, Carl Zeiss). Each fluorescence was observed using different excitation (ex.) and emission (em.) filters as follows.
Hoechst 33342: ex. 405 nm / em. 410-485 nm
Sulfo-Cy5: ex. 633 nm / em. 638-746 nm

These results are shown in FIG. 30. FIG. 30 is a graph showing the uptake of the B7 liposomes into the cells by the fluorescence intensity of Cy5 derived from the B7 liposomes. As shown in FIG. 30, the uptake of the B7 liposomes into the dendritic cells increased as the average particle size approached 200 nm, and the administration sample with an average particle size of 200 nm was the highest. From the results of the aforementioned (1A) and (1D), it was found that the uptake into the lymph nodes is more likely to occur as the average diameter of the liposomes is smaller, whereas the uptake into the dendritic cells is more likely to occur as the average diameter of the liposomes is closer to 200 nm.

Furthermore, based on the results of the aforementioned (1A) to (1D), the following is considered; however, the present invention is not limited to these descriptions. That is, the small-sized liposomes with an average particle size of 50 nm are effectively and quickly accumulated in the lymph nodes, but are difficult to be taken up by the dendritic cells therein, and thus are considered to be eliminated with the passage of time. Furthermore, the large-sized liposomes with an average particle size of 200 nm and 400 nm are difficult to be taken up into the lymph nodes, and thus are considered to be difficult to accumulate in the dendritic cells in the lymph nodes. In contrast, the liposomes with an average particle size of 100 nm are relatively easily taken up into the lymph nodes, and are further effectively taken up by the dendritic cells therein, and thus, at 24 hours after administration, the amount of accumulation in the dendritic cells in the lymph nodes is considered to have increased to about 4 times that of liposomes of other sizes.

Based on the results of the aforementioned (1A) to (1D), it was found that the liposome sample of the aforementioned (1-1) (100 nm) is preferable from the viewpoint of accumulation in the lymph nodes and accumulation in the dendritic cells. From this, it can be said that the PEGylated liposome containing the NKT cell-activating ligand in the present invention preferably has an average particle size of 100 nm ± 40 nm or 100 ± 30 nm, more preferably 100 ± 20 nm, 100 ± 10 nm, or 100 ± 5 nm.

### (2) Influence of changes in surface PEG terminus and surface charge of B7 liposome on amount of accumulation in dendritic cells in lymph node

**(2A)**
The following liposome samples prepared using the dye-containing PBS in Example 1 were used. In the abbreviations of each sample, the charge of the surface of the liposome is shown, wherein (-) means negative charge, (+) means positive charge, and (n) means neutral.
(1-1) 50% B7 / negatively charged PEG terminal -OH liposome, 100 nm (B7 lipo-OH(-))
(1-8) 50% B7 / negatively charged PEG terminal -Me liposome, 100 nm (B7 lipo-Me(-))
(1-11) 0% control / negatively charged PEG terminal -OH liposome, 100 nm (Ctrl lipo-OH(-))
(1-12) 0% control / negatively charged PEG terminal -Me liposome, 100 nm (Ctrl lipo-Me(-))
(1-9) 50% B7 / neutral PEG terminal -OH liposome, 100 nm (B7 lipo-OH(n))
(1-10) 50% B7 / positively charged PEG terminal -OH liposome, 100 nm (B7 lipo-OH(+))
The liposome samples were adjusted to a PEGylated liposome concentration of 1 mg/mL (in terms of total lipids), and used as administration samples. The administration samples had an amount of B7 of 15 µg/30 µL.

Except that the aforementioned administration samples were used, the addition of the administration samples to the human dendritic cell line DC2.4 cells, the incubation, the staining, and the observation by a microscope were performed in the same manner as in the aforementioned (1D).

These results are shown in FIGS. 31 and 32. FIG. 31 is a graph showing the uptake of the B7 liposomes into the dendritic cells by the fluorescence intensity of Cy5 derived from the B7 liposomes. In FIG. 31, the vertical axis is the value obtained by normalizing the fluorescence intensity of Cy5 indicating the B7 liposomes with the fluorescence intensity of HOECHST 33342 indicating the amount of cell nuclei, and corresponds to the uptake amount of the B7 liposomes in the dendritic cells. FIG. 31 shows, from the left, the results of the Ctrl lipo-OH(-) administration group, the Ctrl lipo-Me(-) administration group, the B7 lipo-OH(-) administration group, and the B7 lipo-Me(-) administration group.

As shown in FIG. 31, the fluorescence intensity was about the same between the control liposome administration group not containing the NKT ligand, namely, the Ctrl lipo-OH(-) administration group and the Ctrl lipo-Me(-) administration group. Furthermore, the B7 lipo-Me(-) administration group, in which the PEG terminus of the B7 liposome is a methyl group, also had a fluorescence intensity about the same as that of the control liposome administration group. Thus, in the case of the control liposome Ctrl lipo-OH(-) not containing the NKT cell-activating ligand, even when a phospholipid PEGylated with PEG having a hydroxyl group at its terminus was used, the uptake into the dendritic cells did not improve, whereas the B7 lipo-OH(-) administration group, which contains B7 as a ligand and has a hydroxyl group at the PEG terminus, showed a markedly higher fluorescence intensity indicating the uptake amount of the liposome into the dendritic cells compared with the control liposome administration group. Furthermore, the B7 lipo-OH(-) administration group also showed a markedly higher fluorescence intensity indicating the uptake amount of the liposome in the dendritic cells compared with the B7 lipo-Me(-) administration group, in which the terminus of PEG is a methyl group.

FIG. 32 is a graph showing the uptake of the B7 liposomes into the dendritic cells by the fluorescence intensity of Cy5 derived from the B7 liposomes. In FIG. 32, the vertical axis is the value obtained by normalizing the fluorescence intensity of Cy5 indicating the B7 liposomes with the fluorescence intensity of HOECHST 33342 indicating the amount of cell nuclei, and corresponds to the uptake amount of the B7 liposomes in the dendritic cells. FIG. 32 shows the results of the B7 lipo-OH(-) administration group in which the surface charge of the B7 liposome is negative (-), the B7 lipo-OH(n) administration group in which the surface charge is neutral (n), and the B7 lipo-OH(+) administration group in which the surface charge is positive (+).

The administration samples, namely, the B7 lipo-OH(-), the B7 lipo-OH(n), and the B7 lipo-OH(+), all use a phospholipid PEGylated with PEG having a hydroxyl group at its terminus; however, the B7 lipo-OH(n) and the B7 lipo-OH(+) had the charge of the liposome set to neutral (n) and positive charge (+), respectively, by the addition of the positively charged lipid (DDAB). As a result, as shown in FIG. 32, the B7 lipo-OH(-) administration group, in which the liposome has a negative charge, showed a higher fluorescence intensity indicating the uptake amount of the dendritic cells compared with the B7 lipo-OH(n) administration group and the B7 lipo-OH(+) administration group, in which the charge of the liposome is neutral or positive.

From these results, it was found that the PEGylated liposome in the present invention preferably uses a phospholipid PEGylated with PEG having a hydroxyl group at its terminus, and the charge of the liposome is preferably a negative charge.

**(2B)**
Except that 30 µL of the following administration samples, which are the same as in the aforementioned (2A), were used, in the same manner as in the aforementioned (1A), the administration samples were subcutaneously injected into the subcutaneous tissue of the left flank of a BALB/c mouse (5 weeks old, female) not having cancer, and at 24 hours after administration, the lymph nodes were excised.
the B7 lipo-Me(-)
the B7 lipo-OH(-)
the B7 lipo-OH(n)
the B7 lipo-OH(+)

The excised lymph nodes were thinly sliced, and the nuclei of the cells in the obtained sections were stained with the aforementioned staining reagent (0.1 mg/mL Hoechst 33342). Then, for the stained sections, the distribution of the B7 liposomes in the lymph nodes was confirmed. The distribution of the B7 liposomes was observed in the same manner as in the aforementioned (1D), using a confocal laser scanning microscope (CLSM) (LSM 880, Carl Zeiss).

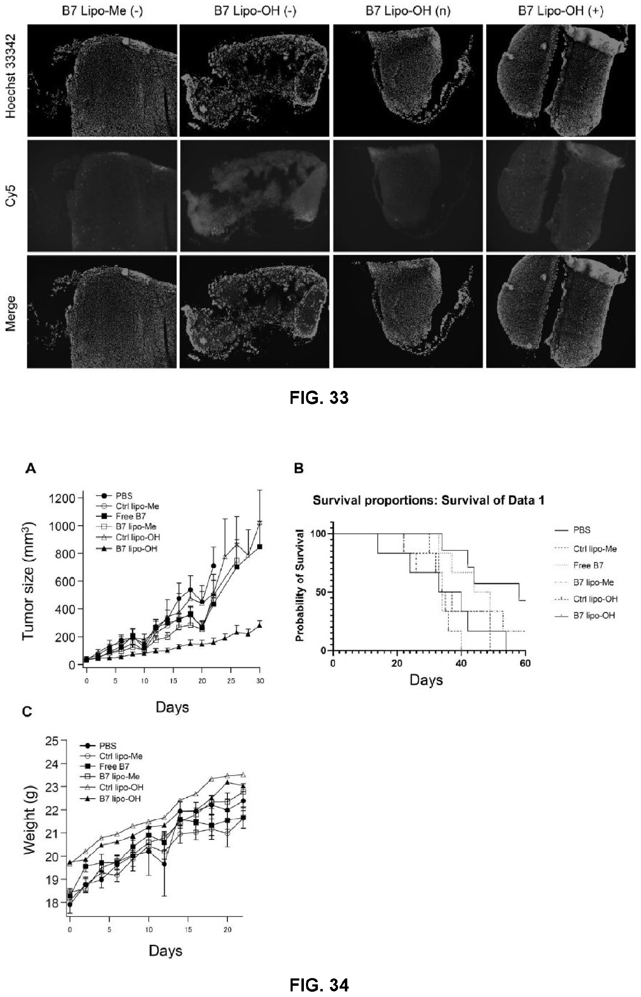

These results are shown in FIG. 33. FIG. 33 shows microscopic observation images of the aforementioned sections, in which the top row is the image of Hoechst 33342 staining, the second row is the fluorescence image of Cy5 derived from the liposomes, and the third row is the image obtained by overlaying the fluorescence distribution of the second row on the staining image of the top row. As shown in FIG. 33, it was confirmed that the B7 liposome B7 lipo-OH(-), in which the surface charge is negatively charged, is the most easily absorbed into the lymph nodes. In contrast, the B7 lipo-Me(-), in which the surface charge is negatively charged but the terminus of PEG is a methyl group, markedly decreased the uptake into the lymph nodes compared with the B7 liposome B7 lipo-OH(-). Furthermore, the B7 lipo-OH(n) and the B7 lipo-OH(+), in which the surface charge is neutral or positively charged, had lower accumulation in the lymph nodes compared with the B7 lipo-OH(-).

From the results of the aforementioned (1A) to (2B), in order to most efficiently accumulate in the lymph nodes and target the dendritic cells in the lymph nodes, the PEGylated liposome containing the NKT cell-activating ligand in the present invention preferably has the following conditions. That is, the liposome preferably has, for example, an average particle size of 100 nm ± 40 nm, 100 ± 30 nm, 100 ± 20 nm, 100 ± 10 nm, or 100 ± 5 nm; a negatively charged surface charge; and a hydroxy group as the PEG terminus on the liposome surface.

### (3) Influence of changes in surface PEG terminus of B7 liposome on anticancer effect

Next, the influence of changes in the surface properties of the liposome on the immune activity was confirmed.

The following liposome samples prepared using PBS in Example 1 were used.
(1-1) 50% B7 / negatively charged PEG terminal -OH liposome, 100 nm (B7 lipo-OH(-))
(1-8) 50% B7 / negatively charged PEG terminal -Me liposome, 100 nm (B7 lipo-Me(-))
(1-11) 0% control / negatively charged PEG terminal -OH liposome, 100 nm (Ctrl lipo-OH(-))
(1-12) 0% control / negatively charged PEG terminal -Me liposome, 100 nm (Ctrl lipo-Me(-))
The liposome samples were adjusted to a PEGylated liposome concentration of 1 mg/mL (in terms of total lipids), and used as administration samples. The administration samples had an amount of B7 of 15 µg/30 µL. In addition, an administration sample of 15 µg/30 µL in which B7 not formulated into a liposome was suspended in PBS (Free B7), and an administration sample of PBS alone (PBS) were used.

In the same manner as in the aforementioned Example 5 (1) [Antitumor effect], 4T1 cells (5.0×10⁵ to 1.0×10⁶ cells/mouse) were subcutaneously inoculated into the vicinity of the second teat from the top of the left chest of a BALB/c mouse (5 weeks old, female, n=6 to 7). When the average tumor volume reached 30 mm³, the mice were randomly divided into groups. Then, except that 30 µL of the aforementioned administration sample was used, according to the administration condition B of the aforementioned Example 5 (1), the administration sample was subcutaneously injected under both sides of the mouse (Day 0). Then, in the same manner as in the aforementioned Example 5 (1), the growth of the tumor, the body weight of the mice, and the survival were monitored.

The results of the antitumor effect are shown in FIG. 34. FIG. 34A is a graph showing the relationship between the tumor volume (V) of the mice and the number of days from the start of administration, FIG. 34B is a graph showing the relationship between the survival rate of the mice and the number of days from the start of administration, and FIG. 34C is a graph showing the relationship between the body weight (g) of the mice and the number of days from the start of administration.

As shown in FIG. 34A, in the PBS administration group (PBS), the Ctrl lipo-OH administration group, the Free B7 administration group, the B7 lipo-Me administration group, and the Ctrl lipo-Me administration group, the tumor volume increased by about 20 to 40-fold on Day 30 relative to Day 0 (30 mm³), and rapid growth of cancer was confirmed. In contrast, in the B7 lipo-OH administration group, the increase in tumor volume on Day 30 relative to Day 0 remained at about 6-fold, and a marked suppression of the increase in tumor volume could be confirmed. From this result, it was found that, in the B7 liposome, by making the PEG terminus a hydroxy group, the cancer suppression effect can be markedly improved.

As shown in FIG. 34B, the B7 lipo-OH administration group could markedly maintain the survival rate of the mice compared with the other administration groups. From this result, it was found that, in the B7 liposome, by making the PEG terminus a hydroxy group, the life-prolonging effect can be markedly improved.

As shown in FIG. 34C, no significant decrease in body weight was confirmed in any of the administration groups. From this result, it is considered that the B7 lipo-OH has no problem in tolerability and safety, and no influence of toxicity.

### (4) Influence of changes in concentration of B7 in B7 liposome on immune activity

It was examined whether the immune activity changes depending on the concentration of B7 in the B7 liposome.

The following liposome samples prepared using PBS in Example 1 were used.
(1-1) 50% B7 / negatively charged PEG terminal -OH liposome, 100 nm (50% B7 lipo)
(1-6) 10% B7 / negatively charged PEG terminal -OH liposome, 100 nm (10% B7 lipo)
(1-7) 2% B7 / negatively charged PEG terminal -OH liposome, 100 nm (2% B7 lipo)
(1-11) 0% control / negatively charged PEG terminal -OH liposome, 100 nm (0% B7 lipo)
The liposome samples were adjusted to a PEGylated liposome concentration of 1 mg/mL (in terms of total lipids), and used as administration samples. The administration sample using the aforementioned (1-1) had an amount of B7 of 15 µg/30 µL, the administration sample using the aforementioned (1-6) had an amount of B7 of 3 µg/30 µL, the administration sample using the aforementioned (1-7) had an amount of B7 of 0.6 µg/30 µL, and the administration sample using the aforementioned (1-11) had an amount of B7 of 0 µg/30 µL.

In the same manner as in the aforementioned Example 5 (1) [Antitumor effect], 4T1 cells (5.0×10⁵ to 1.0×10⁶ cells/mouse) were subcutaneously inoculated into the vicinity of the second teat from the top of the left chest of a BALB/c mouse (5 weeks old, female, n=6 to 7) (Day 0). When the average tumor volume reached 46 mm³, the mice were randomly divided into groups. Then, except that 30 µL of the aforementioned administration sample was used, according to the administration condition A of the aforementioned Example 5 (1), the administration sample was subcutaneously injected under the left flank of the mouse (Day 4). Then, in the same manner as in the aforementioned Example 5 (1), the growth of the tumor, the body weight of the mice, and the survival were monitored.

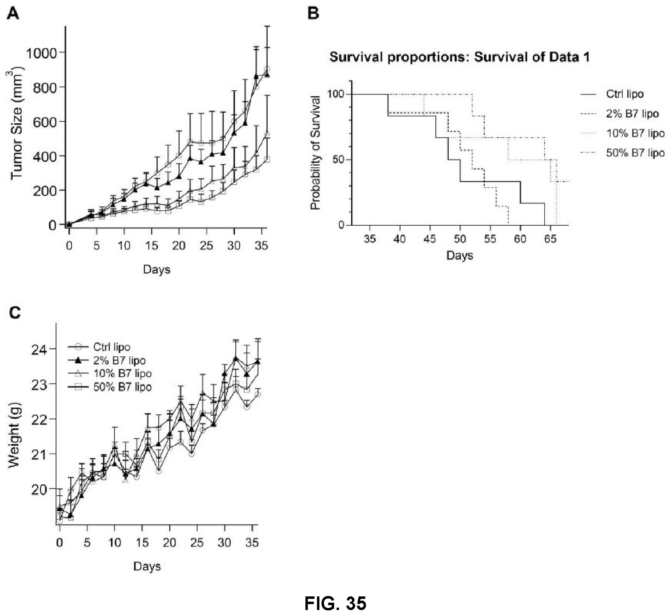

The results of the antitumor effect are shown in FIG. 35. FIG. 35A is a graph showing the relationship between the tumor volume (V) of the mice and the number of days from the day of subcutaneous inoculation of the cancer, FIG. 35B is a graph showing the relationship between the survival rate of the mice and the number of days from the day of subcutaneous inoculation of the cancer, and FIG. 35C is a graph showing the relationship between the body weight of the mice and the number of days from the day of subcutaneous inoculation of the cancer.

As shown in FIG. 35A, in the 0% B7 lipo administration group and the 2% B7 lipo administration group, the tumor volume increased by about 20 to 30-fold on Day 36 relative to Day 0 (46 mm³), and rapid growth of cancer was confirmed. In contrast, in the 10% B7 lipo administration group, the increase in tumor volume on Day 36 relative to Day 0 remained at about 10-fold, and excellent suppression of the increase in tumor volume could be confirmed. Furthermore, in the 50% B7 lipo administration group, the increase in tumor volume on Day 36 relative to Day 0 remained at about 7-fold, and more significant suppression of the increase in tumor volume could be confirmed. From these results, it was found that, with the increase in the B7 concentration of the B7 liposome, the cancer growth suppression effect can be increased.

As shown in FIG. 35B, it was confirmed that the life-prolonging effect increases with the increase in the B7 concentration of the B7 liposome. From this result, it is considered that the higher the B7 concentration contained in the B7 liposome, the more effectively the immune activity occurs, and the antitumor effect and the life-prolonging effect are produced.

As shown in FIG. 35C, no significant decrease in body weight was confirmed in any of the administration groups. From this result, it is considered that the B7 concentration of the B7 liposome has no influence on toxicity.

The present invention has been described above with reference to the embodiments; however, the present invention is not limited to the above embodiments. Various changes that can be understood by those skilled in the art within the scope of the present invention can be made to the configuration and details of the present invention.

This application claims priority based on Japanese Patent Application No. 2023-204574 filed on December 4, 2023, the entire disclosure of which is incorporated herein by reference.

### INDUSTRIAL APPLICABILITY

According to the liposome formulation of the present invention, for example, it has excellent stability during storage, and the effect of activating NKT cells can be obtained in the body.

## Claims

1. A liposome formulation, comprising:
a PEGylated liposome,
wherein the PEGylated liposome comprises an NKT cell-activating ligand and a liposome-constituting lipid,
wherein the NKT cell-activating ligand is α-galactosylceramide or an analog thereof, which is specifically recognized by an NKT cell receptor on an NKT cell, and
wherein the liposome-constituting lipid comprises a choline-containing phospholipid, a PEGylated lipid, and a cholesterol-based lipid.

2. The liposome formulation according to claim 1, wherein the choline-containing phospholipid is a phosphatidylcholine.

3. The liposome formulation according to claim 2, wherein two fatty acids in the phosphatidylcholine are each independently a saturated fatty acid or an unsaturated fatty acid.

4. The liposome formulation according to claim 2 or 3, wherein two fatty acids in the phosphatidylcholine are each independently a fatty acid having 8 to 27 carbon atoms.

5. The liposome formulation according to any one of claims 2 to 4, wherein the phosphatidylcholine is at least one selected from the group consisting of dipalmitoylphosphatidylcholine, dioleoylphosphatidylcholine, palmitoyloleoylphosphatidylcholine, and stearoyloleoylphosphatidylcholine.

6. The liposome formulation according to any one of claims 1 to 5, wherein a fatty acid in the PEGylated lipid is a saturated fatty acid or an unsaturated fatty acid.

7. The liposome formulation according to any one of claims 1 to 6, wherein a fatty acid in the PEGylated lipid is a fatty acid having 8 to 70 carbon atoms.

8. The liposome formulation according to any one of claims 1 to 7, wherein the PEGylated lipid is at least one selected from the group consisting of DSPE-PEG, DMG-PEG, DPPE-PEG, DSG-PEG, DOPE-PEG, ceramide-PEG, and stearic acid-PEG.

9. The liposome formulation according to any one of claims 1 to 8, wherein an average molecular weight of PEG in the PEGylated lipid is from 160 to 44,000 Da.

10. The liposome formulation according to any one of claims 1 to 9, wherein, in the PEGylated lipid, a terminus of PEG is a hydroxyl group.

11. The liposome formulation according to any one of claims 1 to 10, wherein the cholesterol-based lipid is cholesterol.

12. The liposome formulation according to any one of claims 1 to 11, wherein a size of the PEGylated liposome is in a range of an average particle diameter of 10 to 500 nm.

13. The liposome formulation according to any one of claims 1 to 12, wherein, in the PEGylated liposome, a mol% ratio of the choline-containing phospholipid (X), the PEGylated lipid (Y), and the cholesterol-based lipid (Z) is X:Y:Z = 1 : (0.03 to 0.176) : (0.34 to 1.35).

14. The liposome formulation according to any one of claims 1 to 13, wherein the α-galactosylceramide or the analog thereof is a compound represented by formula (I), a salt thereof, or a solvate thereof:

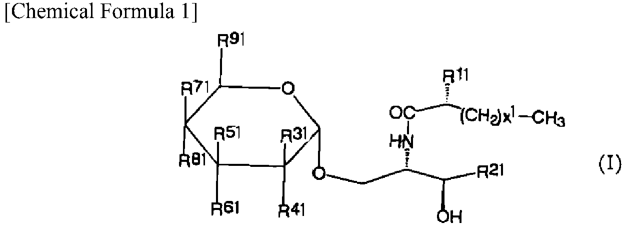

15. The liposome formulation according to any one of claims 1 to 13, wherein the analog of α-galactosylceramide is a compound represented by formula (II), a salt thereof, or a solvate thereof:

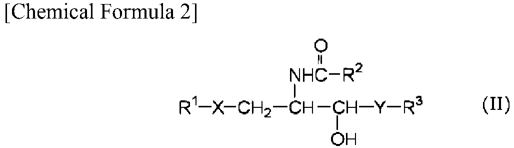

16. The liposome formulation according to any one of claims 1 to 13, wherein the analog of α-galactosylceramide is a compound represented by formula (III), a salt thereof, or a solvate thereof:

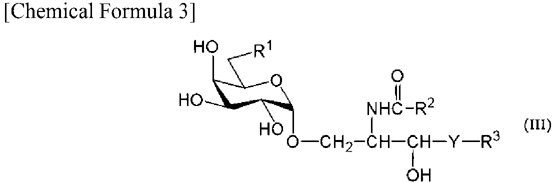

17. The liposome formulation according to any one of claims 1 to 13, wherein the analog of α-galactosylceramide is a compound represented by formula (IV), a salt thereof, or a solvate thereof:

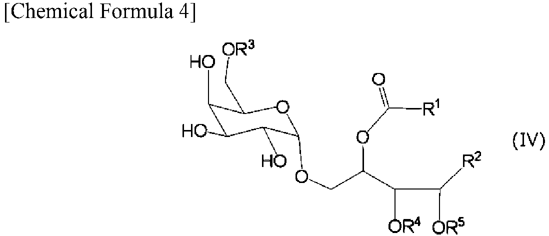

18. The liposome formulation according to any one of claims 1 to 13, wherein the analog of α-galactosylceramide is a compound represented by formula (V), a salt thereof, or a solvate thereof:

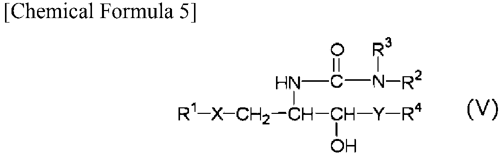

19. The liposome formulation according to any one of claims 1 to 13, wherein the analog of α-galactosylceramide is a compound represented by formula (VI), a salt thereof, or a solvate thereof:

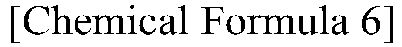

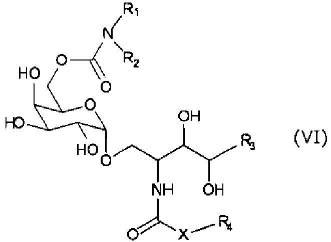

20. The liposome formulation according to claim 19, wherein the analog of α-galactosylceramide is a compound represented by the following formula (VI-1), a salt thereof, or a solvate thereof:

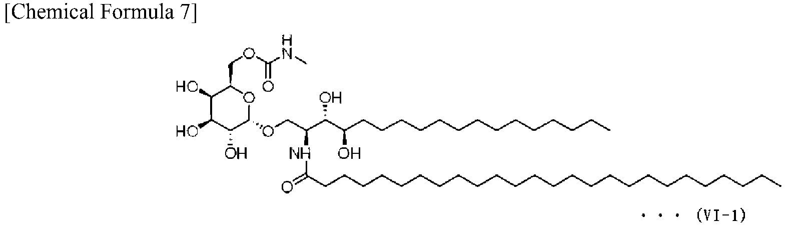

21. The liposome formulation according to any one of claims 1 to 20, wherein the PEGylated liposome satisfies conditions 1, 2, and 3:
(condition 1) a size of the PEGylated liposome is in a range of an average particle diameter of 60 to 140 nm;
(condition 2) in the PEGylated lipid, a terminus of PEG is a hydroxyl group; and
(condition 3) a surface charge of the PEGylated liposome is a negative charge.

22. The liposome formulation according to claim 21, wherein, in condition 3, the surface charge of the PEGylated liposome is from -80 to 5 mV.

23. An NKT cell activator, comprising the liposome formulation according to any one of claims 1 to 22.

24. A pharmaceutical composition, comprising the liposome formulation according to any one of claims 1 to 22.

25. The pharmaceutical composition according to claim 24, which is for anticancer use.

26. The pharmaceutical composition according to claim 25, wherein a target cancer is breast cancer, skin cancer, hematologic cancer, colorectal cancer, prostate cancer, or ovarian cancer.

27. The pharmaceutical composition according to any one of claims 24 to 26, which is an injection.

28. The pharmaceutical composition according to any one of claims 24 to 27, which is for anticancer use, wherein a target cancer is triple-negative breast cancer, and the PEGylated liposome contained in the liposome formulation satisfies conditions 1, 2, and 3:
(condition 1) a size of the PEGylated liposome is in a range of an average particle diameter of 60 to 140 nm;
(condition 2) in the PEGylated lipid, a terminus of PEG is a hydroxyl group; and
(condition 3) a surface charge of the PEGylated liposome is a negative charge.

29. The pharmaceutical composition according to claim 28, wherein, in condition 3, the surface charge of the PEGylated liposome is from -80 to 5 mV.

30. A method of activating an NKT cell, comprising a step of administering, to a subject, the liposome formulation according to any one of claims 1 to 22 or the pharmaceutical composition according to any one of claims 24 to 29.

31. The method of activating an NKT cell according to claim 30, wherein the subject is a human or a non-human animal.

32. A method of treating cancer, comprising a step of administering, to a subject, the liposome formulation according to any one of claims 1 to 22 or the pharmaceutical composition according to any one of claims 24 to 29.

33. The method of treating cancer according to claim 30, wherein the subject is a subject having triple-negative breast cancer, and in the step of administering, the pharmaceutical composition according to claim 28 or 29 is administered.
